# EUROPEAN PATENT SPECIFICATION

(11) **EP 1 636 260 B1**
(45) Date of publication and mention of the grant of the patent: **18.02.2009**
(21) Application number: 04736510.1
(22) Date of filing: 10.06.2004
(51) Int. Cl.: C07K 14/475, C12N 15/62, C12N 5/10, A61K 9/00, A61K 48/00, A61P 25/28, C12N 15/86

(54) **IMPROVED SECRETION OF NEUBLASTIN**
VERBESSERTE SEZERNIERUNG VON NEUBLASTIN
SECRETION AMILIOREE DE NEUBLASTINE

(30) Priority: 10.06.2003 DK 200300861; 02.10.2003 US 507483 P
(43) Date of publication of application: 22.03.2006
(73) Proprietor: NsGene A/S, 2750 Ballerup (DK); Biogen Idec MA Inc., Cambridge, MA 02142 (US)
(72) Inventor: WAHLBERG, Lars, U., DK-4550 Asnæs (DK); GRØNBORG, Mette, DK-2400 Copenhagen NV (DK); KUSK, Philip, DK-3450 Lynge (DK); TORNØE, Jens, DK-2100 Copenhagen Ø (DK); PEDERSON, Nels, E., Mansfield, MA 02048 (US); SISK, William, P., Boxborough, MA 01719 (US)
(74) Representative: HOEIBERG A/S
(86) International application number: PCT/DK2004/000411
(87) International publication number: WO 2004/108760

(56) References cited:
- WO-A-01/47946
- WO-A-02/051433
- WO-A-02/078730
- WO-A-20/04094592
- AEBISCHER ET AL: "INTRATHECAL DELIVERY OF CNTF USING ENCAPSULATED GENETICALLY MODIFIED XENOGENEIC CELLS IN AMYOTROPHIC LALTERAL SCLEOSIS PATIENTS" NATURE MEDICINE, NATURE PUBLISHING, CO, US, vol. 2, 1996, pages 696-699, XP002135052 ISSN: 1078-8956
- ROSENBLAD CARL ET AL: "In vivo protection of nigral dopamine neurons by lentiviral gene transfer of the novel GDNF-family member neublastin/artemin" MOLECULAR AND CELLULAR NEUROSCIENCE, vol. 15, no. 2, February 2000 (2000-02), pages 199-214, XP002262602 ISSN: 1044-7431
- BALOH ROBERT H ET AL: "Functional mapping of receptor specificity domains of glial cell line-derived neurotrophic factor (GDNF) family ligands and production of GFRalpha1 RET-specific agonists" JOURNAL OF BIOLOGICAL CHEMISTRY, vol. 275, no. 5, 4 February 2000 (2000-02-04), pages 3412-3420, XP002317632 ISSN: 0021-9258
- SAARMA MART: "GDNF: A stranger in the TGF-beta superfamily?" EUROPEAN JOURNAL OF BIOCHEMISTRY, vol. 267, no. 24, December 2000 (2000-12), pages 6968-6971, XP002317633 ISSN: 0014-2956
- AEBISCHER P ET AL: "Recombinant proteins for neurodegenerative diseases: the delivery issue" TRENDS IN NEUROSCIENCE, ELSEVIER, AMSTERDAM, NL, vol. 24, no. 9, 1 September 2001 (2001-09-01), pages 533-540, XP004298585 ISSN: 0166-2236

### SEQUENCE LISTING

<110> NsGene A/S
   Biogen Idec MA Inc.
   Grønhorg, Mette
   Wahlberg, Lars
   Tornøe, Jens
   Kusk, Philip
   Pederson, Nels E.
   Sisk, William P.
<120> Improved secretion of Neublastin
<130> P 951 PC00
<160> 82
<170> PatentIn version 3.1
<210> 1
   <211> 19
   <212> PRT
   <213> Homo sapiens
<400> 1
   Met Asp Cys Thr Trp Arg Ile Leu Phe Leu Val Ala Ala Ala Thr Gly
   1               5                   10                  15
   Thr His Ala
<210> 2
   <211> 19
   <212> PRT
   <213> Macaca mulatta
<400> 2
   Met Lys His Leu Trp Phe Phe Leu Leu Leu Val Ala Ala Pro Arg Trp
   1               5                   10                  15
   Val Leu Ser
<210> 3
   <211> 19
   <212> PRT
   <213> Callithrix jacchus
<400> 3
   Met Asp Trp Thr Trp Arg Ile Phe Leu Leu Val Ala Thr Ala Thr Gly
   1               5                   10                  15
   Ala His Ser
<210> 4
   <211> 19
   <212> PRT
   <213> Mus musculus
<400> 4
   Met Lys Cys Ser Trp Val Ile Phe Phe Leu Met Ala Val Val Thr Gly
   1 5 10 15
   Val Asn Ser
<210> 5
   <211> 19
   <212> PRT
   <213> Sus scrofa
<400> 5
   Met Glu Phe Arg Leu Asn Trp Val Val Leu Phe Ala Leu Leu Gln Gly
   1 5 10 15
   Val Gln Gly
<210> 6
   <211> 19
   <212> PRT
   <213> Rattus norvegicus
<400> 6
   Met Lys Cys Ser Trp Ile Ile Leu Phe Leu Met Ala Leu Thr Thr Gly
   1 5 10 15
   Val Asn Ser
<210> 7
   <211> 478
   <212> DNA
   <213> artificial sequence
<220>
<223> murine IgSP - human NBN113
<220>
   <221> Intron
   <222> (47)..(125)
   <223>
<220>
   <221> exon
   <222> (1)..(46)
   <223>
<220>
   <221> exon
   <222> (126)..(478)
   <223>
<400> 7
   atg aaa tgc agc tgg gtt atc ttc ttc ctg atg gca gtg gtt aca g          46
   Met Lys Cys Ser Trp Val Ile Phe Phe Leu Met Ala Val Val Thr
   1               5                   10                  15
   gtaaggggct cccaagtccc aaacttgagg gtccataaac tctgtgacag tggcaatcac     106
   tttgcctttc tttctacag gg gtg aat tcg gct ggg ggc ccg ggc agc cgc      157
                        Gly Val Asn Ser Ala Gly Gly Pro Gly Ser Arg
                                        20                  25
   gct cgg gca gcg ggg gcg cgg ggc tgc cgc ctg cgc tcg cag ctg gtg       205
   Ala Arg Ala Ala Gly Ala Arg Gly Cys Arg Leu Arg Ser Gln Leu Val
               30                  35                  40
   ccg gtg cgc gcg ctc ggc ctg ggc cac cgc tcc gac gag ctg gtg cgt       253
   Pro Val Arg Ala Leu Gly Leu Gly His Arg Ser Asp Glu Leu Val Arg
       45                  50                  55
   ttc cgc ttc tgc agc ggc tcc tgc cgc cgc gcg cgc tct cca cac gac       301
   Phe Arg Phe Cys Ser Gly Ser Cys Arg Arg Ala Arg Ser Pro His Asp
       60                  65                  70
   ctc agc ctg gcc agc cta ctg ggc gcc ggg gcc ctg cga ccg ccc ccg       349
   Leu Ser Leu Ala Ser Leu Leu Gly Ala Gly Ala Leu Arg Pro Pro Pro
   75                  80                  85                  90
   ggc tcc cgg ccc gtc agc cag ccc tgc tgc cga ccc acg cgc tac gaa       397
   Gly Ser Arg Pro Val Ser Gln Pro Cys Cys Arg Pro Thr Arg Tyr Glu
                   95                  100                 105
   gcg gtc tcc ttc atg gac gtc aac agc acc tgg aga acc gtg gac cgc       445
   Ala Val Ser Phe Met Asp Val Asn Ser Thr Trp Arg Thr Val Asp Arg
               110                 115                 120
   ctc tcc gcc acc gcc tgc ggc tgc ctg ggc tga                           478
   Leu Ser Ala Thr Ala Cys Gly Cys Leu Gly
           125                 130
<210> 8
   <211> 399
   <212> DNA
   <213> artificial sequence
<220>
   <223> Murine IgSP - human NBNs13
   <220>
   <221> CDS
   <222> (1)..(399)
   <223>
<220>
   <221> sig_peptide
   <222> (1)..(57)
   <223> Murine IgSP
<220>
   <221> misc_feature
   <222> (58)..(396)
   <223> Human 113 Neublastin
<400> 8
   atg aaa tgc agc tgg gtt atc ttc ttc ctg atg gca gtg gtt aca ggg          48
   Met Lys Cys Ser Trp Val Ile Phe Phe Leu Met Ala Val Val Thr Gly
   1               5                   10                  15
   gtg aat tcg gct ggg ggc ccg ggc agc cgc gct cgg gca gcg ggg gcg          96
   Val Asn Ser Ala Gly Gly Pro Gly Ser Arg Ala Arg Ala Ala Gly Ala
               20                  25                  30
   cgg ggc tgc cgc ctg cgc tcg cag ctg gtg ccg gtg cgc gcg ctc ggc         144
    Arg Gly Cys Arg Leu Arg Ser Gln Leu Val Pro Val Arg Ala Leu Gly
            35                  40                  45
   ctg ggc cac cgc tcc gac gag ctg gtg cgt ttc cgc ttc tgc agc ggc        192
   Leu Gly His Arg Ser Asp Glu Leu Val Arg Phe Arg Phe Cys Ser Gly
       50                  55                  60
   tcc tgc cgc cgc gcg cgc tct cca cac gac ctc agc ctg gcc agc cta        240
   Ser Cys Arg Arg Ala Arg Ser Pro His Asp Leu Ser Leu Ala Ser Leu
   65                  70                  75                  80
   ctg ggc gcc ggg gcc ctg cga ccg ccc ccg ggc tcc cgg ccc gtc agc        288
   Leu Gly Ala Gly Ala Leu Arg Pro Pro Pro Gly Ser Arg Pro Val Ser
                   85                  90                  95
   cag ccc tgc tgc cga ccc acg cgc tac gaa gcg gtc tcc ttc atg gac        336
   Gln Pro Cys Cys Arg Pro Thr Arg Tyr Glu Ala Val Ser Phe Met Asp
               100                 105                 110
   gtc aac agc acc tgg aga acc gtg gac cgc ctc tcc gcc acc gcc tgc        384
   Val Asn Ser Thr Trp Arg Thr Val Asp Arg Leu Ser Ala Thr Ala Cys
           115                 120                 125
   ggc tgc ctg ggc tga                                                    399
   Gly Cys Leu Gly
       130
<210> 9
   <211> 132
   <212> PRT
   <213> artificial sequence
<220>
   <223> Murine IgSP - human NBN113
   <220>
   <221> misc_feature
   <222> (58)..(396)
   <223> Human 113 Neublastin
<400> 9
   Met Lys Cys Ser Trp Val Ile Phe Phe Leu Met Ala Val Val Thr Gly
   1 5 10 15
   Val Asn Ser Ala Gly Gly Pro Gly Ser Arg Ala Arg Ala Ala Gly Ala
   20 25 30
   Arg Gly Cys Arg Leu Arg Ser Gln Leu Val Pro Val Arg Ala Leu Gly
   35 40 45
   Leu Gly His Arg Ser Asp Glu Leu Val Arg Phe Arg Phe Cys Ser Gly
   50 55 60
   Ser Cys Arg Arg Ala Arg Ser Pro His Asp Leu Ser Leu Ala Ser Leu
   65 70 75 80
   Leu Gly Ala Gly Ala Leu Arg Pro Pro Pro Gly Ser Arg Pro Val Ser
   85 90 95
   Gln Pro Cys Cys Arg Pro Thr Arg Tyr Glu Ala Val Ser Phe Met Asp
   100 105 110
   Val Asn Ser Thr Trp Arg Thr Val Asp Arg Leu Ser Ala Thr Ala Cys
   115 120 125
   Gly Cys Leu Gly
   130
<210> 10
   <211> 220
   <212> PRT
   <213> Homo sapiens
<220>
   <221> misc_feature
   <223> Full length human Neublastin
<220>
   <221> signal
   <222> (1)..(39)
   <223>
<220>
   <221> PROPEP
   <222> (40)..(80)
   <223>
<220>
   <221> mat_peptide
   <222> (81)..()
   <223>
<400> 10
   Met Glu Leu Gly Leu Gly Gly Leu Ser Thr Leu Ser His Cys Pro Trp
   -80 -75 -70 -65
   Pro Arg Arg Gln Pro Ala Leu Trp Pro Thr Leu Ala Ala Leu Ala Leu
   -60 -55 -50
   Leu Ser Ser Val Ala Glu Ala Ser Leu Gly Ser Ala Pro Arg Ser Pro
   -45 -40 -35
   Ala Pro Arg Glu Gly Pro Pro Pro Val Leu Ala Ser Pro Ala Gly His
   -30 -25 -20
   Leu Pro Gly Gly Arg Thr Ala Arg Trp Cys Ser Gly Arg Ala Arg Arg
   -15 -10 -5 -1
   Pro Pro Pro Gln Pro Ser Arg Pro Ala Pro Pro Pro Pro Ala Pro Pro
   1 5 10 15
   Ser Ala Leu Pro Arg Gly Gly Arg Ala Ala Arg Ala Gly Gly Pro Gly
   20 25 30
   Ser Arg Ala Arg Ala Ala Gly Ala Arg Gly Cys Arg Leu Arg Ser Gln
   35 40 45
   Leu Val Pro Val Arg Ala Leu Gly Leu Gly His Arg Ser Asp Glu Leu
   50 55 60
   Val Arg Phe Arg Phe Cys Ser Gly Ser Cys Arg Arg Ala Arg Ser Pro
   65 70 75 80
   His Asp Leu Ser Leu Ala Ser Leu Leu Gly Ala Gly Ala Leu Arg Pro
   85 90 95
   Pro Pro Gly Ser Arg Pro Val Ser Gln Pro Cys Cys Arg Pro Thr Arg
   100 105 110
   Tyr Glu Ala Val Ser Phe Met Asp Val Asn Ser Thr Trp Arg Thr Val
   115 120 125
   Asp Arg Leu Ser Ala Thr Ala Cys Gly Cys Leu Gly
   130 135 140
<210> 11
   <211> 224
   <212> PRT
   <213> Mus musculus
<220>
   <221> SIGNAL
   <222> (1)..(39)
   <223>
<220>
   <221> PROPEP
   <222> (40)..(80)
   <223>
<220>
   <221> mat_peptide
   <222> (81)..()
   <223>
<400> 11
   Met Glu Leu Gly Leu Ala Glu Pro Thr Ala Leu Ser His Cys Leu Arg
   -80 -75 -70 -65
   Pro Arg Trp Gln Ser Ala Trp Trp Pro Thr Leu Ala Val Leu Ala Leu
   -60 -55 -50
   Leu Ser Cys Val Thr Glu Ala Ser Leu Asp Pro Met Ser Arg Ser Pro
   -45 -40 -35
   Ala Ala Arg Asp Gly Pro Ser Pro Val Leu Ala Pro Pro Thr Asp His
   -30 -25 -20
   Leu Pro Gly Gly His Thr Ala His Leu Cys Ser Glu Arg Thr Leu Arg
   -15 -10 -5 -1
   Pro Pro Pro Gln Ser Pro Gln Pro Ala Pro Pro Pro Pro Gly Pro Ala
   1 5 10 15
   Leu Gln Ser Pro Pro Ala Ala Leu Arg Gly Ala Arg Ala Ala Arg Ala
   20 25 30
   Gly Thr Arg Ser Ser Arg Ala Arg Thr Thr Asp Ala Arg Gly Cys Arg
   35 40 45
   Leu Arg Ser Gln Leu Val Pro Val Ser Ala Leu Gly Leu Gly His Ser
   50 55 60
   Ser Asp Glu Leu Ile Arg Phe Arg Phe Cys Ser Gly Ser Cys Arg Arg
   65 70 75 80
   Ala Arg Ser Gln His Asp Leu Ser Leu Ala Ser Leu Leu Gly Ala Gly
   85 90 95
   Ala Leu Arg Ser Pro Pro Gly Ser Arg Pro Ile Ser Gln Pro Cys Cys
   100 105 110
   Arg Pro Thr Arg Tyr Glu Ala Val Ser Phe Met Asp Val Asn Ser Thr
   115 120 125
   Trp Arg Thr Val Asp His Leu Ser Ala Thr Ala Cys Gly Cys Leu Gly
   130 135 140
<210> 12
   <211> 224
   <212> PRT
   <213> Rattus norvegicus
<220>
   <221> SIGNAL
   <222> (1)..(39)
   <223>
<220>
   <221> PROPEP
   <222> (40)..(80)
   <223>
<220>
   <221> mat_peptide
   <222> (81)..()
   <223>
<400> 12
   Met Glu Leu Gly Leu Gly Glu Pro Thr Ala Leu Ser His Cys Leu Arg
   -80                 -75                 -70                 -65
   Pro Arg Trp Gln Pro Ala Leu Trp Pro Thr Leu Ala Ala Leu Ala Leu
                   -60                 -55                 -50
   Leu Ser Ser Val Thr Glu Ala Ser Leu Asp Pro Met Ser Arg Ser Pro
               -45                 -40                 -35
   Ala Ser Arg Asp Val Pro Ser Pro Val Leu Ala Pro Pro Thr Asp Tyr
           -30                 -25                 -20
   Leu Pro Gly Gly His Thr Ala His Leu Cys Ser Glu Arg Ala Leu Arg
       -15                 -10                 -5                  -1
   Pro Pro Pro Gln Ser Pro Gln Pro Ala Pro Pro Pro Pro Gly Pro Ala
   1               5                   10                  15
   Leu Gln Ser Pro Pro Ala Ala Leu Arg Gly Ala Arg Ala Ala Arg Ala
               20                  25                  30
   Gly Thr Arg Ser Ser Arg Ala Arg Ala Thr Asp Ala Arg Gly Cys Arg
           35                  40                  45
   Leu Arg Ser Gln Leu Val Pro Val Ser Ala Leu Gly Leu Gly His Ser
       50                  55                  60
   Ser Asp Glu Leu Ile Arg Phe Arg Phe Cys Ser Gly Ser Cys Arg Arg
   65 70 75 80
   Ala Arg Ser Pro His Asp Leu Ser Leu Ala Ser Leu Leu Asp Ala Gly
   85 90 95
   Ala Leu Arg Ser Pro Pro Gly Ser Arg Pro Ile Ser Gln Pro Cys Cys
   100 105 110
   Arg Pro Thr Arg Tyr Glu Ala Val Ser Phe Met Asp Val Asn Ser Thr
   115 120 125
   Trp Arg Thr Val Asp His Leu Ser Ala Thr Ala Cys Gly Cys Leu Gly
   130 135 140
<210> 13
   <211> 116
   <212> PRT
   <213> Homo sapiens
<400> 13
   Ala Ala Arg Ala Gly Gly Pro Gly Ser Arg Ala Arg Ala Ala Gly Ala
   1 5 10 15
   Arg Gly Cys Arg Leu Arg Ser Gln Leu Val Pro Val Arg Ala Leu Gly
   20 25 30
   Leu Gly His Arg Ser Asp Glu Leu Val Arg Phe Arg Phe Cys Ser Gly
   35 40 45
   Ser Cys Arg Arg Ala Arg Ser Pro His Asp Leu Ser Leu Ala Ser Leu
   50 55 60
   Leu Gly Ala Gly Ala Leu Arg Pro Pro Pro Gly Ser Arg Pro Val Ser
   65 70 75 80
   Gln Pro Cys Cys Arg Pro Thr Arg Tyr Glu Ala Val Ser Phe Met Asp
   85 90 95
   Val Asn Ser Thr Trp Arg Thr Val Asp Arg Leu Ser Ala Thr Ala Cys
   100 105 110
   Gly Cys Leu Gly
   115
<210> 14
   <211> 113
   <212> PRT
   <213> Homo sapiens
<210> 15
   <211> 119
   <212> PRT
   <213> Mus musculus
<400> 15
   Gly Ala Arg Ala Ala Arg Ala Gly Thr Arg Ser Ser Arg Ala Arg Thr
   1               5                   10                  15
   Thr Asp Ala Arg Gly Cys Arg Leu Arg Ser Gln Leu Val Pro Val Ser
               20                  25                  30
   Ala Leu Gly Leu Gly His Ser Ser Asp Glu Leu Ile Arg Phe Arg Phe
           35                  40                  45
   Cys Ser Gly Ser Cys Arg Arg Ala Arg Ser Gln His Asp Leu Ser Leu
       50                  55                  60
   Ala Ser Leu Leu Gly Ala Gly Ala Leu Arg Ser Pro Pro Gly Ser Arg
   65                  70                  75                  80
   Pro Ile Ser Gln Pro Cys Cys Arg Pro Thr Arg Tyr Glu Ala Val Ser
                   85                  90                  95
   Phe Met Asp Val Asn Ser Thr Trp Arg Thr Val Asp His Leu Ser Ala
               100                 105                 110
   Thr Ala Cys Gly Cys Leu Gly
   115
<210> 16
   <211> 116
   <212> PRT
   <213> Mus musculus
<400> 16
   Ala Ala Arg Ala Gly Thr Arg Ser Ser Arg Ala Arg Thr Thr Asp Ala
   1 5 10 15
   Arg Gly Cys Arg Leu Arg Ser Gln Leu Val Pro Val Ser Ala Leu Gly
   20 25 30
   Leu Gly His Ser Ser Asp Glu Leu Ile Arg Phe Arg Phe Cys Ser Gly
   35 40 45
   Ser Cys Arg Arg Ala Arg Ser Gln His Asp Leu Ser Leu Ala Ser Leu
   50 55 60
   Leu Gly Ala Gly Ala Leu Arg Ser Pro Pro Gly Ser Arg Pro Ile Ser
   65 70 75 80
   Gln Pro Cys Cys Arg Pro Thr Arg Tyr Glu Ala Val Ser Phe Met Asp
   85 90 95
   Val Asn Ser Thr Trp Arg Thr Val Asp His Leu Ser Ala Thr Ala Cys
   100 105 110
   Gly Cys Leu Gly
   115
<210> 17
   <211> 116
   <212> PRT
   <213> Rattus norvegicus
<400> 17
   Ala Ala Arg Ala Gly Thr Arg Ser Ser Arg Ala Arg Ala Thr Asp Ala
   1 5 10 15
   Arg Gly Cys Arg Leu Arg Ser Gln Leu Val Pro Val Ser Ala Leu Gly
   20 25 30
   Leu Gly His Ser Ser Asp Glu Leu Ile Arg Phe Arg Phe Cys Ser Gly
   35 40 45
   Ser Cys Arg Arg Ala Arg Ser Pro His Asp Leu Ser Leu Ala Ser Leu
   50 55 60
   Leu Asp Ala Gly Ala Leu Arg Ser Pro Pro Gly Ser Arg Pro Ile Ser
   65 70 75 80
   Gln Pro Cys Cys Arg Pro Thr Arg Tyr Glu Ala Val Ser Phe Met Asp
   85 90 95
   Val Asn Ser Thr Trp Arg Thr Val Asp His Leu Ser Ala Thr Ala Cys
   100 105 110
   Gly Cys Leu Gly
   115
<210> 18
   <211> 113
   <212> PRT
   <213> Rattus norvegicus
<400> 18
   Ala Gly Thr Arg Ser Ser Arg Ala Arg Ala Thr Asp Ala Arg Gly Cys
   1 5 10 15
   Arg Leu Arg Ser Gln Leu Val Pro Val Ser Ala Leu Gly Leu Gly His
   20 25 30
   Ser Ser Asp Glu Leu Ile Arg Phe Arg Phe Cys Ser Gly Ser Cys Arg
   35 40 45
   Arg Ala Arg Ser Pro His Asp Leu Ser Leu Ala Ser Leu Leu Asp Ala
   50 55 60
   Gly Ala Leu Arg Ser Pro Pro Gly Ser Arg Pro Ile Ser Gln Pro Cys
   65 70 75 80
   Cys Arg Pro Thr Arg Tyr Glu Ala Val Ser Phe Met Asp Val Asn Ser
   85 90 95
   Thr Trp Arg Thr Val Asp His Leu Ser Ala Thr Ala Cys Gly Cys Leu
   100 105 110
   Gly
<210> 19
   <211> 104
   <212> PRT
   <213> Homo sapiens
<400> 19
   Ala Ala Gly Ala Arg Gly Cys Arg Leu Arg Ser Gln Leu Val Pro Val
   1               5                   10                  15
   Arg Ala Leu Gly Leu Gly His Arg Ser Asp Glu Leu Val Arg Phe Arg
               20                  25                  30
   Phe Cys Ser Gly Ser Cys Arg Arg Ala Arg Ser Pro His Asp Leu Ser
           35                  40                  45
   Leu Ala Ser Leu Leu Gly Ala Gly Ala Leu Arg Pro Pro Pro Gly Ser
       50                  55                  60
   Arg Pro Val Ser Gln Pro Cys Cys Arg Pro Thr Arg Tyr Glu Ala Val
   65                  70                  75                  80
   Ser Phe Met Asp Val Asn Ser Thr Trp Arg Thr Val Asp Arg Leu Ser
                   85                  90                  95
   Ala Thr Ala Cys Gly Cys Leu Gly
               100
<210> 20
   <211> 99
   <212> PRT
   <213> Homo sapiens
<400> 20
   Gly Cys Arg Leu Arg Ser Gln Leu Val Pro Val Arg Ala Leu Gly Leu
   1               5                   10                  15
   Gly His Arg Ser Asp Glu Leu Val Arg Phe Arg Phe Cys Ser Gly Ser
               20                  25                  30
   Cys Arg Arg Ala Arg Ser Pro His Asp Leu Ser Leu Ala Ser Leu Leu
           35                  40                  45
   Gly Ala Gly Ala Leu Arg Pro Pro Pro Gly Ser Arg Pro Val Ser Gln
       50                  55                  60
   Pro Cys Cys Arg Pro Thr Arg Tyr Glu Ala Val Ser Phe Met Asp Val
   65                  70                  75                  80
   Asn Ser Thr Trp Arg Thr Val Asp Arg Leu Ser Ala Thr Ala Cys Gly
                   85                  90                  95
   Cys Leu Gly
<210> 21
   <211> 140
   <212> PRT
   <213> Homo sapiens
<400> 21
   Pro Pro Pro Gln Pro Ser Arg Pro Ala Pro Pro Pro Pro Ala Pro Pro
   1               5                   10                  15
   Ser Ala Leu Pro Arg Gly Gly Arg Ala Ala Arg Ala Gly Gly Pro Gly
               20                  25                  30
   Ser Arg Ala Arg Ala Ala Gly Ala Arg Gly Cys Arg Leu Arg Ser Gln
           35                  40                  45
   Leu Val Pro Val Arg Ala Leu Gly Leu Gly His Arg Ser Asp Glu Leu
       50                  55                  60
   Val Arg Phe Arg Phe Cys Ser Gly Ser Cys Arg Arg Ala Arg Ser Pro
   65                  70                  75                  80
   His Asp Leu Ser Leu Ala Ser Leu Leu Gly Ala Gly Ala Leu Arg Pro
                   85                  90                  95
   Pro Pro Gly Ser Arg Pro Val Ser Gln Pro Cys Cys Arg Pro Thr Arg
               100                 105                 110
   Tyr Glu Ala Val Ser Phe Met Asp Val Asn Ser Thr Trp Arg Thr Val
           115                 120                 125
   Asp Arg Leu Ser Ala Thr Ala Cys Gly Cys Leu Gly
       130                 135                 140
<210> 22
   <211> 106
   <212> PRT
   <213> Homo sapiens
<400> 22
   Ala Arg Ala Ala Gly Ala Arg Gly Cys Arg Leu Arg Ser Gln Leu Val
   1               5                   10                  15
   Pro Val Arg Ala Leu Gly Leu Gly His Arg Ser Asp Glu Leu Val Arg
               20                  25                  30
   Phe Arg Phe Cys Ser Gly Ser Cys Arg Arg Ala Arg Ser Pro His Asp
           35                  40                  45
   Leu Ser Leu Ala Ser Leu Leu Gly Ala Gly Ala Leu Arg Pro Pro Pro
       50                  55                  60
   Gly Ser Arg Pro Val Ser Gln Pro Cys Cys Arg Pro Thr Arg Tyr Glu
   65                  70                  75                  80
   Ala Val Ser Phe Met Asp Val Asn Ser Thr Trp Arg Thr Val Asp Arg
   85 90 95
   Leu Ser Ala Thr Ala Cys Gly Cys Leu Gly
   100 105
<210> 23
   <211> 102
   <212> PRT
   <213> Homo sapiens
<400> 23
   Gly Ala Arg Gly Cys Arg Leu Arg Ser Gln Leu Val Pro Val Arg Ala
   1 5 10 15
   Leu Gly Leu Gly His Arg Ser Asp Glu Leu Val Arg Phe Arg Phe Cys
   20 25 30
   Ser Gly Ser Cys Arg Arg Ala Arg Ser Pro His Asp Leu Ser Leu Ala
   35 40 45
   Ser Leu Leu Gly Ala Gly Ala Leu Arg Pro Pro Pro Gly Ser Arg Pro
   50 55 60
   Val Ser Gln Pro Cys Cys Arg Pro Thr Arg Tyr Glu Ala Val Ser Phe
   65 70 75 80
   Met Asp Val Asn Ser Thr Trp Arg Thr Val Asp Arg Leu Ser Ala Thr
   85 90 95
   Ala Cys Gly Cys Leu Gly
   100
<210> 24
   <211> 39
   <212> PRT
   <213> Homo sapiens
<400> 24
   Met Glu Leu Gly Leu Gly Gly Leu Ser Thr Leu Ser His Cys Pro Trp
   1 5 10 15
   Pro Arg Arg Gln Pro Ala Leu Trp Pro Thr Leu Ala Ala Leu Ala Leu
   20 25 30
   Leu Ser Ser Val Ala Glu Ala
   35
<210> 25
   <211> 179
   <212> PRT
   <213> artificial sequence
<220>
   <223> deltapro human NBN140
   <220>
   <221> signal
   <222> (1)..(39)
   <223>
<220>
   <221> mat_peptide
   <222> (40)..()
   <223>
<400> 25
   Met Glu Leu Gly Leu Gly Gly Leu Ser Thr Leu Ser His Cys Pro Trp
                   -35                 -30                 -25
   Pro Arg Arg Gln Pro Ala Leu Trp Pro Thr Leu Ala Ala Leu Ala Leu
               -20                 -15                 -10
   Leu Ser Ser Val Ala Glu Ala Pro Pro Pro Gln Pro Ser Arg Pro Ala
           -5                  -1  1               5
   Pro Pro Pro Pro Ala Pro Pro Ser Ala Leu Pro Arg Gly Gly Arg Ala
   10                  15                  20                  25
   Ala Arg Ala Gly Gly Pro Gly Ser Arg Ala Arg Ala Ala Gly Ala Arg
                   30                  35                  40
   Gly Cys Arg Leu Arg Ser Gln Leu Val Pro Val Arg Ala Leu Gly Leu
               45                  50                  55
   Gly His Arg Ser Asp Glu Leu Val Arg Phe Arg Phe Cys Ser Gly Ser
           60                  65                  70
   Cys Arg Arg Ala Arg Ser Pro His Asp Leu Ser Leu Ala Ser Leu Leu
       75                  80                  85
   Gly Ala Gly Ala Leu Arg Pro Pro Pro Gly Ser Arg Pro Val Ser Gln
   90                  95                  100                 105
   Pro Cys Cys Arg Pro Thr Arg Tyr Glu Ala Val Ser Phe Met Asp Val
                   110                 115                 120
   Asn Ser Thr Trp Arg Thr Val Asp Arg Leu Ser Ala Thr Ala Cys Gly
               125                 130                 135
   Cys Leu Gly
           140
<210> 26
   <211> 152
   <212> PRT
   <213> artificial sequence
<220>
   <223> deltapro human NBN113
   <220>
   <221> SIGNAL
   <222> (1)..(39)
   <223>
<220>
   <221> mat_peptide
   <222> (40)..()
   <223>
<400> 26
   Met Glu Leu Gly Leu Gly Gly Leu Ser Thr Leu Ser His Cys Pro Trp
                   -35                 -30                 -25
   Pro Arg Arg Gln Pro Ala Leu Trp Pro Thr Leu Ala Ala Leu Ala Leu
               -20                 -15                 -10
   Leu Ser Ser Val Ala Glu Ala Ala Gly Gly Pro Gly Ser Arg Ala Arg
           -5                  -1  1               5
   Ala Ala Gly Ala Arg Gly Cys Arg Leu Arg Ser Gln Leu Val Pro Val
   10                  15                  20                  25
   Arg Ala Leu Gly Leu Gly His Arg Ser Asp Glu Leu Val Arg Phe Arg
                   30                  35                  40
   Phe Cys Ser Gly Ser Cys Arg Arg Ala Arg Ser Pro His Asp Leu Ser
               45                  50                  55
   Leu Ala Ser Leu Leu Gly Ala Gly Ala Leu Arg Pro Pro Pro Gly Ser
           60                  65                  70
   Arg Pro Val Ser Gln Pro Cys Cys Arg Pro Thr Arg Tyr Glu Ala Val
       75                  80                  85
   Ser Phe Met Asp Val Asn Ser Thr Trp Arg Thr Val Asp Arg Leu Ser
   90                  95                  100                 105
   Ala Thr Ala Cys Gly Cys Leu Gly
                   110
<210> 27
   <211> 145
   <212> PRT
   <213> artificial sequence
<220>
   <223> deltapro human NBN106
   <220>
   <221> signal
   <222> (1)..(39)
   <223>
<220>
   <221> mat_peptide
   <222> (40)..()
   <223>
<400> 27
   Met Glu Leu Gly Leu Gly Gly Leu Ser Thr Leu Ser His Cys Pro Trp
                   -35                 -30                 -25
   Pro Arg Arg Gln Pro Ala Leu Trp Pro Thr Leu Ala Ala Leu Ala Leu
               -20                 -15                 -10
   Leu Ser Ser Val Ala Glu Ala Ala Arg Ala Ala Gly Ala Arg Gly Cys
           -5                  -1  1               5
   Arg Leu Arg Ser Gln Leu Val Pro Val Arg Ala Leu Gly Leu Gly His
   10                  15                  20                  25
   Arg Ser Asp Glu Leu Val Arg Phe Arg Phe Cys Ser Gly Ser Cys Arg
                   30                  35                  40
   Arg Ala Arg Ser Pro His Asp Leu Ser Leu Ala Ser Leu Leu Gly Ala
               45                  50                  55
   Gly Ala Leu Arg Pro Pro Pro Gly Ser Arg Pro Val Ser Gln Pro Cys
           60                  65                  70
   Cys Arg Pro Thr Arg Tyr Glu Ala Val Ser Phe Met Asp Val Asn Ser
       75                  80                  85
   Thr Trp Arg Thr Val Asp Arg Leu Ser Ala Thr Ala Cys Gly Cys Leu
   90                  95                  100                 105
   Gly
<210> 28
   <211> 143
   <212> PRT
   <213> artificial sequence
<220>
<223> deltapro human NBN104
<400> 28
   Met Glu Leu Gly Leu Gly Gly Leu Ser Thr Leu Ser His Cys Pro Trp
   1               5                   10                  15
   Pro Arg Arg Gln Pro Ala Leu Trp Pro Thr Leu Ala Ala Leu Ala Leu
               20                  25                  30
   Leu Ser Ser Val Ala Glu Ala Ala Ala Gly Ala Arg Gly Cys Arg Leu
           35                  40                  45
   Arg Ser Gln Leu Val Pro Val Arg Ala Leu Gly Leu Gly His Arg Ser
       50                  55                  60
   Asp Glu Leu Val Arg Phe Arg Phe Cys Ser Gly Ser Cys Arg Arg Ala
   65                  70                  75                  80
   Arg Ser Pro His Asp Leu Ser Leu Ala Ser Leu Leu Gly Ala Gly Ala
                   85                  90                  95
   Leu Arg Pro Pro Pro Gly Ser Arg Pro Val Ser Gln Pro Cys Cys Arg
               100                 105                 110
   Pro Thr Arg Tyr Glu Ala Val Ser Phe Met Asp Val Asn Ser Thr Trp
           115                 120                 125
   Arg Thr Val Asp Arg Leu Ser Ala Thr Ala Cys Gly Cys Leu Gly
       130                 135                 140
<210> 29
   <211> 141
   <212> PRT
   <213> artificial sequence
<220>
   <223> deltapro human NBN102
   <220>
   <221> SIGNAL
   <222> (1)..(39)
   <223>
<220>
   <221> mat_peptide
   <222> (40)..()
   <223>
<400> 29
   Met Glu Leu Gly Leu Gly Gly Leu Ser Thr Leu Ser His Cys Pro Trp
                   -35                 -30                 -25
   Pro Arg Arg Gln Pro Ala Leu Trp Pro Thr Leu Ala Ala Leu Ala Leu
               -20                 -15                 -10
   Leu Ser Ser Val Ala Glu Ala Gly Ala Arg Gly Cys Arg Leu Arg Ser
           -5                  -1  1               5
   Gln Leu Val Pro Val Arg Ala Leu Gly Leu Gly His Arg Ser Asp Glu
   10                  15                  20                  25
   Leu Val Arg Phe Arg Phe Cys Ser Gly Ser Cys Arg Arg Ala Arg Ser
                   30                  35                  40
   Pro His Asp Leu Ser Leu Ala Ser Leu Leu Gly Ala Gly Ala Leu Arg
               45                  50                  55
   Pro Pro Pro Gly Ser Arg Pro Val Ser Gln Pro Cys Cys Arg Pro Thr
           60                  65                  70
   Arg Tyr Glu Ala Val Ser Phe Met Asp Val Asn Ser Thr Trp Arg Thr
       75                  80                  85
   Val Asp Arg Leu Ser Ala Thr Ala Cys Gly Cys Leu Gly
   90                  95                  100
<210> 30
   <211> 138
   <212> PRT
   <213> artificial sequence
<220>
<223> deltapro human NBN99
   <220>
   <221> SIGNAL
   <222> (1)..(39)
   <223>
<220>
   <221> mat_peptide
   <222> (40)..()
   <223>
<400> 30
   Met Glu Leu Gly Leu Gly Gly Leu Ser Thr Leu Ser His Cys Pro Trp
   -35 -30 -25
   Pro Arg Arg Gln Pro Ala Leu Trp Pro Thr Leu Ala Ala Leu Ala Leu
   -20 -15 -10
   Leu Ser Ser Val Ala Glu Ala Gly Cys Arg Leu Arg Ser Gln Leu Val
   -5 -1 1 5
   Pro Val Arg Ala Leu Gly Leu Gly His Arg Ser Asp Glu Leu Val Arg
   10 15 20 25
   Phe Arg Phe Cys Ser Gly Ser Cys Arg Arg Ala Arg Ser Pro His Asp
   30 35 40
   Leu Ser Leu Ala Ser Leu Leu Gly Ala Gly Ala Leu Arg Pro Pro Pro
   45 50 55
   Gly Ser Arg Pro Val Ser Gln Pro Cys Cys Arg Pro Thr Arg Tyr Glu
   60 65 70
   Ala Val Ser Phe Met Asp Val Asn Ser Thr Trp Arg Thr Val Asp Arg
   75 80 85
   Leu Ser Ala Thr Ala Cys Gly Cys Leu Gly
   90 95
<210> 31
   <211> 159
   <212> PRT
   <213> artificial sequence
<220>
   <223> Murin IgSP- human NBN140
<220>
   <221> SIGNAL
   <222> (1)..(19)
   <223>
<220>
   <221> mat_peptide
   <222> (20)..()
   <223>
<400> 31
   Met Lys Cys Ser Trp Val Ile Phe Phe Leu Met Ala Val Val Thr Gly
   -15 -10 -5
   Val Asn Ser Pro Pro Pro Gln Pro Ser Arg Pro Ala Pro Pro Pro Pro
   -1 1 5 10
   Ala Pro Pro Ser Ala Leu Pro Arg Gly Gly Arg Ala Ala Arg Ala Gly
   15 20 25
   Gly Pro Gly Ser Arg Ala Arg Ala Ala Gly Ala Arg Gly Cys Arg Leu
   30 35 40 45
   Arg Ser Gln Leu Val Pro Val Arg Ala Leu Gly Leu Gly His Arg Ser
   50 55 60
   Asp Glu Leu Val Arg Phe Arg Phe Cys Ser Gly Ser Cys Arg Arg Ala
   65 70 75
   Arg Ser Pro His Asp Leu Ser Leu Ala Ser Leu Leu Gly Ala Gly Ala
   80 85 90
   Leu Arg Pro Pro Pro Gly Ser Arg Pro Val Ser Gln Pro Cys Cys Arg
   95 100 105
   Pro Thr Arg Tyr Glu Ala Val Ser Phe Met Asp Val Asn Ser Thr Trp
   110 115 120 125
   Arg Thr Val Asp Arg Leu Ser Ala Thr Ala Cys Gly Cys Leu Gly
   130 135 140
<210> 32
   <211> 132
   <212> PRT
   <213> artificial sequence
<220>
<223> Murin IgSP- human NBN113
   <220>
   <221> SIGNAL
   <222> (1)..(19)
   <223>
<220>
   <221> mat_peptide
   <222> (20)..()
   <223>
<400> 32
   Met Lys Cys Ser Trp Val Ile Phe Phe Leu Met Ala Val Val Thr Gly
   -15 -10 -5
   Val Asn Ser Ala Gly Gly Pro Gly Ser Arg Ala Arg Ala Ala Gly Ala
   -1 1 5 10
   Arg Gly Cys Arg Leu Arg Ser Gln Leu Val Pro Val Arg Ala Leu Gly
   15 20 25
   Leu Gly His Arg Ser Asp Glu Leu Val Arg Phe Arg Phe Cys Ser Gly
   30 35 40 45
   Ser Cys Arg Arg Ala Arg Ser Pro His Asp Leu Ser Leu Ala Ser Leu
   50 55 60
   Leu Gly Ala Gly Ala Leu Arg Pro Pro Pro Gly Ser Arg Pro Val Ser
   65 70 75
   Gln Pro Cys Cys Arg Pro Thr Arg Tyr Glu Ala Val Ser Phe Met Asp
   80 85 90
   Val Asn Ser Thr Trp Arg Thr Val Asp Arg Leu Ser Ala Thr Ala Cys
   95 100 105
   Gly Cys Leu Gly
   110
<210> 33
   <211> 125
   <212> PRT
   <213> artificial sequence
<220>
<223> Murin IgSP- human NBN106
<220>
   <221> SIGNAL
   <222> (1)..(19)
   <223>
<220>
   <221> mat_peptide
   <222> (20)..()
   <223>
<400> 33
   Met Lys Cys Ser Trp Val Ile Phe Phe Leu Met Ala Val Val Thr Gly
                   -15                 -10                 -5
   Val Asn Ser Ala Arg Ala Ala Gly Ala Arg Gly Cys Arg Leu Arg Ser
           -1  1                   5                   10
   Gln Leu Val Pro Val Arg Ala Leu Gly Leu Gly His Arg Ser Asp Glu
       15                  20                  25
   Leu Val Arg Phe Arg Phe Cys Ser Gly Ser Cys Arg Arg Ala Arg Ser
   30                  35                  40                  45
   Pro His Asp Leu Ser Leu Ala Ser Leu Leu Gly Ala Gly Ala Leu Arg
                   50                  55                  60
   Pro Pro Pro Gly Ser Arg Pro Val Ser Gln Pro Cys Cys Arg Pro Thr
               65                  70                  75
   Arg Tyr Glu Ala Val Ser Phe Met Asp Val Asn Ser Thr Trp Arg Thr
           80                  85                  90
   Val Asp Arg Leu Ser Ala Thr Ala Cys Gly Cys Leu Gly
       95                  100                 105
<210> 34
   <211> 123
   <212> PRT
   <213> artificial sequence
<220>
   <223> Murin IgSP- human NBN1104
   <220>
   <221> SIGNAL
   <222> (1)..(19)
   <223>
<220>
   <221> mat_peptide
   <222> (20)..()
   <223>
<400> 34
   Met Lys Cys Ser Trp Val Ile Phe Phe Leu Met Ala Val Val Thr Gly
                   -15                 -10                 -5
   Val Asn Ser Ala Ala Gly Ala Arg Gly Cys Arg Leu Arg Ser Gln Leu
           -1  1                   5                   10
   Val Pro Val Arg Ala Leu Gly Leu Gly His Arg Ser Asp Glu Leu Val
       15                  20                  25
   Arg Phe Arg Phe Cys Ser Gly Ser Cys Arg Arg Ala Arg Ser Pro His
   30                  35                  40                  45
   Asp Leu Ser Leu Ala Ser Leu Leu Gly Ala Gly Ala Leu Arg Pro Pro
                   50                  55                  60
   Pro Gly Ser Arg Pro Val Ser Gln Pro Cys Cys Arg Pro Thr Arg Tyr
               65                  70                  75
   Glu Ala Val Ser Phe Met Asp Val Asn Ser Thr Trp Arg Thr Val Asp
           80                  85                  90
   Arg Leu Ser Ala Thr Ala Cys Gly Cys Leu Gly
       95                  100
<210> 35
   <211> 121
   <212> PRT
   <213> artificial sequence
<220>
   <223> Murin IgSP- human NBN102
   <220>
   <221> SIGNAL
   <222> (1)..(19)
   <223>
<220>
   <221> mat_peptide
   <222> (20)..()
   <223>
<400> 35
   Met Lys Cys Ser Trp Val Ile Phe Phe Leu Met Ala Val Val Thr Gly
                   -15                 -10                 -5
   Val Asn Ser Gly Ala Arg Gly Cys Arg Leu Arg Ser Gln Leu Val Pro
           -1  1               5                   10
   Val Arg Ala Leu Gly Leu Gly His Arg Ser Asp Glu Leu Val Arg Phe
       15                  20                  25
   Arg Phe Cys Ser Gly Ser Cys Arg Arg Ala Arg Ser Pro His Asp Leu
   30                  35                  40                  45
   Ser Leu Ala Ser Leu Leu Gly Ala Gly Ala Leu Arg Pro Pro Pro Gly
                   50                  55                  60
   Ser Arg Pro Val Ser Gln Pro Cys Cys Arg Pro Thr Arg Tyr Glu Ala
               65                  70                  75
   Val Ser Phe Met Asp Val Asn Ser Thr Trp Arg Thr Val Asp Arg Leu
           80                  85                  90
   Ser Ala Thr Ala Cys Gly Cys Leu Gly
       95                  100
<210> 36
   <211> 118
   <212> PRT
   <213> artificial sequence
<220>
   <223> Murin IgSP- human NBN99
   <220>
   <221> SIGNAL
   <222> (1)..(19)
   <223>
<220>
   <221> mat_peptide
   <222> (20)..()
   <223>
<400> 36
   Met Lys Cys Ser Trp Val Ile Phe Phe Leu Met Ala Val Val Thr Gly
                   -15                 -10                 -5
   Val Asn Ser Gly Cys Arg Leu Arg Ser Gln Leu Val Pro Val Arg Ala
           -1  1               5                   10
   Leu Gly Leu Gly His Arg Ser Asp Glu Leu Val Arg Phe Arg Phe Cys
       15                  20                  25
   Ser Gly Ser Cys Arg Arg Ala Arg Ser Pro His Asp Leu Ser Leu Ala
   30                  35                  40                  45
   Ser Leu Leu Gly Ala Gly Ala Leu Arg Pro Pro Pro Gly Ser Arg Pro
                   50                  55                  60
   Val Ser Gln Pro Cys Cys Arg Pro Thr Arg Tyr Glu Ala Val Ser Phe
               65                  70                  75
   Met Asp Val Asn Ser Thr Trp Arg Thr Val Asp Arg Leu Ser Ala Thr
           80                  85                  90
   Ala Cys Gly Cys Leu Gly
       95
<210> 37
   <211> 18
   <212> PRT
   <213> Rattus rattus
<400> 37
   Met Lys Trp Val Thr Phe Leu Leu Leu Leu Phe Ile Ser Gly Ser Ala
   1               5                   10                  15
   Phe Ser
<210> 38
   <211> 158
   <212> PRT
   <213> artificial sequence
<220>
   <223> Rat albumin signal peptide - NBN140
   <220>
   <221> SIGNAL
   <222> (1)..(18)
   <223>
<220>
   <221> mat_peptide
   <222> (19)..()
   <223>
<400> 38
   Met Lys Trp Val Thr Phe Leu Leu Leu Leu Phe Ile Ser Gly Ser Ala
               -15                 -10                 -5
   Phe Ser Pro Pro Pro Gln Pro Ser Arg Pro Ala Pro Pro Pro Pro Ala
       -1  1               5                   10
   Pro Pro Ser Ala Leu Pro Arg Gly Gly Arg Ala Ala Arg Ala Gly Gly
   15                  20                  25                  30
   Pro Gly Ser Arg Ala Arg Ala Ala Gly Ala Arg Gly Cys Arg Leu Arg
                   35                  40                  45
   Ser Gln Leu Val Pro Val Arg Ala Leu Gly Leu Gly His Arg Ser Asp
               50                  55                  60
   Glu Leu Val Arg Phe Arg Phe Cys Ser Gly Ser Cys Arg Arg Ala Arg
           65                  70                  75
   Ser Pro His Asp Leu Ser Leu Ala Ser Leu Leu Gly Ala Gly Ala Leu
       80                  85                  90
   Arg Pro Pro Pro Gly Ser Arg Pro Val Ser Gln Pro Cys Cys Arg Pro
   95                  100                 105                 110
   Thr Arg Tyr Glu Ala Val Ser Phe Met Asp Val Asn Ser Thr Trp Arg
                   115                 120                 125
   Thr Val Asp Arg Leu Ser Ala Thr Ala Cys Gly Cys Leu Gly
               130                 135                 140
<210> 39
   <211> 131
   <212> PRT
   <213> artificial sequence
<220>
   <223> Rat albumin signal peptide - NBN113
   <220>
   <221> SIGNAL
   <222> (1)..(18)
   <223>
<220>
   <221> mat_peptide
   <222> (19)..()
   <223>
<400> 39
   Met Lys Trp Val Thr Phe Leu Leu Leu Leu Phe Ile Ser Gly Ser Ala
   -15 -10 -5
   Phe Ser Ala Gly Gly Pro Gly Ser Arg Ala Arg Ala Ala Gly Ala Arg
   -1 1 5 10
   Gly Cys Arg Leu Arg Ser Gln Leu Val Pro Val Arg Ala Leu Gly Leu
   15 20 25 30
   Gly His Arg Ser Asp Glu Leu Val Arg Phe Arg Phe Cys Ser Gly Ser
   35 40 45
   Cys Arg Arg Ala Arg Ser Pro His Asp Leu Ser Leu Ala Ser Leu Leu
   50 55 60
   Gly Ala Gly Ala Leu Arg Pro Pro Pro Gly Ser Arg Pro Val Ser Gln
   65 70 75
   Pro Cys Cys Arg Pro Thr Arg Tyr Glu Ala Val Ser Phe Met Asp Val
   80 85 90
   Asn Ser Thr Trp Arg Thr Val Asp Arg Leu Ser Ala Thr Ala Cys Gly
   95 100 105 110
   Cys Leu Gly
<210> 40
   <211> 124
   <212> PRT
   <213> artificial sequence
<220>
   <223> Rat albumin signal peptide - NBN106
   <220>
   <221> SIGNAL
   <222> (1) .. (18)
   <223>
<220>
   <221> mat_peptide
   <222> (19)..()
   <223>
<400> 40
   Met Lys Trp Val Thr Phe Leu Leu Leu Leu Phe Ile Ser Gly Ser Ala
   -15 -10 -5
   Phe Ser Ala Arg Ala Ala Gly Ala Arg Gly Cys Arg Leu Arg Ser Gln
   -1 1 5 10
   Leu Val Pro Val Arg Ala Leu Gly Leu Gly His Arg Ser Asp Glu Leu
   15 20 25 30
   Val Arg Phe Arg Phe Cys Ser Gly Ser Cys Arg Arg Ala Arg Ser Pro
   35 40 45
   His Asp Leu Ser Leu Ala Ser Leu Leu Gly Ala Gly Ala Leu Arg Pro
   50 55 60
   Pro Pro Gly Ser Arg Pro Val Ser Gln Pro Cys Cys Arg Pro Thr Arg
   65 70 75
   Tyr Glu Ala Val Ser Phe Met Asp Val Asn Ser Thr Trp Arg Thr Val
   80 85 90
   Asp Arg Leu Ser Ala Thr Ala Cys Gly Cys Leu Gly
   95 100 105
<210> 41
   <211> 122
   <212> PRT
   <213> artificial sequence
<220>
   <223> Rat albumin signal peptide - NBN104
   <220>
   <221> SIGNAL
   <222> (1)..(18)
   <223>
<220>
   <221> mat_peptide
   <222> (19)..()
   <223>
<400> 41
   Met Lys Trp Val Thr Phe Leu Leu Leu Leu Phe Ile Ser Gly Ser Ala
   -15 -10 -5
   Phe Ser Ala Ala Gly Ala Arg Gly Cys Arg Leu Arg Ser Gln Leu Val
   -1 1 5 10
   Pro Val Arg Ala Leu Gly Leu Gly His Arg Ser Asp Glu Leu Val Arg
   15 20 25 30
   Phe Arg Phe Cys Ser Gly Ser Cys Arg Arg Ala Arg Ser Pro His Asp
   35 40 45
   Leu Ser Leu Ala Ser Leu Leu Gly Ala Gly Ala Leu Arg Pro Pro Pro
   50 55 60
   Gly Ser Arg Pro Val Ser Gln Pro Cys Cys Arg Pro Thr Arg Tyr Glu
   65 70 75
   Ala Val Ser Phe Met Asp Val Asn Ser Thr Trp Arg Thr Val Asp Arg
   80 85 90
   Leu Ser Ala Thr Ala Cys Gly Cys Leu Gly
   95 100
<210> 42
   <211> 120
   <212> PRT
   <213> artificial sequence
<220>
   <223> Rat albumin signal peptide - NBN102
   <220>
   <221> SIGNAL
   <222> (1)..(18)
   <223>
<220>
   <221> mat_peptide
   <222> (19)..()
   <223>
<400> 42
   Met Lys Trp Val Thr Phe Leu Leu Leu Leu Phe Ile Ser Gly Ser Ala
   -15 -10 -5
   Phe Ser Gly Ala Arg Gly Cys Arg Leu Arg Ser Gln Leu Val Pro Val
   -1 1 5 10
   Arg Ala Leu Gly Leu Gly His Arg Ser Asp Glu Leu Val Arg Phe Arg
   15 20 25 30
   Phe Cys Ser Gly Ser Cys Arg Arg Ala Arg Ser Pro His Asp Leu Ser
   35 40 45
   Leu Ala Ser Leu Leu Gly Ala Gly Ala Leu Arg Pro Pro Pro Gly Ser
   50 55 60
   Arg Pro Val Ser Gln Pro Cys Cys Arg Pro Thr Arg Tyr Glu Ala Val
   65 70 75
   Ser Phe Met Asp Val Asn Ser Thr Trp Arg Thr Val Asp Arg Leu Ser
   80 85 90
   Ala Thr Ala Cys Gly Cys Leu Gly
   95 100
<210> 43
   <211> 117
   <212> PRT
   <213> artificial sequence
<220>
   <223> Rat albumin signal peptide -NBN99
   <220>
   <221> SIGNAL
   <222> (1)..(18)
   <223>
<220>
   <221> mat_peptide
   <222> (19)..()
   <223>
<400> 43
   Met Lys Trp Val Thr Phe Leu Leu Leu Leu Phe Ile Ser Gly Ser Ala
   -15 -10 -5
   Phe Ser Gly Cys Arg Leu Arg Ser Gln Leu Val Pro Val Arg Ala Leu
   -1 1 5 10
   Gly Leu Gly His Arg Ser Asp Glu Leu Val Arg Phe Arg Phe Cys Ser
   15 20 25 30
   Gly Ser Cys Arg Arg Ala Arg Ser Pro His Asp Leu Ser Leu Ala Ser
   35 40 45
   Leu Leu Gly Ala Gly Ala Leu Arg Pro Pro Pro Gly Ser Arg Pro Val
   50 55 60
   Ser Gln Pro Cys Cys Arg Pro Thr Arg Tyr Glu Ala Val Ser Phe Met
   65 70 75
   Asp Val Asn Ser Thr Trp Arg Thr Val Asp Arg Leu Ser Ala Thr Ala
   80 85 90
   Cys Gly Cys Leu Gly
   95
<210> 44
   <211> 19
   <212> PRT
   <213> artificial sequence
<220>
   <223> Modified rat Albumin signal peptide
   <400> 44
   Met Lys Trp Val Thr Phe Leu Leu Phe Leu Leu Phe Ile Ser Gly Asp
   1 5 10 15
   Ala Phe Ala
<210> 45
   <211> 159
   <212> PRT
   <213> artificial sequence
<220>
   <223> Modified rat albumin signal peptide - NBN140
   <220>
   <221> SIGNAL
   <222> (1)..(19)
   <223>
<220>
   <221> mat_peptide
   <222> (20)..()
   <223>
<400> 45
   Met Lys Trp Val Thr Phe Leu Leu Phe Leu Leu Phe Ile Ser Gly Asp
   -15 -10 -5
   Ala Phe Ala Pro Pro Pro Gln Pro Ser Arg Pro Ala Pro Pro Pro Pro
   -1 1 5 10
   Ala Pro Pro Ser Ala Leu Pro Arg Gly Gly Arg Ala Ala Arg Ala Gly
   15 20 25
   Gly Pro Gly Ser Arg Ala Arg Ala Ala Gly Ala Arg Gly Cys Arg Leu
   30 35 40 45
   Arg Ser Gln Leu Val Pro Val Arg Ala Leu Gly Leu Gly His Arg Ser
   50 55 60
   Asp Glu Leu Val Arg Phe Arg Phe Cys Ser Gly Ser Cys Arg Arg Ala
   65 70 75
   Arg Ser Pro His Asp Leu Ser Leu Ala Ser Leu Leu Gly Ala Gly Ala
   80 85 90
   Leu Arg Pro Pro Pro Gly Ser Arg Pro Val Ser Gln Pro Cys Cys Arg
   95 100 105
   Pro Thr Arg Tyr Glu Ala Val Ser Phe Met Asp Val Asn Ser Thr Trp
   110 115 120 125
   Arg Thr Val Asp Arg Leu Ser Ala Thr Ala Cys Gly Cys Leu Gly
   130 135 140
<210> 46
   <211> 132
   <212> PRT
   <213> artificial sequence
<220>
   <223> Modified rat albumin signal peptide - NBN113
   **<220>**
   <221> SIGNAL
   <222> (1)..(19)
   <223>
<220>
   <221> mat_peptide
   <222> (20)..()
   <223>
<400> 46
   Met Lys Trp Val Thr Phe Leu Leu Phe Leu Leu Phe Ile Ser Gly Asp
                   -15                 -10                 -5
   Ala Phe Ala Ala Gly Gly Pro Gly Ser Arg Ala Arg Ala Ala Gly Ala
           -1  1               5                   10
   Arg Gly Cys Arg Leu Arg Ser Gln Leu Val Pro Val Arg Ala Leu Gly
       15                  20                  25
   Leu Gly His Arg Ser Asp Glu Leu Val Arg Phe Arg Phe Cys Ser Gly
   30                  35                  40                  45
   Ser Cys Arg Arg Ala Arg Ser Pro His Asp Leu Ser Leu Ala Ser Leu
                   50                  55                  60
   Leu Gly Ala Gly Ala Leu Arg Pro Pro Pro Gly Ser Arg Pro Val Ser
               65                  70                  75
   Gln Pro Cys Cys Arg Pro Thr Arg Tyr Glu Ala Val Ser Phe Met Asp
           80                  85                  90
   Val Asn Ser Thr Trp Arg Thr Val Asp Arg Leu Ser Ala Thr Ala Cys
       95                  100                 105
   Gly Cys Leu Gly
   110
<210> 47
   <211> 125
   <212> PRT
   <213> artificial sequence
<220>
   <223> Modified rat albumin signal peptide - NBN106
   <220>
   <221> SIGNAL
   <222> (1)..(19)
   <223>
   <220>
   <221> mat_peptide
   <222> (20)..()
   <223>
<400> 47
   Met Lys Trp Val Thr Phe Leu Leu Phe Leu Leu Phe Ile Ser Gly Asp
                   -15                 -10                 -5
   Ala Phe Ala Ala Arg Ala Ala Gly Ala Arg Gly Cys Arg Leu Arg Ser
           -1  1               5                   10
   Gln Leu Val Pro Val Arg Ala Leu Gly Leu Gly His Arg Ser Asp Glu
       15                  20                  25
   Leu Val Arg Phe Arg Phe Cys Ser Gly Ser Cys Arg Arg Ala Arg Ser
   30                  35                  40                  45
   Pro His Asp Leu Ser Leu Ala Ser Leu Leu Gly Ala Gly Ala Leu Arg
                   50                  55                  60
   Pro Pro Pro Gly Ser Arg Pro Val Ser Gln Pro Cys Cys Arg Pro Thr
               65                  70                  75
   Arg Tyr Glu Ala Val Ser Phe Met Asp Val Asn Ser Thr Trp Arg Thr
           80                  85                  90
   Val Asp Arg Leu Ser Ala Thr Ala Cys Gly Cys Leu Gly
       95                  100                 105
<210> 48
   <211> 123
   <212> PRT
   <213> artificial sequence
<220>
   <223> Modified rat albumin signal peptide - NBN104
   <220>
   <221> SIGNAL
   <222> (1)..(19)
   <223>
<220>
   <221> mat_peptide
   <222> (20)..0
   <223>
<400> 48
   Met Lys Trp Val Thr Phe Leu Leu Phe Leu Leu Phe Ile Ser Gly Asp
                   -15                 -10                 -5
   Ala Phe Ala Ala Ala Gly Ala Arg Gly Cys Arg Leu Arg Ser Gln Leu
           -1  1               5                   10
   Val Pro Val Arg Ala Leu Gly Leu Gly His Arg Ser Asp Glu Leu Val
       15                  20                  25
   Arg Phe Arg Phe Cys Ser Gly Ser Cys Arg Arg Ala Arg Ser Pro His
   30                  35                  40                  45
   Asp Leu Ser Leu Ala Ser Leu Leu Gly Ala Gly Ala Leu Arg Pro Pro
                   50                  55                  60
   Pro Gly Ser Arg Pro Val Ser Gln Pro Cys Cys Arg Pro Thr Arg Tyr
               65                  70                  75
   Glu Ala Val Ser Phe Met Asp Val Asn Ser Thr Trp Arg Thr Val Asp
           80                  85                  90
   Arg Leu Ser Ala Thr Ala Cys Gly Cys Leu Gly
       95                  100
<210> 49
   <211> 121
   <212> PRT
   <213> artificial sequence
<220>
   <223> Modified rat albumin signal peptide - NBN102
   <220>
   <221> SIGNAL
   <222> (1)..(19)
   <223>
<220>
   <221> mat_peptide
   <222> (20)..()
   <223>
<400> 49
   Met Lys Trp Val Thr Phe Leu Leu Phe Leu Leu Phe Ile Ser Gly Asp
                   -15                 -10                 -5
   Ala Phe Ala Gly Ala Arg Gly Cys Arg Leu Arg Ser Gln Leu Val Pro
           -1  1               5                   10
   Val Arg Ala Leu Gly Leu Gly His Arg Ser Asp Glu Leu Val Arg Phe
       15                  20                  25
   Arg Phe Cys Ser Gly Ser Cys Arg Arg Ala Arg Ser Pro His Asp Leu
   30                  35                  40                  45
   Ser Leu Ala Ser Leu Leu Gly Ala Gly Ala Leu Arg Pro Pro Pro Gly
                   50                  55                  60
   Ser Arg Pro Val Ser Gln Pro Cys Cys Arg Pro Thr Arg Tyr Glu Ala
               65                  70                  75
   Val Ser Phe Met Asp Val Asn Ser Thr Trp Arg Thr Val Asp Arg Leu
           80                  85                  90
   Ser Ala Thr Ala Cys Gly Cys Leu Gly
       95                  100
<210> 50
   <211> 118
   <212> PRT
   <213> artificial sequence
<220>
   <223> Modified rat albumin signal peptide - NBN99
   <220>
   <221> SIGNAL
   <222> (1)..(19)
   <223>
<220>
   <221> mat_peptide
   <222> (20)..()
   <223>
<400> 50
   Met Lys Trp Val Thr Phe Leu Leu Phe Leu Leu Phe Ile Ser Gly Asp
                   -15                 -10                 -5
   Ala Phe Ala Gly Cys Arg Leu Arg Ser Gln Leu Val Pro Val Arg Ala
           -1  1               5                   10
   Leu Gly Leu Gly His Arg Ser Asp Glu Leu Val Arg Phe Arg Phe Cys
       15                  20                  25
   Ser Gly Ser Cys Arg Arg Ala Arg Ser Pro His Asp Leu Ser Leu Ala
   30                  35                  40                  45
   Ser Leu Leu Gly Ala Gly Ala Leu Arg Pro Pro Pro Gly Ser Arg Pro
                   50                  55                  60
   Val Ser Gln Pro Cys Cys Arg Pro Thr Arg Tyr Glu Ala Val Ser Phe
               65                  70                  75
   Met Asp Val Asn Ser Thr Trp Arg Thr Val Asp Arg Leu Ser Ala Thr
           80                  85                  90
   Ala Cys Gly Cys Leu Gly
       95
<210> 51
   <211> 26
   <212> PRT
   <213> Homo sapiens
<400> 51
   Met Ala Thr Gly Ser Arg Thr Ser Leu Leu Leu Ala Phe Gly Leu Leu
   1               5                   10                  15
   Cys Leu Ser Trp Leu Gln Glu Gly Ser Ala
               20                  25
<210> 52
   <211> 166
   <212> PRT
   <213> artificial sequence
<220>
   <223> Human Growth Hormone SP- NBN140
   <220>
   <221> SIGNAL
   <222> (1)..(26)
   <223>
<220>
   <221> mat_peptide
   <222> (27).. ()
   <223>
<400> 52
   Met Ala Thr Gly Ser Arg Thr Ser Leu Leu Leu Ala Phe Gly Leu Leu
   -25 -20 -15
   Cys Leu Ser Trp Leu Gln Glu Gly Ser Ala Pro Pro Pro Gln Pro Ser
   -10 -5 -1 1 5
   Arg Pro Ala Pro Pro Pro Pro Ala Pro Pro Ser Ala Leu Pro Arg Gly
   10 15 20
   Gly Arg Ala Ala Arg Ala Gly Gly Pro Gly Ser Arg Ala Arg Ala Ala
   25 30 35
   Gly Ala Arg Gly Cys Arg Leu Arg Ser Gln Leu Val Pro Val Arg Ala
   40 45 50
   Leu Gly Leu Gly His Arg Ser Asp Glu Leu Val Arg Phe Arg Phe Cys
   55 60 65 70
   Ser Gly Ser Cys Arg Arg Ala Arg Ser Pro His Asp Leu Ser Leu Ala
   75 80 85
   Ser Leu Leu Gly Ala Gly Ala Leu Arg Pro Pro Pro Gly Ser Arg Pro
   90 95 100
   Val Ser Gln Pro Cys Cys Arg Pro Thr Arg Tyr Glu Ala Val Ser Phe
   105 110 115
   Met Asp Val Asn Ser Thr Trp Arg Thr Val Asp Arg Leu Ser Ala Thr
   120 125 130
   Ala Cys Gly Cys Leu Gly
   135 140
<210> 53
   <211> 139
   <212> PRT
   <213> artificial sequence
<220>
   <223> Human Growth Hormone SP- NBN113
   <220>
   <221> SIGNAL
   <222> (1)..(26)
   <223>
<220>
   <221> mat_peptide
   <222> (27)..()
   <223>
<400> 53
   Met Ala Thr Gly Ser Arg Thr Ser Leu Leu Leu Ala Phe Gly Leu Leu
       -25                 -20                 -15
   Cys Leu Ser Trp Leu Gln Glu Gly Ser Ala Ala Gly Gly Pro Gly Ser
   -10                 -5                  -1  1               5
   Arg Ala Arg Ala Ala Gly Ala Arg Gly Cys Arg Leu Arg Ser Gln Leu
               10                  15                  20
   Val Pro Val Arg Ala Leu Gly Leu Gly His Arg Ser Asp Glu Leu Val
           25                  30                  35
   Arg Phe Arg Phe Cys Ser Gly Ser Cys Arg Arg Ala Arg Ser Pro His
       40                  45                  50
   Asp Leu Ser Leu Ala Ser Leu Leu Gly Ala Gly Ala Leu Arg Pro Pro
   55                  60                  65                  70
   Pro Gly Ser Arg Pro Val Ser Gln Pro Cys Cys Arg Pro Thr Arg Tyr
                   75                  80                  85
   Glu Ala Val Ser Phe Met Asp Val Asn Ser Thr Trp Arg Thr Val Asp
               90                  95                  100
   Arg Leu Ser Ala Thr Ala Cys Gly Cys Leu Gly
           105                 110
<210> 54
   <211> 132
   <212> PRT
   <213> artificial sequence
<220>
   <223> Human Growth Hormone SP- NBN106
   <220>
   <221> SIGNAL
   <222> (1)..(26)
   <223>
<220>
   <221> mat_peptide
   <222> (27).,()
   <223>
<400> 54
   Met Ala Thr Gly Ser Arg Thr Ser Leu Leu Leu Ala Phe Gly Leu Leu
       -25                 -20                 -15
   Cys Leu Ser Trp Leu Gln Glu Gly Ser Ala Ala Arg Ala Ala Gly Ala
   -10                 -5                  -1  1               5
   Arg Gly Cys Arg Leu Arg Ser Gln Leu Val Pro Val Arg Ala Leu Gly
               10                  15                  20
   Leu Gly His Arg Ser Asp Glu Leu Val Arg Phe Arg Phe Cys Ser Gly
           25                  30                  35
   Ser Cys Arg Arg Ala Arg Ser Pro His Asp Leu Ser Leu Ala Ser Leu
       40                  45                  50
   Leu Gly Ala Gly Ala Leu Arg Pro Pro Pro Gly Ser Arg Pro Val Ser
   55                  60                  65                  70
   Gln Pro Cys Cys Arg Pro Thr Arg Tyr Glu Ala Val Ser Phe Met Asp
                   75                  80                  85
   Val Asn Ser Thr Trp Arg Thr Val Asp Arg Leu Ser Ala Thr Ala Cys
               90                  95                  100
   Gly Cys Leu Gly
           105
<210> 55
   <211> 130
   <212> PRT
   <213> artificial sequence
<220>
   <223> Human Growth Hormone SP- NBN104
   <220>
   <221> SIGNAL
   <222> (1)..(26)
   <223>
<220>
   <221> mat_peptide
   <222> (27)..()
   <223>
<400> 55
   Met Ala Thr Gly Ser Arg Thr Ser Leu Leu Leu Ala Phe Gly Leu Leu
       -25                 -20                 -15
   Cys Leu Ser Trp Leu Gln Glu Gly Ser Ala Ala Ala Gly Ala Arg Gly
   -10                 -5                  -1  1               5
   Cys Arg Leu Arg Ser Gln Leu Val Pro Val Arg Ala Leu Gly Leu Gly
               10                  15                  20
   His Arg Ser Asp Glu Leu Val Arg Phe Arg Phe Cys Ser Gly Ser Cys
           25                  30                  35
   Arg Arg Ala Arg Ser Pro His Asp Leu Ser Leu Ala Ser Leu Leu Gly
       40                  45                  50
   Ala Gly Ala Leu Arg Pro Pro Pro Gly Ser Arg Pro Val Ser Gln Pro
   55                  60                  65                  70
   Cys Cys Arg Pro Thr Arg Tyr Glu Ala Val Ser Phe Met Asp Val Asn
                   75                  80                  85
   Ser Thr Trp Arg Thr Val Asp Arg Leu Ser Ala Thr Ala Cys Gly Cys
               90                  95                  100
   Leu Gly
<210> 56
   <211> 128
   <212> PRT
   <213> artificial sequence
<220>
   <223> Human Growth Hormone SP- NBN102
   <220>
   <221> SIGNAL
   <222> (1)..(26)
   <223>
<220>
   <221> mat_peptide
   <222> (27)..()
   <223>
<400> 56
   Met Ala Thr Gly Ser Arg Thr Ser Leu Leu Leu Ala Phe Gly Leu Leu
   -25 -20 -15
   Cys Leu Ser Trp Leu Gln Glu Gly Ser Ala Gly Ala Arg Gly Cys Arg
   -10 -5 -1 1 5
   Leu Arg Ser Gln Leu Val Pro Val Arg Ala Leu Gly Leu Gly His Arg
   10 15 20
   Ser Asp Glu Leu Val Arg Phe Arg Phe Cys Ser Gly Ser Cys Arg Arg
   25 30 35
   Ala Arg Ser Pro His Asp Leu Ser Leu Ala Ser Leu Leu Gly Ala Gly
   40 45 50
   Ala Leu Arg Pro Pro Pro Gly Ser Arg Pro Val Ser Gln Pro Cys Cys
   55 60 65 70
   Arg Pro Thr Arg Tyr Glu Ala Val Ser Phe Met Asp Val Asn Ser Thr
   75 80 85
   Trp Arg Thr Val Asp Arg Leu Ser Ala Thr Ala Cys Gly Cys Leu Gly
   90 95 100
<210> 57
   <211> 125
   <212> PRT
   <213> artificial sequence
<220>
   <223> Human Growth Hormone SP- NBN99
   <220>
   <221> SIGNAL
   <222> (1)..(26)
   <223>
<220>
   <221> mat_peptide
   <222> (27)..()
   <223>
<400> 57
   Met Ala Thr Gly Ser Arg Thr Ser Leu Leu Leu Ala Phe Gly Leu Leu
   -25 -20 -15
   Cys Leu Ser Trp Leu Gln Glu Gly Ser Ala Gly Cys Arg Leu Arg Ser
   -10 -5 -1 1 5
   Gln Leu Val Pro Val Arg Ala Leu Gly Leu Gly His Arg Ser Asp Glu
   10 15 20
   Leu Val Arg Phe Arg Phe Cys Ser Gly Ser Cys Arg Arg Ala Arg Ser
   25 30 35
   Pro His Asp Leu Ser Leu Ala Ser Leu Leu Gly Ala Gly Ala Leu Arg
   40 45 50
   Pro Pro Pro Gly Ser Arg Pro Val Ser Gln Pro Cys Cys Arg Pro Thr
   55 60 65 70
   Arg Tyr Glu Ala Val Ser Phe Met Asp Val Asn Ser Thr Trp Arg Thr
   75 80 85
   Val Asp Arg Leu Ser Ala Thr Ala Cys Gly Cys Leu Gly
   90 95
<210> 58
   <211> 312
   <212> DNA
   <213> Homo sapiens
<220>
   <221> misc_feature
   <223> NBN104 nucleotide sequence
<400> 58
   gcagcggggg cgcggggctg ccgcctgcgc tcgcagctgg tgccggtgcg cgcgctcggc 60
   ctgggccacc gctccgacga gctggtgcgt ttccgcttct gcagcggctc ctgccgccgc 120
   gcgcgctctc cacacgacct cagcctggcc agcctactgg gcgccggggc cctgcgaccg 180
   cccccgggct cccggcccgt cagccagccc tgctgccgac ccacgcgcta cgaagcggtc 240
   tccttcatgg acgtcaacag cacctggaga accgtggacc gcctctccgc caccgcctgc 300
   ggctgcctgg gc 312
<210> 59
   <211> 312
   <212> DNA
   <213> artificial sequence
<220>
   <223> synthetic NBN104 nucleotide sequence
   <400> 59
   gccgccggcg ctcgaggctg ccggctgcgg tcccagctgg tgcctgtgcg ggccctgggc 60
   ctgggccacc ggtccgacga gctggtgcgg ttccggttct gctccggctc ctgccggcgg 120
   gcccggtccc ctcacgacct gtccctggcc tccctgctgg gcgccggcgc cctgcggcct 180
   cctcctggct cccggcctgt gtcccagcct tgctgccggc ctacccggta cgaggccgtg 240
   tccttcatgg acgtgaactc cacctggcgg accgtggacc ggctgtccgc caccgcctgc 300
   ggctgcctgg gc 312
<210> 60
   <211> 303
   <212> DNA
   <213> artificial sequence
<220>
   <223> sequence within pNBN026-35 encoding NBN99
<400> 60
   cgaggctgcc ggctgcggtc ccagctggtg cctgtgcggg ccctgggcct gggccaccgg 60
   tccgacgagc tggtgcggtt ccggttctgc tccggctcct gccggcgggc ccggtcccct 120
   cacgacctgt ccctggcctc cctgctgggc gccggcgccc tgcggcctcc tcctggctcc 180
   cggcctgtgt cccagccttg ctgccggcct acccggtacg aggccgtgtc cttcatggac 240
   gtgaactcca cctggcggac cgtggaccgg ctgtccgcca ccgcctgcgg ctgcctgggc 300
   tga 303
<210> 61
   <211> 426
   <212> DNA
   <213> artificial sequence
<220>
   <223> synthetic SP-NBN104 synthetic
<400> 61
   atgagctggg cctgggcggc ctgtccaccc tgtcccactg cccttggcct cggcggcagt 60
   gccctgtggc ctaccctggc cgccctggcc ctgctgtcct ccgtggccga ggccgccgcc 120
   ggcgctcgag gctgccggct gcggtcccag ctggtgcctg tgcgggccct gggcctgggc 180
   caccggtccg acgagctggt gcggttccgg ttctgctccg gctcctgccg gcgggcccgg 240
   tcccctcacg acctgtccct ggcctccctg ctgggcgccg gcgccctgcg gcctcctcct 300
   ggctcccggc ctgtgtccca gccttgctgc cggcctaccc ggtacgaggc cgtgtccttc 360
   atggacgtga actccacctg gcggaccgtg gaccggctgt ccgccaccgc ctgcggctgc 420
   ctgggc 426
<210> 62
   <211> 142
   <212> PRT
   <213> artificial sequence
<220>
   <223> synthetic SP-NBN104 synthetic amino acid sequence
<400> 62
   Met Ser Trp Ala Trp Ala Ala Cys Pro Pro Cys Pro Thr Ala Leu Gly
   1 5 10 15
   Leu Gly Gly Ser Ala Leu Trp Pro Thr Leu Ala Ala Leu Ala Leu Leu
   20 25 30
   Ser Ser Val Ala Glu Ala Ala Ala Gly Ala Arg Gly Cys Arg Leu Arg
   35 40 45
   Ser Gln Leu Val Pro Val Arg Ala Leu Gly Leu Gly His Arg Ser Asp
   50 55 60
   Glu Leu Val Arg Phe Arg Phe Cys Ser Gly Ser Cys Arg Arg Ala Arg
   65 70 75 80
   Ser Pro His Asp Leu Ser Leu Ala Ser Leu Leu Gly Ala Gly Ala Leu
   85 90 95
   Arg Pro Pro Pro Gly Ser Arg Pro Val Ser Gln Pro Cys Cys Arg Pro
   100 105 110
   Thr Arg Tyr Glu Ala Val Ser Phe Met Asp Val Asn Ser Thr Trp Arg
   115 120 125
   Thr Val Asp Arg Leu Ser Ala Thr Ala Cys Gly Cys Leu Gly
   130 135 140
<210> 63
   <211> 432
   <212> DNA
   <213> artificial sequence
<220>
   <223> NBN signal peptide - synthetic NBN104
<400> 63
   atggagctgg gcctgggcgg cctgtccacc ctgtcccact gcccttggcc tcggcggcag    60
   cctgccctgt ggcctaccct ggccgccctg gccctgctgt cctccgtggc cgaggccgcc   120
   gccggcgctc gaggctgccg gctgcggtcc cagctggtgc ctgtgcgggc cctgggcctg   180
   ggccaccggt ccgacgagct ggtgcggttc cggttctgct ccggctcctg ccggcgggcc   240
   cggtcccctc acgacctgtc cctggcctcc ctgctgggcg ccggcgccct gcggcctcct   300
   cctggctccc ggcctgtgtc ccagccttgc tgccggccta cccggtacga ggccgtgtcc   360
   ttcatggacg tgaactccac ctggcggacc gtggaccggc tgtccgccac cgcctgcggc   420
   tgcctgggct ga                                                       432
<210> 64
   <211> 143
   <212> PRT
   <213> artificial sequence
<220>
   <223> NBN signal peptide - NBN104 synthetic sequence
   <400> 64
   Met Glu Leu Gly Leu Gly Gly Leu Ser Thr Leu Ser His Cys Pro Trp
   1               5                   10                  15
   Pro Arg Arg Gln Pro Ala Leu Trp Pro Thr Leu Ala Ala Leu Ala Leu
               20                  25                  30
   Leu Ser Ser Val Ala Glu Ala Ala Ala Gly Ala Arg Gly Cys Arg Leu
           35                  40                  45
   Arg Ser Gln Leu Val Pro Val Arg Ala Leu Gly Leu Gly His Arg Ser
       50                  55                  60
   Asp Glu Leu Val Arg Phe Arg Phe Cys Ser Gly Ser Cys Arg Arg Ala
   65                  70                  75                  80
   Arg Ser Pro His Asp Leu Ser Leu Ala Ser Leu Leu Gly Ala Gly Ala
                   85                  90                  95
   Leu Arg Pro Pro Pro Gly Ser Arg Pro Val Ser Gln Pro Cys Cys Arg
               100                 105                 110
   Pro Thr Arg Tyr Glu Ala Val Ser Phe Met Asp Val Asn Ser Thr Trp
           115                 120                 125
   Arg Thr Val Asp Arg Leu Ser Ala Thr Ala Cys Gly Cys Leu Gly
       130                 135                 140
<210> 65.
   <211> 369
   <212> DNA
   <213> artificial sequence
<220>
   <223> albumin signal peptide- NBN104 synthetic
   <400> 65
   atgaagtggg tgaccttcct gctgctgctg ttcatctccg gctccgcctt ctccgccgcc    60
   ggcgctcgag gctgccggct gcggtcccag ctggtgcctg tgcgggccct gggcctgggc   120
   caccggtccg acgagctggt gcggttccgg ttctgctccg gctcctgccg gcgggcccgg   180
   tcccctcacg acctgtccct ggcctccctg ctgggcgccg gcgccctgcg gcctcctcct   240
   ggctcccggc ctgtgtccca gccttgctgc cggcctaccc ggtacgaggc cgtgtccttc   300
   atggacgtga actccacctg gcggaccgtg gaccggctgt ccgccaccgc ctgcggctgc   360
   ctgggctga                                                          369
<210> 66
   <211> 122
   <212> PRT
   <213> artificial sequence
<220>
   <223> Albumin signal peptide- NBN104 synthetic sequence
   <400> 66
   Met Lys Trp Val Thr Phe Leu Leu Leu Leu Phe Ile Ser Gly Ser Ala
   1               5                   10                  15
   Phe Ser Ala Ala Gly Ala Arg Gly Cys Arg Leu Arg Ser Gln Leu Val
               20                  25                  30
   Pro Val Arg Ala Leu Gly Leu Gly His Arg Ser Asp Glu Leu Val Arg
           35                  40                  45
   Phe Arg Phe Cys Ser Gly Ser Cys Arg Arg Ala Arg Ser Pro His Asp
       50                  55                  60
   Leu Ser Leu Ala Ser Leu Leu Gly Ala Gly Ala Leu Arg Pro Pro Pro
   65                  70                  75                  80
   Gly Ser Arg Pro Val Ser Gln Pro Cys Cys Arg Pro Thr Arg Tyr Glu
                   85                  90                  95
   Ala Val Ser Phe Met Asp Val Asn Ser Thr Trp Arg Thr Val Asp Arg
               100                 105                 110
   Leu Ser Ala Thr Ala Cys Gly Cys Leu Gly
           115                 120
<210> 67
   <211> 662
   <212> DNA
   <213> artificial sequence
<220>
   <223> GHSP with intron- NBN104 synthetic sequence
<400> 67
   atggctacag taagcgcccc taaaatccct ttgggcacaa tgtgtcctga ggggagaggc     60
   ggcgtcctgt agatgggacg ggggcactaa ccctcaggtt tggggcttat gaatgttagt    120
   atcgccatct aagcccagta tttggccaat ctccgaatgt tcctggtccc tggagggagg    180
   cagagagaga gagaaaaaaa aaaacccagc tcctggaaca gggagagcgc tggcctcttg    240
   ctctccagct ccctctgttg ccctccggtt tctccccagg ctcccggacg tccctgctcc    300
   tggcttttgg cctgctctgc ctgtcctggc ttcaagaggg cagtgccgcc gccggcgctc    360
   gaggctgccg gctgcggtcc cagctggtgc ctgtgcgggc cctgggcctg ggccaccggt    420
   ccgacgagct ggtgcggttc cggttctgct ccggctcctg ccggcgggcc cggtcccctc    480
   acgacctgtc cctggcctcc ctgctgggcg ccggcgccct gcggcctcct cctggctccc    540
   ggcctgtgtc ccagccttgc tgccggccta cccggtacga ggccgtgtcc ttcatggacg    600
   tgaactccac ctggcggacc gtggaccggc tgtccgccac cgcctgcggc tgcctgggct    660
   ga                                                                    662
<210> 68
   <211> 130
   <212> PRT
   <213> artificial sequence
<220>
   <223> GHSP-NBN104 synthetic sequence
<400> 68
   Met Ala Thr Gly Ser Arg Thr Ser Leu Leu Leu Ala Phe Gly Leu Leu
   1               5                   10                  15
   Cys Leu Ser Trp Leu Gln Glu Gly Ser Ala Ala Ala Gly Ala Arg Gly
               20                  25                  30
   Cys Arg Leu Arg Ser Gln Leu Val Pro Val Arg Ala Leu Gly Leu Gly
           35                  40                  45
   His Arg Ser Asp Glu Leu Val Arg Phe Arg Phe Cys Ser Gly Ser Cys
       50                  55                  60
   Arg Arg Ala Arg Ser Pro His Asp Leu Ser Leu Ala Ser Leu Leu Gly
   65 70 75 80
   Ala Gly Ala Leu Arg Pro Pro Pro Gly Ser Arg Pro Val Ser Gln Pro
   85 90 95
   Cys Cys Arg Pro Thr Arg Tyr Glu Ala Val Ser Phe Met Asp Val Asn
   100 105 110
   Ser Thr Trp Arg Thr Val Asp Arg Leu Ser Ala Thr Ala Cys Gly Cys
   115 120 125
   Leu Gly
   130
<210> 69
   <211> 372
   <212> DNA
   <213> artificial sequence
<220>
   <223> Modified Albumin signal peptide sequence- NBN104 synthetic sequen ce
<400> 69
   atgaagtggg tgaccttcct gctgttcctg ctgttcatct ccggcgatgc cttcgctgcc      60
   gccggcgctc gaggctgccg gctgcggtcc cagctggtgc ctgtgcgggc cctgggcctg     120
   ggccaccggt ccgacgagct ggtgcggttc cggttctgct ccggctcctg ccggcgggcc     180
   cggtcccctc acgacctgtc cctggcctcc ctgctgggcg ccggcgccct gcggcctcct     240
   cctggctccc ggcctgtgtc ccagccttgc tgccggccta cccggtacga ggccgtgtcc     300
   ttcatggacg tgaactccac ctggcggacc gtggaccggc tgtccgccac cgcctgcggc     360
   tgcctgggct ga                                                         372
<210> 70
   <211> 123
   <212> PRT
   <213> artificial sequence
<220>
   <223> Modified rat albumin signal peptide - NBN104 synthetic sequence
   <400> 70
   Met Lys Trp Val Thr Phe Leu Leu Phe Leu Leu Phe Ile Ser Gly Asp
   1 5 10 15
   Ala Phe Ala Ala Ala Gly Ala Arg Gly Cys Arg Leu Arg Ser Gln Leu
   20 25 30
   Val Pro Val Arg Ala Leu Gly Leu Gly His Arg Ser Asp Glu Leu Val
   35 40 45
   Arg Phe Arg Phe Cys Ser Gly Ser Cys Arg Arg Ala Arg Ser Pro His
   50 55 60
   Asp Leu Ser Leu Ala Ser Leu Leu Gly Ala Gly Ala Leu Arg Pro Pro
   65 70 75 80
   Pro Gly Ser Arg Pro Val Ser Gln Pro Cys Cys Arg Pro Thr Arg Tyr
   85 90 95
   Glu Ala Val Ser Phe Met Asp Val Asn Ser Thr Trp Arg Thr Val Asp
   100 105 110
   Arg Leu Ser Ala Thr Ala Cys Gly Cys Leu Gly
   115 120
<210> 71
   <211> 81
   <212> DNA
   <213> artificial sequence
<220>
   <223> synthetic oligonucleotide
<400> 71
   aagcttgcta gcatgaattc atctcgaggc tgccggctgc ggtcccagct ggtgcctgtg 60
   cgggccctgg gcctgggcca c 81
<210> 72
   <211> 81
   <212> DNA
   <213> artificial sequence
<220>
   <223> synthetic oligonucleotide
<400> 72
   ttctgctccg gctcctgccg gcgggcccgg tcccctcacg acctgtccct ggcctccctg 60
   ctgggcgccg gcgccctgcg g 81
<210> 73
   <211> 81
   <212> DNA
   <213> artificial sequence
<220>
   <223> synthetic oligonucleotide
   <400> 73
   cagccttgct gccggcctac ccggtacgag gccgtgtcct tcatggacgt gaactccacc 60
   tggcggaccg tggaccggct g 81
<210> 74
   <211> 81
   <212> DNA
   <213> artificial sequence
<220>
   <223> synthetic oligonucleotide
   <400> 74
   ggcccgccgg caggagccgg agcagaaccg gaaccgcacc agctcgtcgg accggtggcc 60
   caggcccagg gcccgcacag g 81
<210> 75
   <211> 81
   <212> DNA
   <213> artificial sequence
<220>
   <223> synthetic oligonucleotide
   <400> 75
   gtaccgggta ggccggcagc aaggctggga cacaggccgg gagccaggag gaggccgcag 60
   ggcgccggcg cccagcaggg a 81
<210> 76
   <211> 78
   <212> DNA
   <213> artificial sequence
<220>
   <223> synthetic oligonucleotide
   <400> 76
   cttggaattg tcgacggatc ctcagcccag gcagccgcag gcggtggcgg acagccggtc 60
   cacggtccgc caggtgga 78
<210> 77
   <211> 55
   <212> DNA
   <213> artificial sequence
<220>
   <223> synthetic oligonucleotide
<400> 77
   aagcttagct agcggatcca tgaagtgggt gaccttcctg ctgctgctgt tcatc 55
<210> 78
   <211> 61
   <212> DNA
   <213> artificial sequence
<220>
   <223> synthetic oligonucleotide
   <400> 78
   ggcagcctcg agcgccggcg gcggagaagg cggagccgga gatgaacagc agcagcagga 60
   a 61
<210> 79
   <211> 35
   <212> DNA
   <213> artificial sequence
<220>
   <223> synthetic oligonucleotide
<400> 79
   aagcttagct agcggatcca tggctacagg taagc 35
<210> 80
   <211> 54
   <212> DNA
   <213> artificial sequence
<220>
   <223> synthetic oligonucleotide
<400> 80
   aagcttagct agcggatcca tggagctggg cctgggcggc ctgtccaccc tgtc 54
<210> 81
   <211> 55
   <212> DNA
   <213> artificial sequence
<220>
   <223> synthetic oligonucleotide
<400> 81
   ggcggcagcc tgccctgtgg cctaccctgg ccgccctggc cctgctgtcc tccgt 55
<210> 82
   <211> 492
   <212> DNA
   <213> artificial sequence
<220>
   <223> deltapro human 113 nucleic acid sequence
<400> 82
   tataggatcc gccaccatgg aacttggact tggaggcctc tccacgctgt cccactgccc     60
   ctggcctagg cggcagcctg ccctgtggcc caccctggcc gctctggctc tgctgagcag    120
   cgtcgcagag gccgctgggg gcccgggcag ccgcgctcgg gcagcggggg cgcggggctg    180
   ccgcctgcgc tcgcagctgg tgccggtgcg cgcgctcggc ctgggccacc gctccgacga    240
   gctggtgcgt ttccgcttct gcagcggctc ctgccgccgc gcgcgctctc cacacgacct    300
   cagcctggcc agcctactgg gcgccggggc cctgcgaccg cccccgggct cccggcccgt    360
   cagccagccc tgctgccgac ccacgcgcta cgaagcggtc tccttcatgg acgtcaacag    420
   cacctggaga accgtggacc gcctctccgc caccgcctgc ggctgcctgg gctgagggct    480
   cgctcgagta ta                                                         492

## Description

### FIELD OF INVENTION

The present invention concerns methods and compositions for producing a neublastin polypeptide as well as local delivery of neublastin to specific regions of the nervous system including the central nervous system and the eye for example by gene therapy. The invention includes the delivery of neublastin from transduced or transfected cells encapsulated into a macrocapsule with a semipermeable membrane. The invention further concerns mammalian cells capable of producing neublastin in increased amounts.

### BACKGROUND OF THE INVENTION

Cells have ways to direct de novo synthesised proteins to various compartments of the cells and to the extracellular space. Signal peptides are enclosed in the coding part of the chromosomal DNA and are synthesised as part of the protein by the ribosomal apparatus. Signal peptides make up the N-terminal and cause the newly synthesised polypeptides to be directed into the rough endoplasmatic reticulum. Here, the signal peptide is cleaved from the polypeptide and the mature protein is secreted into the surroundings. Thus, the signal peptide remains inside the cell.

The pro-part of the protein is cleaved from the mature part of the protein and ends outside the cell. For some neurotrophic factors, e.g. NGF, the pro-part of the protein is bioactive as a neuropeptide.

In gene therapy where the inserted gene codes for a protein which is to be secreted, a signal sequence will need to be placed in front of the mature protein to ensure its proper processing through the rough endoplasmic reticulum and the Golgi Apparatus. The first choice is almost invariably the native signal sequence of the protein in question, because it is generally desired that the protein is secreted and/or processed in the same way as it is secreted and processed by the native cell. For some uses it is also desired that the amount of protein expressed is the same as in the native cell. Furthermore, one cannot exclude the possibility that the cleaved signal sequence plays a role in the metabolism of the cell. Finally one of skill in the art would chose to use the pre-pro-part protein to ensure correct processing and folding of the mature protein.

In many cases it has turned out that in vivo transduced and transfected cells which are supposed to secrete a therapeutic factor do not secrete the therapeutic factor in therapeutically sufficient quantities and for sufficient time. This may also be a problem in ex vivo gene therapy where cells are transfected or transduced outside the body and inserted into the patient after genetic modification.

The prior art does not provide much information concerning the coupling of signal peptide with heterologous proteins in mammals. For heterologous expression of mammalian proteins in fungi or yeast it is common practise to replace the mammalian signal peptide with one that is functional in the producer species.

Sah et al. (WO 02/078730) demonstrate systemic administration of a Neublastin polypeptide for the preparation of a pharmaceutical composition for treating, preventing, or delaying neuropathic pain.

### SUMMARY OF THE INVENTION

The present invention relates to a method for producing a biologically active neublastin polypeptide, comprising culturing a cell comprising an expression vector comprising a nucleic acid comprising a promoter sequence operably linked to a nucleotide sequence encoding a signal peptide and a neublastin polypeptide, wherein said nucleotide sequence does not encode a neublastin pro-region and wherein said signal peptide is an immunoglobulin signal peptide, a TGF-beta signal peptide, a GDF signal peptide, an IGF signal peptide, a BMP signal peptide, a Neurotrophin signal peptide, a PDGF signal peptide, an EGF signal peptide, an insulin signal peptide, an ADH signal peptide, an LH signal peptide, an FSH signal peptide, an ACTH signal peptide, an MSH signal peptide, a TSH signal peptide, a DHEA signal peptide, an interleukin signal peptide, a neurturin signal peptide, a GDNF signal peptide, a persephin signal peptide, an NGF signal peptide, a signal peptide being the signal peptide contained in SEQ ID NO:70, or a native Neublastin signal peptide.

The present invention provides a solution to the problem of almost complete absence of secretion of neurotrophic factor, including neublastin, often experienced upon transduction of mammalian cells with viral vectors both in vivo and in vitro. The phenomenon is also observed for plasmid based expression vectors. For some unknown reason mammalian cells are often blocked from secreting the neurotrophic factor encoded by the vector. One possible explanation could be that correct processing of secreted proteins is cell specific. This represents a serious problem in the use of viral vector gene therapy. Today, viral vector gene therapy is considered the most preferred (if not only relevant) method for in vivo or ex vivo gene therapy because the viral vectors ensure stable integration into the genome of the transduced cell.

The invention provides for efficient expression of a mature human neublastin, or a biologically active truncation of a mature human neublastin, i.e., a secreted neublastin polypeptide, as a pre protein, instead of as a pre pro protein. A neublastin pre protein according to the invention generally comprises two components: a secreted neublastin polypeptide (as defined above), and a heterologous signal sequence.

Furthermore the present inventors have shown that by replacing the native signal peptide of neublastin with an alternative signal peptide from other proteins, such as from immunoglobulin heavy chain variable region, the secretion of neublastin is further enhanced, especially from transduced cells.

Furthermore the present inventors have shown that by removing the nucleotide sequence encoding the pro-region from the nucleotide sequence encoding the signal peptide as well as the neublastin polypeptide, then it is possible to express and have a higher amount of neublastin secreted, than if the pro-region is included.

It has been found that although the pro-region is necessary for many proteins to fold correctly, it is possible the produce a biologically active neublastin polypeptide without a pro-region.

In a further aspect the invention relates to the nucleotide sequence encoding the signal peptide and the neublastin polypeptide, the expression vector comprising the nucleotide sequence, a pharmaceutical composition comprising the vector according to the invention and one or more pharmaceutically acceptable adjuvants, excipients, carriers and/or diluents. The pharmaceutical composition can be used for in vivo and ex vivo gene therapy.

In a further aspect the invention relates to an isolated host cell transduced or transfected with the vector according to the invention.

Such genetically modified host cells have turned out to produce unexpectedly high amounts of neublastin compared to cells transduced or transfected with vectors encoding neublastin with its native signal sequence. These transduced or transfected host cells therefore constitute a promising source of producer cells for the industrial scale production of neublastin. The neublastin-secreting cells can also be used for transplantation into mammalian subjects as a source of neublastin. One particular application is ex vivo gene therapy.

In a further aspect the invention relates to a packaging cell line capable of producing an infective vector particle, said vector particle comprising a retrovirally derived genome comprising a 5' retroviral LTR, a tRNA binding site, a packaging signal, a promoter operably linked to a polynucleotide sequence encoding a fusion protein comprising neublastin and a Immunoglobulin signal peptide, an origin of second strand DNA synthesis and a 3' retroviral LTR.

These packaging cell lines can be used for producing the viral vectors according to the invention. They can also be used for in vivo gene therapy when encapsulated and transplanted to the CNS.

In a further aspect, the invention relates to a transgenic non-human mammal comprising at least one cell being transduced or transfected with the vector according to the invention. Such animals that overexpress neublastin can be used for gene profiling and in the screening and development of drugs.

Preferably the transduced or transfected cell has the genotype of the individual animal, i.e. is not an allogenic or xenogenic transplant.

In a further aspect, the invention relates to an implantable cell culture device, the device comprising:
a semipermeable membrane permitting the diffusion of a neurotrophic factor therethrough; and
at least one isolated host cell according to the invention.

These capsules can be used for the local delivery of neublastin upon transplantation into the central nervous system. Localised and prolonged delivery of growth factor is a preferred administration method for the treatment of a number of CNS disorders, including but not limited to Parkinson's disease, Alzheimer's disease, Huntington's disease, stroke, and amyotrophic lateral sclerosis (ALS). The capsules can likewise be used for local and prolonged delivery of neublastin for peripheral disorders including but not limited to neuropathy and neuropathic pain. Further indications include eye disorders.

The capsules of this invention provide for the delivery of viral particles to a desired site in a patient using a capsular approach. Encapsulation of vector-producing cell lines permits continuous delivery of the viral particle to the target site, as opposed to a single infusion. In addition, repeat therapy is possible, with reduced likelihood of immune attack. The capsules have pores large enough to allow passage of viral particles released from the packaging cells, yet prevent host-cell passage into the capsule.

This capsular approach increases the safety and control of the therapy because the devices can easily be retrieved (terminating the transduction treatment) or explanted and reimplanted (modifying the treatment). Further, the chance of infection is reduced because the capsular device is not open or externalised.

Finally, because encapsulation prevents the packaging cells from migrating within the patient, and prolongs the viability of the packaging cells upon implant, fewer cells are likely to be needed for this therapy. This may be advantageous in further lowering an immune reaction in the patient.

In a further aspect the invention relates to use of the vector according to the invention as a medicament.

In a still further aspect the invention relates to use of the vector according to the invention for the preparation of a medicament for the treatment of a nervous system disorder.

In another aspect the invention relates to the use of the vector according to the invention for the preparation of a medicament for the treatment of a CNS disorder.

Furthermore, the invention relates to a method of treating a nervous system disease, said method comprising administering to an individual in need thereof:
a therapeutically effective amount of the vector of the invention; or
a therapeutically effective amount of the pharmaceutical composition comprising the vector.

According to this aspect of the invention there is provided improved in vivo gene therapy methods for the treatment of nervous system diseases. As evidenced by the appended examples, transduction with the viral vectors of the present invention results in hitherto unseen secretion of the encoded neublastin and as a consequence improved therapeutic effect

In a still further aspect the invention relates to a method of treating a nervous system disease, said method comprising transplanting to an individual in need thereof:
- a therapeutically effective amount of the transduced cells of the invention; or
- an implantable device according to the invention or
   This aspect provides another way of treating nervous system disorders based on ex vivo gene therapy and implantation of therapeutic cells capable of secreting increased amounts of neublastin.
   The currently preferred method for large-scale production of neublastin is heterologous expression in E. coli, subsequent lysis, extraction, purification, refolding and optionally cleavage of the protein. An alternative method which is used for production of research scale amounts includes culture of a mammalian producer cell such as CHO cells secreting correctly processed and folded neublastin into the culture medium, from where it can be isolated relatively easily. The mammalian cells of the present invention produce neublastin in higher amounts than seen before for mammalian cells and therefore represent an improved source of cells for producing bioactive neublastin, which is correctly processed and folded neublastin.

### BRIEF DESCRIPTION OF THE DRAWINGS

Figure 1: Alignment of IgSP sequences from various mammals.

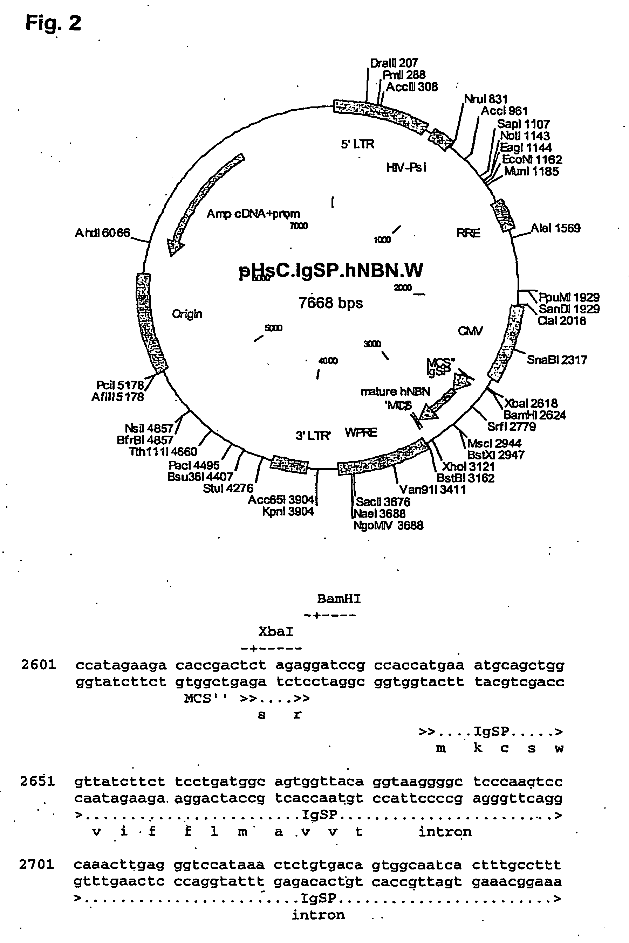

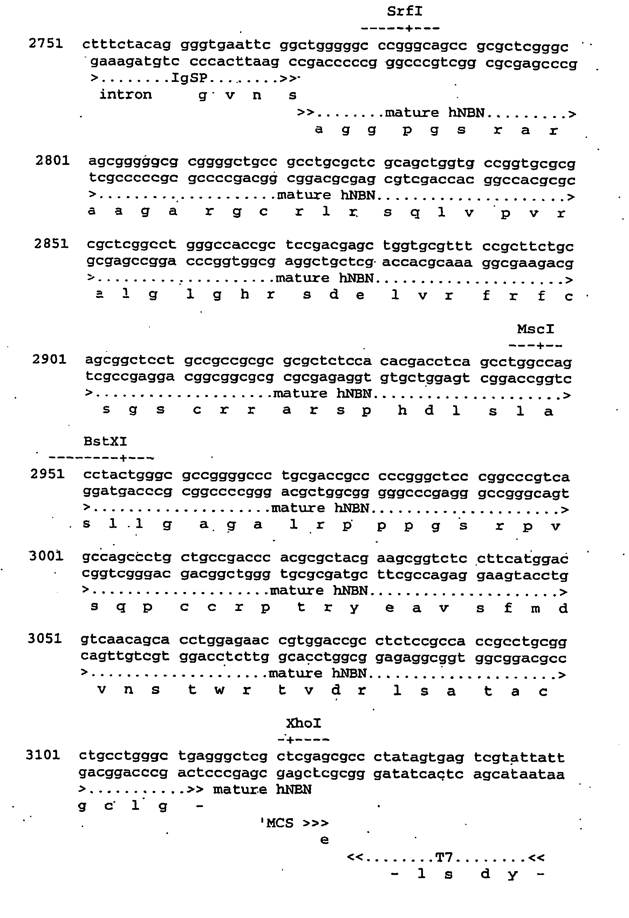

Figure 2: Vector map of the lentiviral vector construct pHsC.IgSP.hNBN.W used for transduction experiments in Example 2.
Figure 3: Vector map of the pNS1n.IgSP.hNBN. Same IgSP-NBN sequence as pHsC.IgSP.hNBN.W between BamHI and Xhol restriction sites used in Example 1.
Figure 4: Determination of NBN activity in the RetL3 ELISA (duplicate samples). (a) NBN activities in cell supernatants were determined using recombinant mouse Artemin produced in E. coli (mART) as standard, (b) Analysis of supernatants from transfected cells, (c) Analysis of supernatants from transduced cells.
Figure 5: Western blot analysis with the anti-NBN antibody #378. (a) Analysis of GFR[alpha]3 affinity purified NBN from CHO-NBN16 cells (CHO-NBN) and 20 ng rat recombinant NBN (rNBN) (b) Analysis of supernatants from ARPE-19 transfected or transduced with IgSP-NBN expression constructs and two CHO cell clones stably overexpressing NBN from a wildtype construct (CHO-NBN25c and CHO-NBN16). Arrows indicate the position of the glycosylated and non-glycosylated monomers of neublastin after reduction by the antibody #378.
Figure 6: Prediction of signal peptide cleavage by SignalP. For explanation see Example 4.
Figure 7: Fig. 7A shows plasmid pHs C.hNBN.W and Fig. 7B shows plasmid pHsCXW. For explanation see Example 6.
Figure 8: Fig. 8A shows relatives NBN release conditioned medium and Fig. 8B and 8C show NBN in different cell lines, see also Example 7.
Figure 9 depicts the sequence of the 104 carboxy (C) terminal amino acids of the native human neublastin polypeptide (SEQ ID NO: 19) and the corresponding DNA sequence encoding the 104 C terminal amino acids of the native human neublastin (SEQ ID NO: 58) aligned with a synthetic gene encoding the 104 C terminal amino acids of the native human neublastin optimized for CHO cell expression (SEQ ID NO: 59). Nucleotides in the synthetic gene that have been changed from the native sequence are indicated (*).
Figure 10 depicts the neublastin sequence within plasmid pNBN026-35 (SEQ ID NO: 60). Immediately upstream of the presented sequence is a "CT" dinucleotide that contributes to a Xhol restriction site. Immediately downstream is a BamHI restriction site.
Figure 11 depicts the DNA (SEQ ID NO: 61) and amino acid (SEQ ID NO: 62) sequence of the 104 C terminal amino acids of neublastin fused to a synthetic signal sequence. The signal sequence is underlined.
Figure 12 depicts the DNA (SEQ ID NO: 63) and amino acid (SEQ ID NO: 64) sequence of the 104 C terminal amino acids of neublastin fused to a neublastin signal sequence. The signal sequence is underlined.
Figure 13A depicts the DNA (SEQ ID NO: 65) and amino acid (SEQ ID NO: 66) sequence of the 104 C terminal amino acids of neublastin fused to an albumin signal sequence. The signal sequence is underlined.
Figure 13B depicts the DNA (SEQ ID NO: 69) and amino acid (SEQ ID NO: 70) sequence of the 104 C terminal amino acids of neublastin fused to a modified albumin signal sequence. The signal sequence is underlined.
Figure 14 depicts the DNA sequence (SEQ ID NO: 67) and amino acid sequence (SEQ ID NO: 68) of the 104 C terminal amino acids of neublastin fused to a human growth hormone signal sequence. The signal sequence, which contains an intron, is underlined.

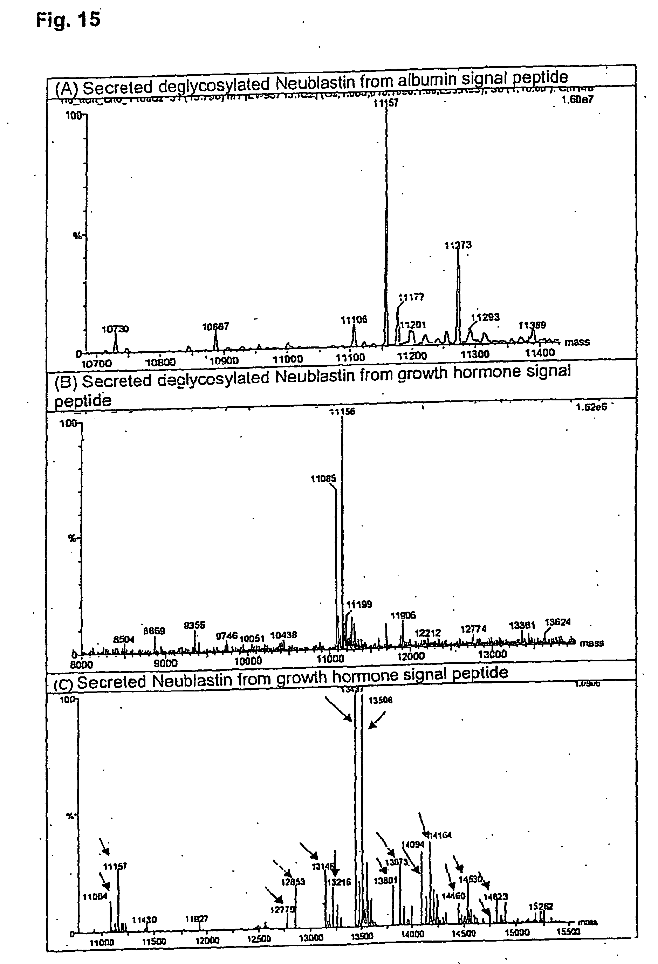

Figure 15 depicts mass spectrometer results of neublastin secreted from CHO cells using the albumin signal sequence (15A) or the human growth hormone signal sequence (15B)(15C). The peaks at 11,156 and 11,157 daltons correspond to a 104-amino acid neublastin C terminal fragment. The peaks at 11,084 and 11,085 daltons correspond to a 103-amino acid neublastin C terminal fragment. Figure 15A depicts deglycosylated neublastin from albumin-directed secretion. Figure 15B depicts deglycosylated neublastin from human growth hormone-directed secretion. Figure 15C depicts neublastin from human growth hormone-directed secretion. Peaks with greater masses correspond to the presence of various glycoforms.
Figure 16 depicts KIRA assay results demonstrating activity of recombinantly produced neublastin produced in CHO cells.

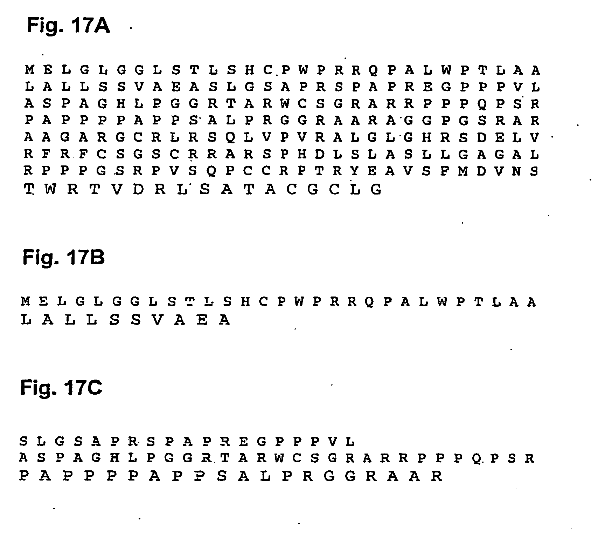

Figure 17A depicts the amino acid sequence of full-length neublastin including the mature protein, the pro-domain and the signal peptide (SEQ ID NO: 10). Figure 17B depicts the amino acid sequence of the full-length native neublastin signal peptide.
Figure 17C depicts the amino acid sequence of the full-length neublastin pro-domain.

### DEFINITIONS

"C terminal amino acids," as used herein, means a series of contiguous amino acids in a polypeptide chain most distal from the amino (N) terminus of the polypeptide.

"Neublastin pro-region" means a region comprising at least amino acids corresponding to amino acids -41 to -11 of SEQ ID NO: 10, 11, 12.

"Preproneublastin polypeptide," (SEQ ID NO: 10) as used herein, means a polypeptide consisting of mature human neublastin, *i.e*., the 113 C terminal amino acids of neublastin (a.a. 108 to 220 of SEQ ID NO: 10), the full length human neublastin prodomain, *i.e*., the 68 amino acids proximal to the N terminus of the mature neublastin (a.a. 40 to 107 of SEQ ID NO: 10), and the human neublastin signal peptide, *i.e*., the 39 amino acids proximal to the N terminus of the neublastin pro-domain (a.a. 1 to 39 of SEQ ID NO: 10).

Signal peptide - eukaryotic signal peptide. A eukaryotic signal peptide is a peptide present on proteins that are destined either to be secreted or to be membrane components. It is usually N-terminal to the protein. In the present context, all signal peptides identified in SignalP (version 2.0 or preferably version 3.0) are considered a signal peptide.

"Functional neublastin signal peptide," as used herein, means first 39 amino acids of SEQ ID NO: 10 or any portion thereof that effects the secretion of the mature neublastin from a cell.

"Functional neublastin signal sequence" means a nucleic acid sequence encoding a functional neublastin signal peptide.

A mammalian signal peptide is a signal peptide derived from a mammalian protein secreted through the ER.

Mature human neublastin polypeptide as used herein means the C-terminal 113 amino acids of native human neublastin, i.e. amino acids 108-220 of SEQ ID No. 10

Mature mouse neublastin polypeptide as used herein means the C-terminal 113 amino acids of native mouse neublastin, i.e. amino acids 112-224 of SEQ ID No. 11

Mature rat neublastin polypeptide as used herein means the C-terminal 113 amino acids of native rat neublastin, i.e. amino acids 112-224 of SEQ ID No. 12

Neublastin polypeptide as used herein means a polypeptide comprising the C-terminal 99-140 amino acids of native human Neublastin, the C-terminal 99-144 of native rat or mouse Neublastin. More preferably a Neublastin polypeptide comprises the C-terminal 99-113 amino acids of native human neublastin, the C-terminal 99-113 amino acids of native rat neublastin, or the C-terminal 99-113 amino acids of mouse neublastin, each with up to 15 amino acid substitutions in the native sequence. In certain contexts it will be understood that "secreted neublastin polypeptide" means a polypeptide to be secreted as opposed to one that has been secreted already. The secreted neublastin polypeptide does not contain a neublastin pro-region.

Functional neublastin prodomain is a peptide located between the signal peptide and the mature peptide, which propeptide is cleavable from the mature peptide by furin after cleavage of the signal peptide.

Bioactivity: ability to bind when dimerised along with GFRα3 to RET and induce RET dimerisation and autophosphorylation. Measured with Kira Elisa or RET L3 Elisa assays.

"Heterologous," as used when referring to a nucleic acid sequence or an amino acid sequence, means a sequence that originates from a source foreign to the particular host cell, or, if from the same host cell, is modified from its original form.

Heterologous signal peptide - a signal peptide not naturally being operatively linked to a neublastin polypeptide.

### DETAILED DESCRIPTION OF THE INVENTION

The invention relates to a method for producing a neublastin polypeptide wherein the nucleotide sequence encoding neublastin polypeptide does not encode a pro-region. In the present context a pro-region comprises at least amino acids -41 to -11 of SEQ. ID. NO.: 10, 11 or 12. More preferably the pro-region comprises at least amino acids -41 to -1 of any of SEQ. ID. NO.: 10, 11 or 12. More preferably the pro-region comprises at least amino acids -41 to 10 of any of SEQ. ID. NO.: 10, 11 or 12, most preferably it comprises the pro-domain of the sequences, corresponding to amino acids -41 to 27 of SEQ ID NO: 10, or amino acids -41 to 31 of SEQ ID NO: 11, or of SEQ ID NO: 12.

### Signal peptides

The expression vector according to the invention comprises a nucleic acid comprising a promoter sequence capable of culturing a cell transduced with an expression vector comprising a nucleic acid comprising a promoter sequence capable of directing expression of a nucleotide sequence encoding a signal peptide operatively linked to a neublastin polypeptide, wherein said nucleotide sequence does not encode a pro-region of a neublastin polypeptide.

During the secretion process, the signal peptide of the neublastin pre-protein is cleaved by the host cell producing the neublastin polypeptide. While the cleavage site is generally defined, a skilled artisan will appreciate that there can be variability in the signal peptide cleavage site. Accordingly, embodiments having some ambiguity with respect to the exact cleavage site are within the scope of the invention.

The signal peptide may be any functional signal peptide, such as a heterologous signal peptide, such as a mammalian signal peptide. The signal peptide may be from any suitable species, such as human, mouse, rat, monkey, pig, dog, cat, cow or horse.

The signal peptide is linked to the neublastin polypeptide, and is preferably directly fused to said neublastin polypeptide, such as the C-terminal end of the signal peptide being fused to the N-terminal end of the neublastin polypeptide.

As evidenced by the appended examples, the use of this signal peptide in general results in an improved secretion of neublastin both in vitro and in vivo. The results were reproducible both with lentivirus-transduced cells (in vivo and in vitro) and with plasmid transfected cells (in vitro). The cells produce the mature protein as a biologically active protein when the signal peptide gene is fused directly to the gene coding for the mature protein (i.e. excluding the pro part).

The inventors have discovered that not only the native neublastin signal peptide functions as a signal peptide, but also heterologous signal peptides are useful and often provide a higher yield than the native signal peptide. The heterologous signal peptide can be selected from the group consisting of a growth factor signal peptide, a hormone signal peptide, a cytokine signal peptide and an immunoglobulin signal peptide.

Thus, examples of signal peptides are signal peptides selected from the group consisting of TGFβ signal peptides, GDF signal peptides, IGF signal peptides, BMP signal peptides, Neurotrophin signal peptides, PDGF signal peptide and EGF signal peptide, signal peptides selected from a hormone signal peptide, said hormone being selected from the group consisting of growth hormone, insulin, ADH, LH, FSH, ACTH, MSH, TSH, T3, T4, and DHEA, or an interleukin signal peptide.

In one embodiment, the signal peptide is selected from the group consisting of neurturin signal peptide, GDNF signal peptide, persephin signal peptide, and NGF signal peptide.

In another embodiment, the signal peptide is selected from the group consisting of albumin signal peptide, modified albumin signal peptide, and growth hormone signal peptide, such as a signal peptide selected from the group consisting of rat albumin signal peptide, modified rat albumin signal peptide, and human growth hormone signal peptide, such as rat albumin signal peptide and human growth hormone signal peptide.

Thus, in some embodiments, the secreted neublastin polypeptide is fused to a native rat albumin signal peptide. This is exemplified by SEQ ID NO: 66. In other embodiments, the secreted neublastin polypeptide is linked to a modified rat albumin signal sequence. This is exemplified by SEQ ID NO: 70. In other embodiments, the secreted neublastin polypeptide is fused to a human growth hormone signal sequence. This is exemplified by SEQ ID NO: 68.

In yet another embodiment, the signal peptide is an immunoglobulin signal peptide, such as the immunoglobulin heavy chain signal peptide. In particular, an immunoglobulin signal peptide may be a signal peptide selected from the group consisting of mouse IgSP (SEQ ID NO 4), rat IgSP (SEQ ID NO 6), porcine IgSP (SEQ ID NO 5), simian IgSP (SEQ ID NO 2 or 3), human IgSP (SEQ ID NO 1), such as mouse IgSP (SEQ ID NO 4) or human IgSP (SEQ ID NO 1).

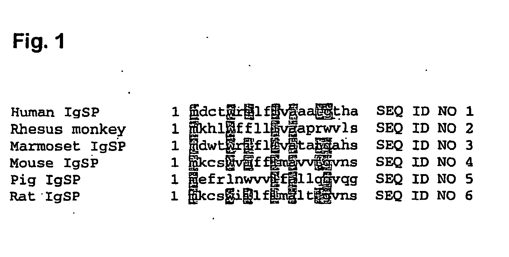

Immunoglobulin signal peptide (IgSP) is a small 19 amino acid peptide known from a large group of mammals. The sequences from human, rhesus monkey, marmoset, rat, mouse and pig are aligned in Figure 1. The percent sequence identity compared to human IgSP varies from 21 (pig) to 68 (marmoset) percent. This relatively large variation indicates that the specific sequence can be altered to a large extent without substantially changing the biological function of the signal peptide. It is also observed that there is cross species reactivity as evidenced by the appended examples. These were carried out with the mouse IgSP which was functional in rat (in vivo experiments) and in human cells (ARPE-19 cells).

Preferably the IgSP is of mouse or human origin because the mouse IgSP is known to be functional in mouse, rat and human beings. For use in human beings, the IgSP preferably is of human origin in order to reduce the risk of any cross species side effect.

In another embodiment the signal peptide is a native neublastin signal peptide such as a native human neublastin signal peptide. In this context the latter construct of native neublastin signal peptide and a neublastin polypeptide is called delta-proneublastin.

In yet another embodiment, the signal peptide is a synthetic signal peptide, such as the signal peptide having the amino acid sequence of AA1-AA38 of SEQ ID NO 62. (MetSerTrpAlaTrpAlaAlaCysProProCysProThrAlaLeuGlyLeuGlyGlySerAlaLeuTrpProTbr-LeuAlaAlaLeuAlaLeuLeuSerSerValAlaGluAla)

### Neublastin

Neublastin polypeptides are proteins, which promote survival, maintain phenotypic differentiation, prevent degeneration, promote regeneration, and restore the activity of neuronal cells and tissues. Neublastin (initially described, e.g., in WO 00/01815) has alternately been referred to as "artemin" (see, e.g., WO 00/18799) and "enovin" (see, e.g., WO 00/04050).

Neublastin has been classified as a distant member of the TGF-β superfamily (Massague, et al,. 1994, Trends in Cell Biology, 4: 172-178) and is a member of glial cell line-derived neurotrophic factor ligand family ("GDNF"; WO 93/06116), in the family which includes GDNF, persephin ("PSP"; Milbrandt et al., 1998, Neuron 20: 245253) and neurturin ("NTN"; WO 97/08196). The ligands of the GDNF subfamily have in common their ability to induce signalling through the RET receptor tyrosine kinase. These three ligands of the GDNF subfamily differ in their relative affinities for a family of neurotrophic receptors, the GFR[alpha] receptors. Neublastin acts preferably through the GFR[alpha]3-RET complex. Baudet et al., Development, 127, pp. 4335-44 (2000); Baloh et al., Neuron, 21, pp. 1291-1302 (1998); Airaksinen et al., Mol. Cell. Neuroscience, 13, pp. 313-325 (1999).

An amino acid sequence comparison of neublastin (SEQ ID NO: 10) to the GDNF subfamily members Neurturin, Persephin and GDNF is shown in Table 1. Neublastin polypeptides useful in this invention preferably hold the GDNF subfamily fingerprint, i.e. the amino acid residues underlined in Table 1.

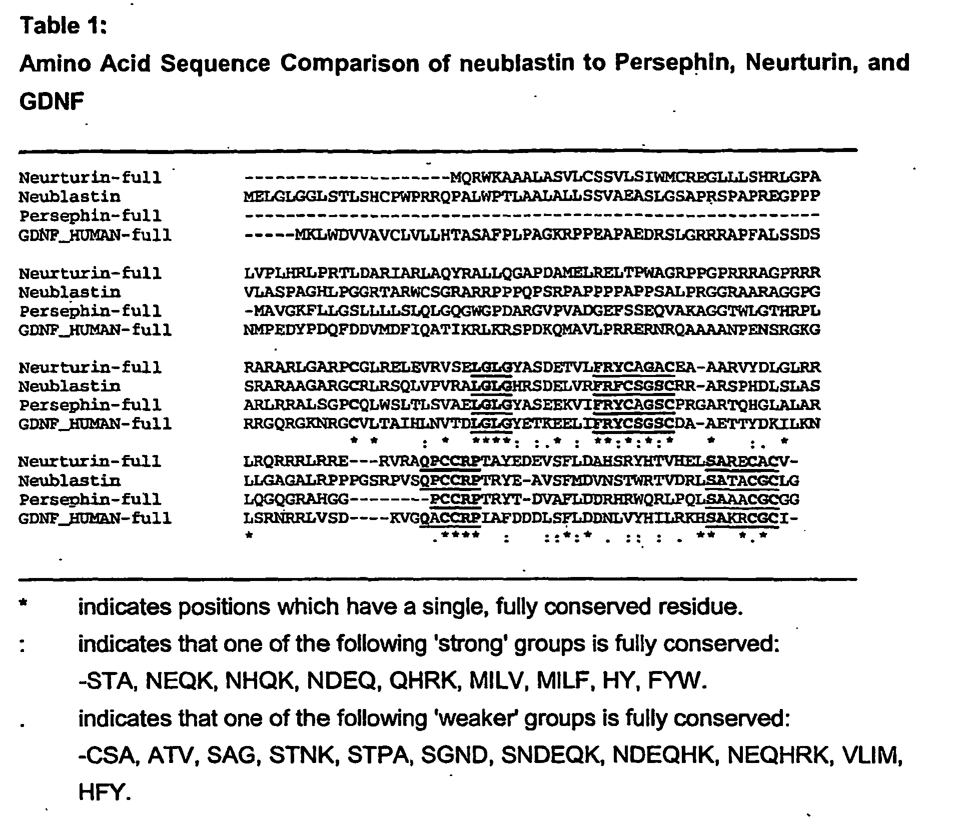

From the amino acid sequence alignment shown in Table 1, it can be seen that neublastin has seven cysteine residues at locations that are conserved within the TGF-[beta] superfamily. Based on this sequence alignment, neublastin was shown to be a member of the GDNF subfamily of neurotrophic factors (LGLG - FR(Y/F)CSGSC - QxCCRP - SAxxCGC, the GDNF subfamily fingerprint, underlined in Table 1).

The neublastin polypeptides useful herein may be provided in any bioactive form, including the form of pre-proteins, mature proteins, glycosylated proteins, phosphorylated proteins, truncated forms, or any other post-translationally modified protein. It is assumed that a bioactive neublastin is in the dimerized form for each NBN variant, because dimer formation is required for activity. Little to no activity is observed in a monomeric NBN polypeptide. A bioactive neublastin polypeptide includes a dimerized polypeptide that, in the presence of a cofactor (such as GFR[alpha]3 or RET), binds to GFR[alpha]3 or to a complex of GFR[alpha]3 and RET, induces dimerization of RET, and autophosphorylation of RET. Accordingly, a "neublastin polypeptide," as used herein, is a polypeptide which possesses neurotrophic activity (e.g., as described in WO 00/01815).

The neublastin polypeptides produced by the methods of this invention display at least one biological activity of native neublastin. Biological activity for purposes of this invention can be determined by any suitable method. A biologically active neublastin polypeptide is a polypeptide that, when dimerized, can bind, along with GFRa3, to RET and induce RET dimerization and autophosphorylation. (See *e.g*. Sanicola et al., 1997, Proc. Natl. Acad. Sci. USA, 94:6238). Any method of determining receptor binding and receptor autophosphorylation may be used to evaluate the biological activity the neublastin polypeptide produced by the methods of the invention. For example, the KIRA assay (ELISA) described in Example 17 can be used to assess neublastin biological activity. (*See also,* Sadick et al., 1996, Anal. Biochem., 235(2):207).

Neublastin in bioactive form can also be detected using the RetL3 ELISA assay described in Example 1. Neublastin without biological function will not be detected by the RetL3 ELISA assay.

The following full-length sequences represent the wild type pre-pro neublastin with wild type signal peptide. Upon transduction or transfection into mammalian cells the resulting mature neublastins are only secreted in very small amounts. The native signal peptide of human, mouse and rat neublastin is represented by the first 39 amino acids. .
- AA₋₈₀-AA₁₄₀ of SEQ ID NO: 10 ("wild type" human prepro),
- AA₋₈₀AA₁₄₄ of SEQ ID No. 11 (mouse prepro),
- AA₋₈₀-AA₁₄₄ of SEQ ID NO: 12 (rat prepro).

The neublastin polypeptide secreted according to the invention can vary in length. Although the mature human neublastin polypeptide normally consists of the C terminal 113 amino acids of pre pro neublastin, not all of the 113 amino acids are required to achieve useful neublastin biological activity. Amino terminal truncation is permissible. Thus, the secreted neublastin polypeptide corresponds to the C terminal 99-113 amino acids of native human neublastin, i.e., its length can be 99, 100, 101, 102, 103, 104, 105, 106, 107, 108, 109, 110, 111, 112, or 113 amino acids. Selection of the exact length of the neublastin polypeptide to be secreted is a design choice, which can be made by one skilled in the art. A secreted human neublastin polypeptide consisting of the C terminal 104 amino acids of native human neublastin is exemplified in the working examples provided below. In addition to varying in length, the secreted human neublastin polypeptide can vary in sequence.

The following "wild-type", neublastin amino acid ("aa" or "AA") sequences are exemplary of those that are useful in the methods and compositions of this invention:
- AA₁-AA₁₄₀ of SEQ ID NO: 10 (mature 140AA; hereafter "140NBN"),
- AA₂₅-AA₁₄₀ of SEQ ID NO: 10 (mature 116AA; hereafter "116NBN"),
- AA₂₈-AA₁₄₀ of SEQ ID NO: 10 (mature 113AA (SEQ ID No. 14); hereafter "113NBN"),
- AA₁-AA₁₄₄ of SEQ ID NO: 11 (mouse mature 144 AA),
- AA₁-AA₁₄₄ of SEQ ID NO: 12 (rat mature-144 AA),
- Peptides with a C-terminal sequence set forth in AA₁₀₇-AA₁₄₀ of SEQ ID No. 10, more preferably AA₇₆-AA₁₄₀ of SEQ ID NO. 10, and which retain the 7 Cys residues characteristic of the GDNF family and of the TGF-beta super family.

In one embodiment, the preferred neublastin polypeptide contains (seven) cysteines conserved as in SEQ ID NO. 10 at positions 43,70,74,107,108,136 and 138. These seven conserved cysteine residues are known within the TGF- superfamily to form three intramonomeric disulfide bonds (contemplated, e.g., in SEQ ID No. 10 between cysteine residues 43-108, 70-136, and 74-138) and one intermonomeric disulfide bond (contemplated, e.g., in SEQ ID NO. 10 between cysteine residues 107-107), which together with the extended beta strand region constitutes the conserved structural motif for the TGF-[beta] superfamily. See, e.g.; Daopin et al., Proteins 1993, 17: 176-192.

Preferably the neublastin polypeptide is one of the mature forms of the wild type protein. It is presently believed that the absence of the pro-region is important for high secretion levels in genetically modified mammalian cells.

Neublastin polypeptides useful in the present invention also include truncated forms of the full-length neublastin molecule. In such truncated molecules, one or more amino acids have been deleted from the N-terminus or the C-terminus, preferably the N-terminus. The truncated neublastin polypeptide may be obtained by providing a mature neublastin polypeptide and contacting the mature neublastin polypeptide with at least one protease under conditions sufficient to produce the truncated neublastin polypeptide. Preferably, at least one protease is an exoprotease, and contacting the mature neublastin polypeptide results in formation of an exopeptidase neublastin polypeptide digestion product that can be further digested with a dipeptidyl peptidase. More preferably according to the present invention the protein encoded by the expression vectors is the truncated form and needs no further processing.

The truncated neublastin polypeptides described herein preferably include a polypeptide sequence that encompasses the seven cysteine residues conserved in the mature neublastin sequence. In certain preferred embodiments, the truncated neublastin polypeptide includes at least the 85 carboxy terminal amino acids of mature 113NBN neublastin polypeptide. In more preferred embodiments the truncated neublastin polypeptide includes at least the 98 carboxy terminal amino acids of mature human 113 NBN.

One truncated form includes the 97 amino acids from the first to the last of the seven cysteine residues of mature neublastin. This corresponds to amino acids no 2 to 97 of SEQ ID No 20.

Other variants of neublastin include truncated NBN forms. Examples of these include:
(i) the 112AA polypeptide sequence designated herein as NBN 112, which possesses the carboxy terminal 112 amino acids of a mature neublastin polypeptide, e.g., amino acids 29-140 of SEQ ID NO. 10.
(ii) the 111 AA polypeptide sequence designated herein as NBN111, which possesses the carboxy terminal 111 amino acids of a mature neublastin polypeptide, e.g., amino acids 30-140 of SEQ ID NO. 10.
(iii) the 110 AA polypeptide sequence designated herein as NBN110, which possesses the carboxy terminal 110 amino acids of a mature neublastin polypeptides, e.g., amino acids 31-140 of SEQ ID NO. 10.
(iv) the 109 AA polypeptide sequence designated herein as NBN109, which possesses the carboxy terminal 109 amino acids of a mature neublastin polypeptide, e:g., amino acids 32-140 of SEQ ID NO. 10.
(v) the 108AA polypeptide sequence designated herein as NBN108, which possesses the carboxy terminal 108 amino acids of a mature neublastin polypeptide, e.g., amino acids 33-140 of SEQ ID NO. 10.
(vi) the 107AA polypeptide sequence designated herein as NBN107, which possesses the carboxy terminal 107 amino acids of a mature neublastin polypeptide, e.g., amino acids 34-140 of SEQ ID NO. 10.
(vi) the 106AA polypeptide sequence designated herein as NBN106, which possesses the carboxy terminal 106 amino acids of a mature neublastin polypeptide, e.g., amino acids 35-140 of SEQ ID NO. 10.
(viii) the 105AA polypeptide sequence designated herein as NBN105, which possesses the carboxy terminal 105 amino acids of a mature neublastin polypeptide, e.g., amino acids 36-140 of SEQ ID NO. 10.
(ix) the 104AA polypeptide sequence designated herein as NBN104, which possesses the carboxy terminal 104 amino acids of a mature neublastin polypeptide, e.g., amino acids 37-140 of SEQ ID NO. 10 (also set forth as SEQ ID No. 19).
(x) the 103AA polypeptide sequence designated wherein as NBN103, which possesses the carboxy terminal 103 amino acids of a mature neublastin polypeptide, e.g., amino acids 38-140 of SEQ ID NO. 10.
(xi) the 102AA polypeptide sequence designated herein as NBN 102, which possesses the carboxy terminal 102 amino acids of a mature neublastin polypeptide, e.g., amino acids 39-140 of SEQ ID NO. 10.
(xii) the 101AA polypeptide sequence designated herein as NBN101, which possesses the carboxy terminal 101 amino acids of a mature neublastin polypeptide, e.g., amino acids 40-140 of SEQ ID NO. 10.
(xiii) the 100AA polypeptide sequence designated herein as NBN100, which possesses the carboxy terminal 100 amino acids of a mature neublastin polypeptide, e.g., amino acids 41-140 of SEQ ID NO. 10.
(xiv) the 99AA polypeptide sequence designated herein as NBN99, which possesses the carboxy terminal 99 amino acids of a mature neublastin polypeptide, e.g., amino acids 42-140 of SEQ ID NO. 10 (also set forth as SEQ ID No. 20).

It is understood that the truncated forms of neublastin disclosed herein (e.g., the 112AA through 99AA forms) have neurotrophic activity.

In most preferred embodiments, the truncated neublastin polypeptide is the 99 aa, 100 aa, 101 aa, 102 aa, 103 aa, 104 aa, 105 aa, 106 aa, 107 aa, 108 aa, 109 aa, 110 aa, 111 aa or 112 aa carboxy terminal amino acids of mature 113 AA neublastin polypeptide (i. e., NBN99, NBN100, NBN101, NBN102, NBN103, NBN104, NBN105, NBN106, NBN107, NBN108, NBN109, NBN110, NBN111 or NBN112, respectively). The sequences may also be found in the mouse and rat neublastin polypeptides as the carboxy terminal 99 aa, 100 aa, 101 aa, 102 aa, 103 aa, 104 aa, 105 aa, 106 aa, 107 aa, 108 aa, 109 aa, 110 aa, 111 aa or 112 aa, respectively, in SEQ ID No. 11 and 12. These most preferred examples of truncated NBN forms are bioactive (referred to "bioactive truncated neublastin polypeptides") as they have been demonstrated to have neurotrophic activity. As stated above, NBN dimerization is required for bioactivity, as little to no activity is observed with the NBN monomeric polypeptide.

Truncated forms of the mouse and rat neublastins are also contemplated. These may consist of the C-terminal 99, 100, 101, 102, 103, 104, 105, 106, 107, 108, 109, 110, 111, 112, 113, 114, 115, or 116 amino acids of SEQ ID No 16 (mouse) or they may consist of the C-terminal 99, 100, 101, 102, 103, 104, 105, 106, 107, 108, 109, 110, 111 or 112 amino acids of SEQ ID No 18 (rat).

Thus, the invention encompasses a neublastin polypeptide selected from the group consisting of mature NBN selected from neublastin having a sequence identified as amino acids 1-140 of SEQ ID No 10, or amino acids 1-144 of SEQ ID No 11 or 12, SEQ ID No 13, 14, 15, 16, 17 or 18, N-terminally truncated NBN, mutated NBN, or mutated and N-truncated NBN, such as a mature NBN selected from the group consisting of neublastin having a sequence identified by SEQ ID No 13, 14, 15, 16, 16, 17, or 18), more particularly a neublastin polypeptide selected from the group consisting of N-terminally truncated neublastin with the 106, 104, 102 or 99 C-terminal amino acids of SEQ ID NO 10 or a neublastin polypeptide selected from the group consisting of the 116 C-terminal amino acids of human neublastin, the 113 C-terminal amino acids of human neublastin, the 104 C-terminal amino acids of human neublastin, and the 116 C-terminal amino acids of human neublastin, a neublastin polypeptide having the amino acid sequence of SEQ ID No 19 or a neublastin polypeptide containing the 99 amino acids of SEQ ID NO 20.

The NBNs useful in this invention also include those NBN polypeptides that have an amino acid sequence with substantial similarity or identity to the various prepro, pro, mature and truncated "neublastin" polypeptides set forth above. Preferably, the neublastin polypeptide used has at least 70%, more preferably 85%, still more preferably 90%, or still further preferably 95% identity or similarity to the mature peptide of the neublastin polypeptides in SEQ ID NO. 10-23. Most preferably the neublastin polypeptide used has at least 99% similarity or identity to the mature peptides of the neublastin polypeptides in SEQ ID No. 10-23.

The degree to which a candidate polypeptide shares homology with a neublastin polypeptide of the invention is determined as the degree of similarity or identity between two amino acid sequences.

A high level of sequence identity indicates likelihood that the first sequence is derived from the second sequence. Amino acid sequence identity requires identical amino acid sequences between two aligned sequences. Thus, a candidate sequence sharing 70% amino acid identity with a reference sequence, requires that, following alignment, 70% of the amino acids in the candidate sequence are identical to the corresponding amino acids in the reference sequence. Identity is determined by computer analysis, such as, without limitations, the ClustalX computer alignment program (Thompson JD, Gibson TJ, Plewniak F, Jeanmougin F, & Higgins DG: "The ClustalX windows interface: flexible strategies for multiple sequence alignment aided by quality analysis tools"; Nucleic Acids Res. 1997, 25 (24): 4876-82), and the default parameters suggested therein. Using this program, the mature part of a polypeptide encoded by an analogous DNA sequence of the invention exhibits a degree of identity of at least 70%, more preferably 85%, still more preferably 90%, or still further preferably 95%, most preferably at least 99% with the amino acid sequences presented herein as SEQ ID NO: 10 (human NBN), SEQ ID NOS: 11 and 12 (rodent NBN).

Other alignment tools are known, such as the dynamic programming algorithm described in Needleman et al., J. Mol. Biol. 48: 443 (1970), and the Align Program, a commercial software package produced by DNAstar, Inc.

Once the alignment between the candidate and reference sequence is made and refined, a percent homology score is calculated. The individual amino acids of each sequence are compared sequentially according to their similarity to each other.

Similarity factors include similar size, shape and electrical charge. One particularly preferred method of determining amino acid similarities is the PAM250 matrix described in Dayhoff et al., Atlas of protein sequence and structure 345-352 (1978 & Sup.). A similarity score is first calculated as the sum of the aligned pairwise amino acid similarity scores. Insertions and deletions are ignored for the purposes of percent homology and identity. Accordingly, gap penalties are not used in this calculation.

The raw score is then normalized by dividing it by the geometric mean of the scores of the candidate compound and the seven cysteine skeleton of the neublastin polypeptides. The geometric mean is the square root of the product of these scores. The normalized raw score is the percent homology.

As noted above, the neublastin polypeptides of the invention include variant polypeptides. In the context of this invention, the term "variant polypeptide" includes a polypeptide (or protein) having an amino acid sequence that differs from the mature peptide presented as part of SEQ ID NO. 10, 13, 14, 19, 20, 21, 22, or 23 (human NBN), or SEQ ID No. 11, 12, 15-18 (rodent NBN), at one or more amino acid positions. Such variant polypeptides include the modified polypeptides described above, as well as conservative substitutions, splice variants, isoforms, homologues from other species, and polymorphisms.

As defined herein, the term "conservative substitutions" denotes the replacement of an amino acid residue by another, biologically similar, residue. Typically, biological similarity, as referred to above, reflects substitutions on the wild type sequence with conserved amino acids.

Substitutes for an amino acid within the sequence of the neublastin polypeptide may be selected from other members of the class to which the amino acid belongs (see Table 1). Furthermore, various amino acids are commonly substituted with neutral amino acids, *e.g*., alanine, leucine, isoleucine, valine, proline, phenylalanine, tryptophan, and methionine. (*See e.g*. MacLennan et al., 1998, Acta Physiol. Scand. Suppl., 643:55-67; Sasaki et al., 1998, Adv. Biophys., 35:1-24). Multiple substitutions are within the scope of the invention; however, all neublastin polypeptides of the invention must possess at least one activity of native neublastin as described infra in Section C, see also the following table:

| Original Residues | Exemplary Substitutions |
|---|---|
| Ala (A) | Val, Leu, Ile |
| Arg (R) | Lys, Gin, Asn |
| Asn (N) | Gln |
| Asp (D) | Glu |
| Cys (C) | Ser, Ala |
| Gln (Q) | Asn |
| Gly (G) | Pro, Ala |
| His (H) | Asn, Gln, Lys, Arg |
| Ile (I) | Leu, Val, Met, Ala, Phe, Nor-leucine |
| Leu (L) | Norleucine, Ile, Val, Met, Ala, Phe |
| Lys (K) | Arg, 1,4-Diamino-butyric Acid, Gln, Asn |
| Met (M) | Leu, Phe, Ile |
| Phe (F) | Leu, Val, Ile, Ala, Tyr |
| Pro (P) | Ala |
| Ser (S) | Thr, Ala, Cys |
| Thr (T) | Ser |
| Trp (W) | Tyr, Phe |
| Tyr (Y) | Trp, Phe, Thr, Ser |
| Val (V) | Ile, Met, Leu, Phe, Ala, Nor-leucine |

For example, one would expect conservative amino acid substitutions to have little or no effect on the biological activity, particularly if they represent less than 10% of the total number of residues in the polypeptide or protein. Preferably, conservative amino acid substitutions represent changes in less than 5% of the polypeptide or protein, most preferably less than 2% of the polypeptide or protein.

The neublastin polypeptide in one embodiment comprises up to 15 amino acid substitutions, such as up to 12 amino acid substitutions, such as up to 10 amino acid substitutions, such as up to 8 amino acid substitutions, such as up to 5 amino acid substitutions. For example, when calculated in accordance, e.g., with human 113NBN, most preferred conservative substitutions would represent fewer than three amino acid substitutions in the wild type mature amino acid sequence. In a particularly preferred embodiment, there is a single amino acid substitution in the mature sequence, wherein both the substituted and replacement amino acids are non-cyclic. Other examples of particularly conservative substitutions include the substitution of one hydrophobic residue for another, such as isoleucine, valine, leucine or methionine, or the substitution of one polar residue for another, such as the substitution of arginine for lysine, glutamic for aspartic acid, or glutamine for asparagine, and the like.

The term conservative substitution also includes the use of a substituted amino acid residue in place of an un-substituted parent amino acid residue provided that antibodies raised to the substituted polypeptide also immunoreact with the un-substituted polypeptide.

Mdifications of this primary amino acid sequence may result in proteins, which have substantially equivalent activity as compared to the unmodified counterpart polypeptide, and thus may be considered functional analogs of the parent proteins. Such modifications may be deliberate, e.g. as by site-directed mutagenesis, or they may occur spontaneously, and include splice variants, isoforms, homologues from other species, and polymorphisms. Such functional analogs are also contemplated according to the invention.

An alignment of 99 C-terminal amino acids from humans, mice and rats is shown below:

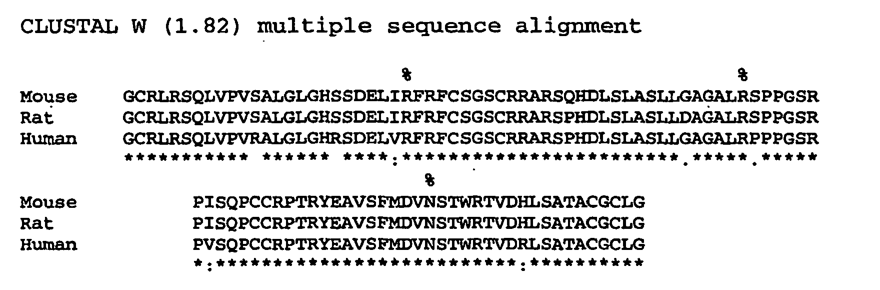

Substitutions are preferably conducted at positions of non-conservancy marked with "no star", ":" pr ":".

Other preferred positions for substitution are denoted with "%".

Moreover, modifications of the primary amino acid sequence may result in proteins, which do not retain the biological activity of the parent protein, including dominant negative forms, etc. A dominant negative protein may interfere with the wild-type protein by binding to, or otherwise sequestering regulating agents, such as upstream or downstream components, that normally interact functionally with the polypeptide. Such dominant negative forms are also contemplated according to the invention.

Biologically active forms of truncated neublastin are known from WO 02/072826 (NsGene and Biogen). Truncated and mutated neublastin molecules are also known from WO 02/060929 (Biogen), especially mutated neublastin comprising an amino acid sequence derived from amino acids 8-113 of SEQ ID No. 14, wherein the variant neublastin polypeptide includes one or more of the amino acid substitutions selected from the group consisting of: an amino acid other than arginine at position 14 in the amino acid sequence of said variant polypeptide, an amino acid other than arginine at position 39 in the amino acid sequence of said variant polypeptide, an amino acid other than arginine at position 68 of said variant polypeptide, and an amino acid other than asparagine at position 95 of said variant polypeptide, for example to a lysine residue, wherein the positions of said amino acids are numbered in accordance with the polypeptide sequence of SEQ ID NO: 10. The mutated forms may be truncated as described above or include the whole length of the mature protein (amino acids 1-113 of SEQ ID NO 14). Preferably the amino acid at position 14, 39 or 68 is a lysine.

### Cleavage of signal peptide

Before deciding on a specific neublastin form to incorporate into an expression construct, the likelihood of cleavage of the signal peptide, such as lgsp can be checked using state of the art prediction tools. One such preferred prediction tool is the SignalP software available at the SignalP WWW server (http://www.cbs.dtu.dk/services/SignalP-2.0/), or preferably, the newer version 3.0 available from the same server (http://www.cbs.dtu.dk/services/SignalP-3.0/).

The **SignalP** WWW server will return three scores between 0 and 1 for each position in your sequence:

### C-score (raw cleavage site score)

The output score from networks trained to recognize cleavage sites νs*.* other sequence positions. Trained to be:
**high** at position +1 (immediately after the cleavage site)
**low** at all other positions.

### S-score (signal peptide score)

The output score from networks trained to recognize signal peptide *vs.* non-signal-peptide positions. Trained to be:
**high** at all positions before the cleavage site
**low** at 30 positions after the cleavage site and in the N-terminals of non-secretory proteins.

### Y-score (combined cleavage site score)

The prediction of cleavage site location is optimized by observing where the C-score is high *and* the S-score changes from a high to a low value. The Y-score formalizes this by combining the height of the C-score with the slope of the S-score.
Specifically, the Y-score is a geometric average between the C-score and a smoothed derivative of the S-score (*i.e*., the difference between the mean S-score over *d* positions before and *d* positions after the current position, where *d* varies with the chosen network ensemble).
All three scores are averages of five networks trained on different partitions of the data.
For each sequence, **SignalP** will report the maximal C-, S-, and Y-scores, and the mean S-score between the N-terminal and the predicted cleavage site. These values are used to distinguish between signal peptides and non-signal peptides. If your sequence is predicted to have a signal peptide, the cleavage site is predicted to be immediately before the position with the maximal Y-score.
For a typical **signal peptide,** the C- and Y-scores will be high at position +1, while the S-score will be high before the cleavage site and low thereafter.
For comparison the prediction can be compared to the predicted cleavage of the wildtype neublastin signal peptide (cleavage between amino acids no 39 and 40 of pre-pro NBN).

Preferred neublastins are those which have a predicted cleavage between the signal peptide and the neublastin in either the SignalP-NN or the SignalP-HMM program. These include but are not limited to NBN113, NBN106, NBN104, NBN102, and NBN99. Particularly preferred are neublastins which have a predicted signal peptide at this position in both SignalP-NN and SignalP-HMM. These include NBN113 and NBN99.

The newer version (3.0) also includes a new score D or Dmax (Discrimination score) that describes "signal peptidedness" that is found to correlate to level of secretion using said signal peptide with the protein in question.

It is preferred that a signal peptide used in the present invention exhibits a Dmax value of at least 0.5, such as at least 0.6, such at least 0.7, such as at least 0.8, with the neublastin polypeptide selected.

References: Henrik Nielsen, Jacob Engelbrecht, Søren Brunak and Gunnar von Heijne: Identification of prokaryotic and eukaryotic signal peptides and prediction of their cleavage sites. Protein Engineering, 10, 1-6 (1997). For the SignaIP-HMM output model: Henrik Nielsen and Anders Krogh: Prediction of signal peptides and signal anchors by a hidden Markov model. In Proceedings of the Sixth International Conference on Intelligent Systems for Molecular Biology (ISMB 6), AAAI Press, Menlo Park, California, pp. 122-130 (1998). Improved prediction of signal peptides - SignalP 3.0. Jannick Dyrløv Bendtsen, Henrik Nielsen, Gunnar von Heijne and Søren Brunak. JMB (2004). Prediction of signal peptides and signal anchors by a hidden Markov model. Henrik Nielsen and Anders Krogh. Proceedings of the Sixth International Conference on Intelligent Systems for Molecular Biology (ISMB 6), AAAI Press, Menlo Park, California, pp. 122-130, 1998.

### Medical use

In one aspect the invention relates to the use of the vector according to the invention for the preparation of a medicament for the treatment of a nervous system disorder. The nervous system disorder can be a disorder of the peripheral nervous system or the central nervous system. Neublastin is useful for treating a defect in a neuron, including without limitation lesioned neurons and traumatized neurons. Peripheral nerves that experience trauma include, but are not limited to, nerves of the medulla or of the spinal cord. Neublastin is useful in the treatment of CNS disorders include, such as a neurodegenerative disease, e.g., cerebral ischaemic neuronal damage; neuropathy, e.g., peripheral neuropathy, Alzheimer's disease, Huntington's disease, amyotrophic lateral sclerosis (ALS). Neublastin is further contemplated for use in the treatment of impaired memory, e.g., memory impairment associated with dementia.

In a further aspect the invention relates to the use of the vector according to the invention for the preparation of a medicament for the treatment of Neublastin is also Neublastin is also known as a therapeutic candidate for treating peripheral neuropathies, such as neuropathic pain in a mammal. The neuropathic pain may be associated with toxin-induced nerve damage, pathogen-induced nerve damage, trauma-induced nerve damage, drug-induced nerve damage, idiopathic neuropathy, diabetic neuropathy, inflammation-induced nerve damage, or neurodegeneration. Neublastin can also be used for treating peripheral neuropathy in a mammal. The peripheral neuropathy may include the group consisting of trauma-induced neuropathies, viral-induced neuropathies, chemotherapy-induced neuropathies, toxin-induced neuropathies, drug-induced neuropathies, vitamin-deficiency-induced neuropathies; idiopathic neuropathies; and diabetic neuropathies. Methods and compositions for treatment of neuropathic pain using neublastin are disclosed in WO 02/078730 (Biogen).

Preferably, Neublastin is used for treating a disorder selected from the group consisting of peripheral neuropathy including neuropathic pain, spinal cord injury, spinal root avulsion, tic doloreaux, causalgia, corneal wounds and retinopathies.

According to one preferred embodiment of the invention the neurodegenerative disease to be treated is Parkinson's disease. Neublastin is known to increase survival of dopaminergic neurons (WO 00/01815 NsGene; Baloh et al 1998 Neuron 21:1291-1302).

The vectors, capsules, and compositions of the present invention can also be used for the treatment of eye diseases, such as retinitis pigmentosa, macular degeneration, glaucoma, and diabetic retinopathy. Neublastin may also be used in the treatment of corneal wounds and ulcers (EP 1 223 966 Biopharm).

Nervous system diseases may be treated by administering to an individual in need thereof a therapeutically effective amount of the vector of the invention; or a therapeutically effective amount of the pharmaceutical composition of the invention.

Also provided are stereotaxic coordinates for the portions of the brain to be transduced to or into which to transplant naked or encapsulated cells (Table II):

**TABLE II**

| BRAIN REGION | MEDIAL-LATERAL DIMENSION | DORSAL-VENTRAL DIMENSION | ANTERIOR-POSTERIOR DIMENSION |
|---|---|---|---|
| Gpe | 1.6 to 2.7 | 1.0 to -1.0 | 2.0 to -1.0 |
| Gpi | 0.5 to 2.0 | 0.5 to -0.7 | 0.7 to 2.0 |
| SNr | 0.5 to 1.5 | -0.6 to -1.5 | 0.7 to -0.7 |
| STN | 0.5 to 2.0 | 0.0 to -1.0 | 0.6 to -1.0 |
| NBM | 1.5 to 2.5 | 0.0 to -1.2 | 0.5 to 1.6 |
| Striatum: | 0.5 to 2.0 | 1.5 to 3.0 | 1.5 to 3.0 |
| caudate | 1.2 to 3.3 | 1.5 to -1.0 | 2.5 to -1.2 |
| putamen | | | |

In the foregoing table: the medial-lateral dimensions are relative to midline of the brain; the anterior-posterior dimensions are relative to the midpoint between the anterior commissure and posterior commissure with negative indicating the posterior direction; the dorsal-ventral dimensions are relative to a line connecting the midpoints of the anterior and posterior commissures with negative being ventral to said line; all dimensions are in centimeters; and Gpe means external segment of globus pallidus; Gpi means internal segment of globus pallidus; Snr means substantia nigra pars reticulata; STN means subthalamic nucleus; NBM means nucleus basalis of meynert; and caudate means caudate nucleus.

Instead of in vivo transduction, nervous system diseases can be treated by transplanting to an individual in need thereof:
i. a therapeutically effective amount of the transduced or transfected cells according to the invention; or
ii. an implantable device comprising transduced or transfected cells.

Preferably the transplantation comprises cells or implantable devices.

Said transplantation may comprise an autologous transplant, an allogeneic transplant or a xenogeneic transplant.

### Target Tissues for Treatment of Neurodegenerative Disorders in the Central Nervous system

An important parameter is the selection of a suitable target tissue. A region of the brain is selected for its retained responsiveness to neurotrophic factors. Targeting of an area may be achieved by delivering a dosage unit of a gene therapy vector as herein described or by implanting naked or encapsulated cells according to the invention.

In humans, CNS neurons which retain responsiveness to neurotrophic factors into adulthood include the cholinergic basal forebrain neurons, the entorhinal cortical neurons, the thalamic neurons, the locus coeruleus neurons, the spinal sensory neurons and the spinal motor neurons.

An initial scan, such as an MRI scan, may be performed on the patient to determine the precise location of the treatment site. For example, in treating Parkinson's disease, the basal ganglia, including substantia nigra, are treatment sites. The affected areas of the brain will likely of a size such that selection of 5 or fewer delivery sites will be sufficient for restoration of a clinically significant number of dopaminergic neurons. The same number of delivery sites may apply outside the brain.

For in vivo gene therapy, delivery may be systemic or local. By systemic delivery is intended administration of gene therapy vector intramuscularly, subcutaneously, or intraperiotoneally, which will result in continuous release of Neublastin to the circulatory system.

For in vivo gene therapy, specific in vivo gene delivery sites are selected so as to cluster in an area of neuronal or terminal loss. Such areas may be identified clinically using a number of known techniques, including magnetic resonance imaging (MRI) and biopsy. In humans, non-invasive, in vivo imaging methods such as MRI will be preferred. Once areas of neuronal or terminal loss are identified, delivery sites are selected for stereotaxic distribution so each unit dosage of Neublastin is delivered into the brain or spinal cord at, or within 500 µm from, a targeted cell, and no more than about 10 mm from another delivery site. Within the brain, gene therapy vector may be administered to the parenchyma or the ventricles.

Within the eye, gene therapy vector may be administered to the vitreous, the subretinal space and to the sub-tenar capsule.

For the treatment of peripheral neuropathy including neuropathic pain, the gene therapy vector may be administered to an area of the body involved in transmission of pain sensation. Such area may include the spinal cord and intrathecal administration.

In one embodiment, the vector is administered intramuscularly, subcutaneously, or intraperitoneally. In another embodiment, the vector or composition is administered to an area of the body involved in transmission of pain sensation. In a third embodiment, the vector or composition is administered intrathecally or to the spinal cord. In a fourth embodiment, the vector is administered to the brain, including the parenchyma, and the ventricles. In a fifth embodiment, the vector is administered into the eye, including the vitreous, subretinal space, and sub-tenar capsule.

### Dosing Requirements and Delivery Protocol for in vivo gene therapy

A further important parameter is the dosage of neublastin to be delivered into the target tissue. In this regard, "unit dosage" refers generally to the concentration of neublastin/ml of neublastin composition. For viral vectors, the neublastin concentration is defined by the number of viral particles/ml of neurotrophic composition. Optimally, for delivery of neublastin using a viral expression vector, each unit dosage of neublastin will comprise 2.5 to 25 µL of a neublastin composition, wherein the composition includes a viral expression vector in a pharmaceutically acceptable fluid and provides from 10¹⁰ up to 10¹⁵ neublastin containing viral particles per ml of neublastin composition. Such high titers are particularly used for adeno-associated virus. For lentivirus, the titer is normally lower, such as from 10⁸ to 10¹⁰ transducing units per ml (TU/ml), determined as described in Example 2.

The neublastin composition is delivered to each delivery cell site in the target tissue by microinjection, infusion, scrape loading, electroporation or other means suitable to directly deliver the composition directly into the delivery site tissue through a surgical incision. The delivery is accomplished slowly, such as over a period of about 5-10 minutes (depending on the total volume of neublastin composition to be delivered).

Those of skill in the art will appreciate that the direct delivery method employed by the invention obviates a limiting risk factor associated with in vivo gene therapy; to wit, the potential for transduction of non-targeted cells with the vector carrying the neublastin encoding transgene. In the invention, delivery is direct and the delivery sites are chosen so diffusion of secreted neublastin takes place over a controlled and pre-determined region of the brain to optimize contact with targeted neurons, while minimizing contact with non-targeted cells.

### Gene therapy vectors

Broadly, gene therapy seeks to transfer new genetic material to the cells of a patient with resulting therapeutic benefit to the patient. Such benefits include treatment or prophylaxis of a broad range of diseases, disorders and other conditions.

Ex vivo gene therapy approaches involve modification of isolated cells, which are then infused, grafted or otherwise transplanted into the patient. See, e.g., U.S. Pat Nos. 4,868,116, 5,399,346 and 5,460,959. In vivo gene therapy seeks to directly target host patient tissue in vivo.

Viruses useful as gene transfer vectors include papovavirus, adenovirus, vaccinia virus, adeno-associated virus, herpesvirus, and retroviruses. Suitable retroviruses include the group consisting of HIV, SIV, FIV, EIAV, MoMLV.

Preferred viruses for treatment of disorders of the nervous system are lentiviruses and adeno-associated viruses. Both types of viruses can integrate into the genome without cell divisions, and both types have been tested in pre-clinical animal studies for indications of the nervous system, in particular the central nervous system.

Methods for preparation of AAV are described in the art, e.g. US 5,677,158. US 6,309,634 and US 6,683,058 describe examples of delivery of AAV to the central nervous system.

Special and preferred types of retroviruses include the lentiviruses which can transduce a cell and integrate into its genome without cell division. Thus preferably the vector is a replication-defective lentivirus particle. Such a lentivivus particle can be produced from a lentiviral vector comprising a 5' lentiviral LTR, a tRNA binding site, a packaging signal, a promoter operably linked to a polynucleotide signal encoding said fusion protein, an origin of second strand DNA synthesis and a 3' lentiviral LTR. Methods for preparation and in vivo administration of lentivirus to neural cells are described in US 20020037281 (Methods for transducing neural cells using lentiviral vectors).

Retroviral vectors are the vectors most commonly used in human clinical trials, since they carry a 7-8 kb which is more than many other viral vectors and since they have the ability to infect cells and have their genetic material stably integrated into the host cell with high efficiency. See, e.g., WO 95/30761; WO 95/24929. Oncovirinae require at least one round of target cell proliferation for transfer and integration of exogenous nucleic acid sequences into the patient. Retroviral vectors integrate randomly into the patient's genome.

Two classes of retroviral particles have been described; ecotropic, which can infect mouse cells efficiently, and amphotropic, which can infect cells of many species. A third class includes xenotrophic retrovirus which can infect cells of another species than the species which produced the virus. Their ability to integrate only into the genome of dividing cells has made retroviruses attractive for marking cell lineages in developmental studies and for delivering therapeutic or suicide genes to cancers or tumors. These vectors may be particularly useful in the central nervous system, where there is a relative lack of cell division in adult patients.

For use in human patients, the retroviral vectors must be replication defective. This prevents further generation of infectious retroviral particles in the target tissue - instead the replication defective vector becomes a "captive" transgene stable incorporated into the target cell genome. Typically in replication defective vectors, the gag, env, and pol genes have been deleted (along with most of the rest of the viral genome). Heterologous DNA is inserted in place of the deleted viral genes. The heterologous genes may be under the control of the endogenous heterologous promoter, another heterologous promoter active in the target cell, or the retroviral 5' LTR (the viral LTR is active in diverse tissues). Typically, retroviral vectors have a transgene capacity of about 7-8 kb.

Replication defective retroviral vectors require provision of the viral proteins necessary for replication and assembly in trans, from, e.g., engineered packaging cell lines. It is important that the packaging cells do not release replication competent virus and/or helper virus. This has been achieved by expressing viral proteins from RNAs lacking the Ψ signal, and expressing the gag/pol genes and the env gene from separate transcriptional units. In addition, in some packaging cell lines, the 5' LTR's have been replaced with non-viral promoters controlling expression of these genes and polyadenylation signals have been added. These designs minimize the possibility of recombination leading to production of replication competent vectors, or helper viruses. See, e.g., U.S. Pat. No. 4,861,719.

The invention further relates to a nucleic acid construct comprising a nucleic acid sequence encoding a neublastin polypeptide and a signal sequence. The signal peptide and the neublastin polypeptide are as described above. Accordingly, in some embodiments, the nucleic acid construct encodes a sequence consisting of the 113 C terminal codons of the pre pro neublastin polypeptide. In certain embodiments, the nucleic acid encodes a sequence consisting of the 104 C terminal codons of the pre pro neublastin polypeptide.

The nucleic acid sequence may comprise a heterologous signal peptide, such as an albumin signal sequence, *e.g*., a rat albumin signal sequence, and comprises the nucleic acid sequence of SEQ ID NO: 65. In some embodiments, the nucleic acid construct encodes a modified albumin signal sequence, *e.g*., a rat albumin signal sequence. One exemplary embodiment is a nucleic acid construct comprising SEQ ID NO: 69. In other embodiments, the nucleic acid construct encodes a human growth hormone signal sequence. One exemplary embodiment is a nucleic acid construct comprising SEQ ID NO: 67. The human growth hormone signal sequence may comprise an intron.

In a specific embodiment of the invention, the nucleic acid construct contains a nucleic acid sequence optimized for expression in a transfected host cell. Optimization of codon usage can be advantageous by providing increased polypeptide yield, or improved efficiency of transcription or translation. One exemplary embodiment of an optimized nucleic acid construct of the invention is set forth in SEQ ID NO: 59.

Due to the known degeneracy of the genetic code, wherein more than one codon can encode the same amino acid, a DNA sequence can vary from that shown in SEQ ID NOS: 65, 67, or 69 and still encode a polypeptide having the corresponding amino acid sequence of SEQ ID NOS: 66, 68, or 70 respectively. Such variant DNA sequences can result from silent mutations (e.g. occurring during PCR amplification), or can be the product of deliberate mutagenesis of a native sequence, *e.g.*, codon optimization.

The nucleic acid construct can be a vector. Examples of suitable plasmid vectors include but are not limited to pFRT/lac Zeo, pFRT/dhfr-1, (Invitrogen, Carlsbad, CA) pUC, pGEM and pGEX (Pharmacia, Peapack, NJ). Other suitable vectors include viral vectors (*e.g*. replication defective retroviruses, adenoviruses and adeno-associated viruses), Which serve equivalent functions.

### Expression vectors

Expression vectors may include one or more regulatory sequences operatively linked to the nucleic acid sequence to be expressed. Examples of regulatory sequences include promoters, enhancers, and polyadenylation signals. Such regulatory sequences are described, for example, in Goeddel, 1990 Methods Enzymol., 185:3.' Regulatory sequences include those which direct constitutive expression of a nucleotide sequence in many types of host cells and those which direct expression of the nucleotide sequence only in certain host cells (*e.g*. tissue-specific regulatory sequences). It will be appreciated by those skilled in the art that the design of the expression vector will depend on such factors as the choice of the host cell to be transformed, the level of expression of protein desired, and the like. The expression vectors of the invention can be introduced into host cells to thereby produce proteins or peptides.

Construction of vectors for recombinant expression of neublastin for use in the invention may be accomplished using conventional techniques which do not require detailed explanation to one of ordinary skill in the art For review, however, those of ordinary skill may wish to consult Maniatis et al., in Molecular Cloning: A Laboratory Manual, Cold Spring Harbor Laboratory (NY 1982).

Briefly, construction of recombinant expression vectors employs standard ligation techniques. For analysis to confirm correct sequences in vectors constructed, the ligation mixtures may be used to transfect/transduce a host cell and successful genetically altered cells may be selected by antibiotic resistance where appropriate.

Vectors from the transfected/transduced cells are prepared, analysed by restriction and/or sequenced by, for example, the method of Messing, et al. (Nucleic Acids Res., 9: 309-, 1981), the method of Maxam, et al. (Methods in Enzymology, 65: 499, 1980), the Sanger dideoxy method, or other suitable methods which will be known to those skilled in the art.

Size separation of cleaved fragments is performed using conventional gel electrophoresis as described, for example, by Maniatis, et al. (Molecular Cloning, pp. 133-134, 1982).

Expression elements employed in the invention may vary in their strength and specificities. Depending on the host/vector system utilized, any of a number of suitable transcription and translation elements, including constitutive and inducible promoters, may be used in the expression vector. When cloning in mammalian cell systems, promoters derived from the genome of mammalian cells (*e.g*. metallothionein promoter) or from mammalian viruses (*e.g*. the CMV promoter, the adenovirus late promoter; the vaccinia virus 7.5 K promoter) may be used; when generating cell lines that contain multiple copies of expression product, SV40-, BPV- and EBV-based vectors may be used with an appropriate selectable marker.

Expression of a gene is controlled at the transcription, translation or post-translation levels. Transcription initiation is an early and critical event in gene expression. This depends on the promoter and enhancer sequences and is influenced by specific cellular factors that interact with these sequences. The transcriptional unit of many prokaryotic genes consists of the promoter and in some cases enhancer or regulator elements (Banerji et al., Cell 27: 299 (1981); Corden et al., Science 209: 1406 (1980); and Breathnach and Chambon, Ann. Rev. Biochem. 50: 349 (1981)). For retroviruses, control elements involved in the replication of the retroviral genome reside in the long terminal repeat (LTR) (Weiss et al., eds., The molecular biology of tumor viruses: RNA tumor viruses, Cold Spring Harbor Laboratory, (NY 1982)). Moloney murine leukemia virus (MLV) and Rous sarcoma virus (RSV) LTRs contain promoter and enhancer sequences (Jolly et al., Nucleic Acids Res. 11: 1855 (1983); Capecchi et al., In: Enhancer and eukaryotic gene expression, Gulzman and Shenk, eds., pp. 101-102, Cold Spring Harbor Laboratories (NY 1991). Other potent promoters include those derived from cytomegalovirus (CMV) and other wild-type viral promoters.

Promoter and enhancer regions of a number of non-viral promoters have also been described (Schmidt et al., Nature 314: 285 (1985); Rossi and deCrombrugghe, Proc. Natl. Acad. Sci. USA 84: 5590-5594 (1987)). Methods for maintaining and increasing expression of transgenes in quiescent cells include the use of promoters including collagen type I (1 and 2) (Prockop and Kivirikko, N. Eng. J. Med. 311: 376 (1984) ; Smith and Niles, Biochem. 19: 1820 (1980) ; de Wet et al., J. Biol. Chem., 258: 14385 (1983)), SV40 and LTR promoters.

In mammalian host cells, a number of viral based expression systems may be utilized. In cases where an adenovirus is used as an expression vector, a coding sequence may be ligated to an adenovirus transcription/translation control complex, *e.g*., the late promoter and tripartite leader sequence. This chimeric gene may then be inserted in the adenovirus genome by in vitro or in vivo recombination. Insertion in a non-essential region of the viral genome (*e.g*. region E1 or E3) will result in a recombinant virus that is viable and capable of expressing peptide in infected hosts (see *e.g.* Logan & Shenk, 1984, Proc. Natl. Acad. Sci. USA, 81:3655). Alternatively, the vaccinia 7.5 K promoter may be used (see, *e.g.,* Mackett et al., 1982, Proc. Natl. Acad. Sci. USA, 79:7415; Mackett et al., 1984, J. Virol., 49:857; Panicali et al., 1982, Proc. Natl. Acad. Sci. USA, 79:4927).

According to one embodiment of the invention, the promoter is a constitutive promoter selected from the group consisting of: ubiquitin promoter, CMV promoter, JeT promoter, SV40 promoter, and Elongation Factor 1 alpha promoter (EF1-alpha).

Examples of inducible/repressible promoters include: Tet-On, Tet-Off, Rapamycin-inducible promoter, Mx1.

In addition to using viral and non-viral promoters to drive transgene expression, an enhancer sequence may be used to increase the level of transgene expression. Enhancers can increase the transcriptional activity not only of their native gene but also of some foreign genes (Armelor, Proc. Natl. Acad. Sci. USA 70: 2702 (1973)). For example, in the present invention collagen enhancer sequences are used with the collagen promoter 2 (I) to increase transgene expression. In addition, the enhancer element found in SV40 viruses may be used to increase transgene expression. This enhancer sequence consists of a 72 base pair repeat as described by Gruss et al., Proc. Natl. Acad. Sci. USA 78: 943 (1981); Benoist and Chambon, Nature 290: 304 (1981), and Fromm and Berg, J. Mol. Appl. Genetics, 1: 457 (1982). This repeat sequence can increase the transcription of many different viral and cellular genes when it is present in series with various promoters (Moreau et al., Nucleic Acids Res. 9: 6047 (1981).

Transgene expression may also be increased for long term stable expression using cytokines to modulate promoter activity. Several cytokines have been reported to modulate the expression of transgene from collagen 2 (I) and LTR promoters (Chua et al., connective Tissue Res., 25: 161-170 (1990); Elias et al., Annals N. Y. Acad. Sci., 580 : 233-244 (1990)); Seliger et al., J. Immunol. 141: 2138-2144 (1988) and Seliger et al., J. Virology 62: 619-621 (1988)). For example, transforming growth factor (TOF), interleukin (IL)-I, and interferon (INF) down regulate the expression of transgenes driven by various promoters such as LTR. Tumor necrosis factor (TNF) and TGF 1 up regulate, and may be used to control, expression of transgenes driven by a promoter. Other cytokines that may prove useful include basic fibroblast growth factor (bFGF) and epidermal growth factor (EGF).

Collagen promoter with the collagen enhancer sequence (Coll (E)) can also be used to increase transgene expression by suppressing further any immune response to the vector which may be generated in a treated brain notwithstanding its immune-protected status. In addition, anti-inflammatory agents including steroids, for example dexamethasone, may be administered to the treated host immediately after vector composition delivery and continued, preferably, until any cytokine-mediated inflammatory response subsides. An immunosuppression agent such as cyclosporin may also be administered to reduce the production of interferons, which downregulates LTR promoter and Coll (E) promoter-enhancer, and reduces transgene expression.

The vector may comprise further sequences such as a sequence coding for the Cre-recombinase protein, and LoxP sequences. A further way of ensuring temporary expression of the neublastin is through the use of the Cre-LoxP system which results in the excision of part of the inserted DNA sequence either upon administration of Cre-recombinase to the cells (Daewoong et al, Nature Biotechnology 19:929-933) or by incorporating a gene coding for the recombinase into the virus construct (Plück, lnt J Exp Path, 77:269-278). Incorporating a gene for the recombinase in the virus construct together with the LoxP sites and a structural gene (a neublastin in the present case) often results in expression of the structural gene for a period of approximately five days.

Vectors used in methods of the invention may also include a nucleic acid sequence encoding a selectable marker that can be used to identify successfully transformed host cells. Suitable selectable markers for use in cultured mammalian cells include genes that confer resistance to drugs, such as neomycin, hygromycin, and methotrexate. The selectable marker may be an amplifiable selectable marker. One amplifiable selectable marker is the DHFR gene. Another suitable amplifiable marker is the DHFRr cDNA (Simonsen and Levinson, 1983, Proc. Natl. Acad. Sci. (USA) 80:2495). Additional selectable markers are reviewed by Thilly *(Mammalian Cell Technology,* Butterworth Publishers, Stoneham, MA). Suitable selectable markers can be chosen by any person skilled in the art. Selectable markers may be introduced into the host cell in the same vector as the neublastin pre sequence, or as part of a separate vector. The selectable marker and the neublastin sequence may be under the control of different promoters or the same promoter, the latter arrangement producing a dicistronic message. Constructs of this type are known in the art (see *e.g*. U.S. Pat. No. 4,713,339).

Expression vectors used in the methods of the invention may also encode tags that facilitate purification of the recombinantly produced neublastin polypeptide. Examples include, but are not limited to, vector pUR278 (Ruther et al., 1983, EMBO J., 2:1791) in which the coding sequences of the neublastin polypeptide described herein may be ligated into the vector in frame with the lac z coding region so that a hybrid protein is produced; pGEX vectors may also be used to express the neublastin polypeptide with a glutathione S-transferase (GST) tag. These proteins are usually soluble and can easily be purified from cells by adsorption to glutathione-agarose beads followed by elution in the presence of free glutathione. The vectors include cleavage sites (thrombin or factor Xa protease or PreScission Protease^{™} (Pharmacia, Peapack, N.J.)) for easy removal of the tag after purification. Other fusion tags are known in the art, *e.g*., histidine tags, maltose binding protein tags.

### Pharmaceutical preparations for gene therapy

To form a neublastin composition for use in the invention; neublastin encoding expression viral vectors may be placed into a pharmaceutically acceptable suspension, solution or emulsion. Suitable mediums include saline and liposomal preparations.

More specifically, pharmaceutically acceptable carriers may include sterile aqueous of non-aqueous solutions, suspensions, and emulsions. Examples of nonaqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils.

Intravenous vehicles include fluid and nutrient replenishers, electrolyte replenishers (such as those based on Ringer's dextrose), and the like.

Preservatives and other additives may also be present such as, for example, antimicrobials, antioxidants, chelating agents, and inert gases and the like. Further, a composition of neublastin transgenes may be lyophilized using means well known in the art, for subsequent reconstitution and use according to the invention.

A colloidal dispersion system may also be used for targeted gene delivery.

Colloidal dispersion systems include macromolecule complexes, nanocapsules, microspheres, beads, and lipid-based systems including oil-in-water emulsions, micelles, mixed micelles, and liposoms. Liposomes are artificial membrane vesicles which are useful as delivery vehicles in vitro and in vivo. It has been shown that large unilamellar vesicles (LUV), which range in size from 0.2-4.0 µm can encapsulate a substantial percentage of an aqueous buffer containing large macro molecules. RNA, DNA and intact virions can be encapsulated within the aqueous interior and be delivered to cells in a biologically active form (Fraley, et al., Trends Biochem. Sci., 6: 77,1981). In addition to mammalian cells, liposomes have been used for delivery of operatively encoding transgenes in plant, yeast and bacterial cells. In order for a liposome to be an efficient gene transfer vehicle, the following characteristics should be present: (1) encapsulation of the genes encoding the neublastin at high efficiency while not compromising their biological activity; (2) preferential and substantial binding to a target cell in comparison to non-target cells; (3) delivery of the aqueous contents of the vesicle to the target cell cytoplasm at high efficiency; and (4) accurate and effective expression of genetic information (Mannino, et al., Biotechniques, 6: 682,1988).

The composition of the liposome is usually a combination of phospholipids, particularly high-phase-transition-temperature phospholipids, usually in combination with steroids, especially cholesterol. Other phospholipids or other lipids may also be used. The physical characteristics of liposomes depend on pH, ionic strength, and the presence of divalent cations.

Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Particularly useful are diacylphosphatidylglycerols, where the lipid moiety contains from 14-18 carbon atoms, particularly from 16-18 carbon atoms, and is saturated. Illustrative phospholipids include egg phosphatidylcholine, dipalmitoylphosphatidylcholine and distearoylphosphatidylcholine.

The targeting of liposomes can be classified based on anatomical and mechanistic factors. Anatomical classification is based on the level of selectivity, for example, organ-specific, cell-specific, and organelle-specfic. Mechanistic targeting can be distinguished based upon whether it is passive or active. Passive targeting utilizes the natural tendency of liposomes to distribute to cells of the reticulo-endothelial system (RES) in organs which contain sinusoidal capillaries.

Active targeting, on the other hand, involves alteration of the liposome by coupling the liposome to a specific ligand such as a monoclonal antibody, sugar, glycolipid, or protein, or by changing the composition or size of the liposome in order to achieve targeting to organs and cell types other than the naturally occurring sites of localization.

The surface of the targeted gene delivery system may be modified in a variety of ways. In the case of a liposomal targeted delivery system, lipid groups can be incorporated into the lipid bilayer of the liposome in order to maintain the targeting ligand in stable association with the liposomal bilayer. Various linking groups can be used for joining the lipid chains to the targeting ligand.

A further example of a delivery system includes transplantation into the therapeutic area of a composition of packaging cells capable of producing vector particles as described in the present invention. Methods for encapsulation and transplantation of such cells are known in the art, in particular from WO 97/44065 (Cytotherapeutics). By selecting a packaging cell line capable of producing lentiviral particles, transduction of non-dividing cells in the therapeutic area is obtained. By using retroviral particles capable of transducing only dividing cells, transduction is restricted to de-novo differentiated cells in the therapeutic area.

### Methods for Delivery of Gene Therapy Vector Composition

Following the protocol defined by the invention, direct delivery of a neublastin composition may be achieved by means familiar to those of skill in the art, including microinjection through a surgical incision (see, e.g., Capecchi, Cell, 22: 479-488 (1980)); electroporation (see, e.g., Andreason and Evans, Biotechniques, 6: 650-660 (1988)); infusion, chemical complexation with a targeting molecule or co-precipitant (e.g., liposome, calcium), and microparticle bombardment of the target tissue (Tang, et al., Nature, 356: 152-154 (1992)).

### Encapsulation of cells

Encapsulated cell therapy is based on the concept of isolating cells from the recipient host's immune system by surrounding the cells with a semipermeable biocompatible material before implantation within the host The invention includes a device in which cells are encapsulated in an immunoisolatory capsule. An "immunoisolatory capsule" means that the capsule, upon implantation into a recipient host, minimizes the deleterious effects of the host's immune system on the cells in the core of the device. Cells are immunoisolated from the host by enclosing them within implantable polymeric capsules formed by a microporous membrane. This approach prevents the cell-to cell contact between host and implanted tissues, eliminating antigen recognition through direct presentation. The membranes used can also be tailored to control the diffusion of molecules, such as antibody and complement, based on their molecular weight (Lysaght et al., 56 J. Cell Biochem. 196 (1996), Colton, 14 Trends Biotechnol. 158 (1996)). Using encapsulation techniques, Cells can be transplanted into a host without immune rejection, either with or without use of immunosuppressive drugs. Useful biocompatible polymer capsules usually contain a core that contains cells, either suspended in a liquid medium or immobilized within an immobilizing matrix, and a surrounding or peripheral region of permselective matrix or membrane ("jacket") that does not contain isolated cells, that is biocompatible, and that is sufficient to protect cells in the core from detrimental immunological attack. Encapsulation hinders elements of the immune system from entering the capsule, thereby protecting the encapsulated cells from immune destruction. The semipermeable nature of the capsule membrane also permits the biologically active molecule of interest to easily diffuse from the capsule into the surrounding host tissue.

The capsule can be made from a biocompatible material. A "biocompatible material" is a material that, after implantation in a host, does not elicit a detrimental host response sufficient to result in the rejection of the capsule or to render it inoperable, for example through degradation. The biocompatible material is relatively impermeable to large molecules, such as components of the host's immune system, but is permeable to small molecules, such as insulin, growth factors, and nutrients, while allowing metabolic waste to be removed. A variety of biocompatible materials are suitable for delivery of growth factors by the composition of the invention. Numerous biocompatible materials are known, having various outer surface morphologies and other mechanical and structural characteristics. Preferably the capsule of this invention will be similar to those described by PCT International patent applications WO 92/19195 or WO 95/05452 or U.S. Pat. Nos. 5,639,275; 5,653.975; 4,892,538; 5,156,844; 5,283,187; or U.S. Pat. No. 5,550,050. Such capsules allow for the passage of metabolites, nutrients and therapeutic substances while minimizing the detrimental effects of the host immune system. Components of the biocompatible material may include a surrounding semipermeable membrane and the internal cell-supporting scaffolding. Preferably, the genetically altered cells are seeded onto the scaffolding, which is encapsulated by the permselective membrane. The filamentous cell-supporting scaffold may be made from any biocompatible material selected from the group consisting of acrylic, polyester, polyethylene, polypropylene polyacetonitrile, polyethylene teraphthalate, nylon, polyamides, polyurethanes, polybutester, silk, cotton, chitin, carbon, or biocompatible metals. Also, bonded fiber structures can be used for cell implantation (U.S. Pat. No. 5,512,600). Biodegradable polymers include those comprised of poly(lactic acid) PLA, poly(lactic-coglycolic acid) PLGA, and poly(glycolic acid) PGA and their equivalents. Foam scaffolds have been used to provide surfaces onto which transplanted cells may adhere (PCT International patent application Ser. No. 98/05304). Woven mesh tubes have been used as vascular grafts (PCT International patent application WO 99/52573). Additionally, the core can be composed of an immobilizing matrix formed from a hydrogel, which stabilizes the position of the cells. A hydrogel is a 3-dimensional network of cross-linked hydrophilic polymers in the form of a gel, substantially composed of water.

Various polymers and polymer blends can be used to manufacture the surrounding semipermeable membrane, including polyacrylates (including acrylic copolymers), polyvinylidenes, polyvinyl chloride copolymers, polyurethanes, polystyrenes, polyamides, cellulose acetates, cellulose nitrates, polysulfones (including polyether sulfones), polyphosphazenes, polyacrylonitriles, poly(acrylonitrile/covinyl chloride), as well as derivatives, copolymers and mixtures thereof. Preferably, the surrounding semipermeable membrane is a biocompatible semipermeable hollow fiber membrane. Such membranes and methods of making them are disclosed by U.S. Pat. Nos. 5,284,761 and 5,158,881. The surrounding semipermeable membrane is formed from a polyether sulfone hollow fiber, such as those described by U.S. Pat. No. 4,976,859 or U.S. Pat. No. 4,968,733. An alternate surrounding semipermeable membrane material is poly(acrylonitrile/covinyl chloride).

The capsule can be any configuration appropriate for maintaining biological activity and providing access for delivery of the product or function, including for example, cylindrical, rectangular, disk-shaped, patch-shaped, ovoid, stellate, or spherical. Moreover, the capsule can be coiled or wrapped into a mesh-like or nested structure. If the capsule is to be retrieved after it is implanted, configurations which tend to lead to migration of the capsules from the site of implantation, such as spherical capsules small enough to travel in the recipient host's blood vessels, are not preferred. Certain shapes, such as rectangles, patches, disks, cylinders, and flat sheets offer greater structural integrity and are preferable where retrieval is desired.

When macrocapsules are used, preferably between 10³ and 10⁸ cells are encapsulated, most preferably 10⁵ to 10⁷ cells are encapsulated in each device. Dosage may be controlled by implanting a fewer or greater number of capsules, preferably between 1 and 10 capsules per patient.

The scaffolding may be coated with extracellular matrix (ECM) molecules. Suitable examples of extracellular matrix molecules include, for example, collagen, laminin, and fibronectin. The surface of the scaffolding may also be modified by treating with plasma irradiation to impart charge to enhance adhesion of cells.

Any suitable method of sealing the capsules may be used, including the use of polymer adhesives or crimping, knotting and heat sealing. In addition, any suitable "dry" sealing method can also be used, as described, e.g., in U.S. Pat. No. 5,653,687.

The encapsulated cell devices are implanted according to known techniques. Many implantation sites are contemplated for the devices and methods of this invention. These implantation sites include, but are not limited to, the central nervous system, including the brain, spinal cord (see, U.S. Pat. Nos. 5,106,627, 5,156,844, and 5,554,148), intrathecal implantation, and the aqueous and vitreous humors of the eye (see, PCT International patent application WO 97/34586), the subretinal space, and the sub-tenar capsule. Within the brain, the devices may be implanted in the parenchyma and the ventricles. For systemic delivery, implantation may be intrarmuscular, subcutaneous, or intraperitoneal.

The ARPE-19 cell line is a superior platform cell line for encapsulated cell based delivery technology and is also useful for unencapsulated cell based delivery technology. The ARPE-19 cell line is hardy (i.e., the cell line is viable under stringent conditions, such as implantation in the central nervous system or the intra-ocular environment). ARPE-19 cells can be genetically modified to secrete a substance of therapeutic interest. ARPE-19 cells have a relatively long life span. ARPE-19 cells are of human origin. Furthermore, encapsulated ARPE-19 cells have good in vivo device viability. ARPE-19 cells can deliver an efficacious quantity of growth factor. ARPE-19 cells elicit a negligible host immune reaction. Moreover, ARPE-19 cells are non-tumorigenic.

Methods and apparatus for implantation of capsules into the CNS are described in US 5,487,739.

In one aspect the invention relates to a biocompatible capsule comprising: a core comprising living packaging cells that secrete a viral vector for infection of a target cell, wherein the viral vector is a vector according to the invention; and an external jacket surrounding said core, said jacket comprising a permeable biocompatible material, said material having a porosity selected to permit passage of retroviral vectors of 100 nm diameter thereacross, permitting release of said viral vector from said capsule.

Preferably, the core additionally comprises a matrix, the packaging cells being immobilized by the matrix. According to one embodiment, the jacket comprises a hydrogen or thermoplastic material.

Examples of suitable cells for packaging cell lines include HEK293, NIH3T3, PG13, and ARPE-19 cells. Preferred cells include PG13 and 3T3 cells.

Methods and devices for encapsulation of packaging cells are disclosed in US 6,027,721.

### Host cells

The nucleic acid constructs of the invention can be used to produce neublastin polypeptide. Eukaryotic cells may be transfected with a nucleic acid construct which encodes a recombinant neublastin polypeptide operatively linked to a heterologous signal sequence. Methods of making nucleic acid constructs and transfecting cells with the constructs are known in the art. (See *e.g*., Ausubel et al., eds., 1988, Current Protocols in Molecular Biology, Greene Publishing Associates & Wiley-Interscience: New York; Sambrook et al. 1989, Molecular Cloning: A Laboratory Manual, 2 ed., Cold Spring Harbor Laboratory Press: Cold Spring Harbor, NY). For example, cells can be transfected using electroporation, calcium phosphate precipitation, or infection with a viral vector. In some embodiments, the transformed host cell is a mammalian cell, *e.g*., a CHO cell, a COS cell, a HeLa cell, or an NIH 3T3 cell.

The transformed host cells are cultured in an appropriate growth medium and under conditions such that the secreted neublastin polypeptide is expressed and secreted from the cell. An appropriate growth medium is a medium containing nutrients required for the growth of cells. Nutrients required for cell growth may include a carbon source, a nitrogen source, essential amino acids, vitamins, minerals and growth factors. Optionally, the media can contain bovine calf serum or fetal calf serum. The growth medium can be designed to select for cells containing the nucleic acid construct. This can be done, for example, by drug selection or deficiency in an essential nutrient which is complemented by the selectable marker on the nucleic acid construct or co-transfected with the nucleic acid construct. Cultured mammalian cells are sometimes grown in commercially available serum-containing or serum-free media (e.g. MEM, DMEM)(Invitrogen, Carlsbad, CA). Factors to be considered in the selection of a medium appropriate for the particular cell line used are known in the art.

Thus, in one aspect the invention relates to isolated host cells transduced or transfected with the vector according to the invention. These cells preferably are mammalmalian host cells because these are capable of secreting and processing the encoded neublastin correctly.

Preferred species include the group consisting of rodent (mouse, rat), rabbit, dog, cat, pig, monkey, human being.

Examples of primary cultures and cell lines that are good candidates for transduction with the vectors of the present invention include the group consisting of CHO, HEK293, COS, PC12, HiB5, RN33b, neuronal cells, foetal cells, RPE cell lines, ARPE-19, human immortalised fibroblasts, C2C12, HeLa, HepG2, striatal cells, neurons, astrocytes, intemeurons.

One preferred type of cell line for encapsulation and for naked cell therapy are retinal pigment epithelial cells (RPE cells). The source of RPE cells is by primary cell isolation from the mammalian retina. Protocols for harvesting RPE cells are well-defined (Li and Turner, 1988, Exp. Eye Res. 47:911-917; Lopez et al., 1989, Invest. Ophthalmol. Vis. Sci. 30:586-588) and considered a routine methodology. In most of the published reports of RPE cell cotransplantation, cells are derived from the rat (Li and Turner, 1988; Lopez et al., 1989). Preferably, RPE cells are derived from humans. In addition to isolated primary RPE cells, cultured human RPE cell lines may be used in the practice of the invention.

For in vivo transduction, the preferred group of host cells include striatal cells, neurons, astrocytes and intemeurons. For ex vivo gene therapy, the preferred group of cells include neuronal cells, neuronal precursor cells, neuronal progenitor cells, stem cells and foetal cells. Stem cells and neuronal precursor cells have the advantage that they can integrate into the tissue and migrate. For encapsulation and for implantation of naked cells the preferred cells include retinal pigmented epithelial cells, including ARPE-19 cells; human immortalised fibroblasts; and human immortalised astrocytes. Particularly preferred are ARPE-19

In another embodiment the therapeutic cell line is selected from the group consisting of: human fibroblast cell lines, human astrocyte cell lines, human mesencephalic cell lines, and human endothelial cell lines, preferably immortalised with TERT, SV40T or vmyc.

The method for generating an immortalised human astrocyte cell lines has previously been described (Price TN, Burke JF, Mayne LV. A novel human astrocyte cell line (A735) with astrocyte-specific neurotransmitter function. In Vitro Cell Dev Biol Anim. 1999 May; 35(5):279-88.). This protocol may be used to generate astrocyte cell lines.

The following three modifications of that protocol are preferably made to generate additional human astrocyte cell lines.

Human foetal brain tissue dissected from 5-12 weeks old foetuses may be used instead of 12-16 weeks old tissue.
The immortalisation gene *v-myc,* or TERT (telomerase) may be used instead of the *SV40 T antigen.*
Retroviral gene transfer may be used instead of transfection with plasmids.

The neublastin polypeptide may also be expressed in a transgenic animal, such as a rodent, cow, pig, sheep or goat. A transgenic animal is a non-human animal that has incorporated a foreign gene into its genome such that the foreign gene is passed from parent to offspring. Exogenous genes can be introduced into single-celled embryos (Brinster et al,. 1985, Proc. Natl. Acad Sci. USA, 82:4438). Methods of producing transgenic animals are known in the art, (Wagner et al., 1981, Proc. Natl. Acad. Sci. USA 78:6376; McKnight et al., 1983, Cell 34:335; Brinster et al., 1983, Nature 306:332; Ritchie et al., 1984, Nature 312:511; Baldassarre et al., 2003, Theriogenology 59:831; Robl et al., 2003, Theriogenology 59:107; Malassagne et al., 2003, Xenotransplantation 10(3):267).

### In vitro production of neublastin

In a separate aspect the invention relates to mammalian cells, such as the cells defined above, capable of secreting a neublastin polypeptide in amounts in excess of 500 ng/10⁶ cells/24 hours, more preferably in excess of 600 ng/10⁶ cells/24 hours, more preferably in excess of 700 ng/10⁶ cells/24 hours, more preferably in excess of 800 ng/10⁶ cells/24 hours, more preferably in excess of 900 ng/10⁶ cells/24 hours, such as in excess of 1000 ng/10⁶, cells/24 hours.

Preferably the secreted neublastin is biologically active as determined by the RetL3 ELISA assay described in Example 1. This obviates the need for glycosylation, cleavage and re-folding.

The preferred host cells are selected from the group consisting of CHO, HEK293 COS, PC12, HiB5, RN33b, C2C12, HeLa, HepG2, and ARPE-19 cells. More preferably the group consists of CHO, HEK293, COS, and ARPE-19.

Neublastin or a functional equivalent thereof can be produced by culturing these cells and recovering the neublastin from the culture medium without the need to refold or glycosylate the protein.

Expression can be increased even further by the inclusion of enhancer elements such as WPRE (US 6,136,597).

### Support matrix for neublastin producing cells

The present invention further comprises culturing neublastin producing cells in vitro on a support matrix prior to implantation into the mammalian nervous system. The preadhesion of cells to microcarriers prior to implantation is designed to enhance the long-term viability of the transplanted cells and provide long term functional benefit.

To increase the long term viability of the transplanted cells, i.e., transplanted neublastin secreting cells, the cells to be transplanted can be attached in vitro to a support matrix prior to transplantation. Materials of which the support matrix can be comprised include those materials to which cells adhere following in vitro incubation, and on which cells can grow, and which can be implanted into the mammalian body without producing a toxic reaction, or an Inflammatory reaction which would destroy the implanted cells or otherwise interfere with their biological or therapeutic activity. Such materials may be synthetic or natural chemical substances, or substances having a biological origin.

The matrix materials include, but are not limited to, glass and other silicon oxides, polystyrene, polypropylene, polyethylene, polyvinylidene fluoride, polyurethane, polyalginate, polysulphone, polyvinyl alcohol, acrylonitrile polymers, polyacrylamide, polycarbonate, polypentent, nylon, amylases, natural and modified gelatin and natural and codified collagen, natural and modified polysaccharides, including dextrans and celluloses (e.g., nitrocellulose), agar, and magnetite. Either resorbable or non-resorbable materials may be used. Also intended are extracellular matrix materials, which are well-known in the art. Extracellular matrix materials may be obtained commercially or prepared by growing cells which secrete such a matrix, removing the secreting cells, and allowing the cells which are to be transplanted to interact with and adhere to the matrix. The matrix material on which the cells to be implanted grow, or with which the cells are mixed, may be an indigenous product of RPE cells. Thus, for example, the matrix material may be extracellular matrix or basement membrane material, which is produced and secreted by RPE cells to be implanted.

To improve cell adhesion, survival and function, the solid matrix may optionally be coated on its external surface with factors known in the art to promote cell adhesion, growth or survival. Such factors include cell adhesion molecules, extracellular matrix, such as, for example, fibronectin, laminin, collagen, elastin, glycosaminoglycans, or proteoglycans or growth factors.

Alternatively, if the solid matrix to which the implanted cells are attached is constructed of porous material, the growth- or survival promoting factor or factors may be incorporated into the matrix material, from which they would be slowly released after implantation in vivo.

When attached to the support according to the present invention, the cells used for transplantation are generally on the "outer surface" of the support The support may be solid or porous. However, even in a porous support, the cells are in direct contact with the external milieu without an intervening membrane or other barrier. Thus, according to the present invention, the cells are considered to be on the "outer surface" of the support even though the surface to which they adhere may be in the form of internal folds or convolutions of the porous support material which are not at the exterior of the particle or bead itself.

The configuration of the support is preferably spherical, as in a bead, but may be cylindrical, elliptical, a flat sheet or strip, a needle or pin shape, and the like. A preferred form of support matrix is a glass bead. Another preferred bead is a polystyrene bead.

Bead sizes may range from about 10 µm to 1 mm in diameter, preferably from about 90 µm to about 150 µm. For a description of various microcarrier beads, see, for example, isher Biotech Source 87-88, Fisher Scientific Co., 1987, pp. 72-75; Sigma Cell Culture Catalog, Sigma Chemical Co., St, Louis, 1991, pp. 162-163; Ventrex Product Catalog, Ventrex Laboratories, 1989. The upper limit of the bead's size may be dictated by the bead's stimulation of undesired host reactions, which may interfere with the function of the transplanted cells or cause damage to the surrounding tissue. The upper limit of the bead's size may also be dictated by the method of administration. Such limitations are readily determinable by one of skill in the art.

### Neublastin polypeptide

The invention further relates to a neublastin polypeptide comprising a signal peptide and a neublastin polypeptide, wherein the polypeptide lacks a neublastin pro-region, such as a neublastin polypeptide fused to a signal peptide. In particular, the signal peptide is linked through its C-terminal to the N-terminal of the neublastin polypeptide through a peptide bond. The signal peptide and the neublastin polypeptide are as defined above.

### Examples:

### EXAMPLE 1: In vitro transfection with IgSP-NBN construct

### Construction of IgSP-NBN constructs

IgSP.NBN was generated by overlap PCR. In the first amplification step, the mature fragment of NBN was amplified by PCR from the pUbi1z.NBN.BamHl vector using the primers NBNs-IgSP.Flap (5'- GGTGAATTCGGCTGGGGGCCCGGGCAGCC-3') and NBNas+Xhol(5'- TATACTCGAGCGAGCCCTCAGCCCAGGCA -3'). In a second PCR reaction, the IgSP sequence was amplified from the pNUT-IgSP-CNTF vector (ref. US 6,361,741) using the primers IgSPKozak1s+BamHl (5'-TATAGGATCCGCCACCATGAAATGCAGCTGGGTTATC-3') and IgSPas-NBN.Flap (5'- GGCCCCCAGCCGAATTCACCCCTGTAGAAAG-3'). In the third step the products of step 1 and 2 were combined in a final PCR reaction that generates IgSP-NBN by using equal amounts of the two products as template with the primers IgSPKo-zak1s+BamHI and NBN-as+Xhol.

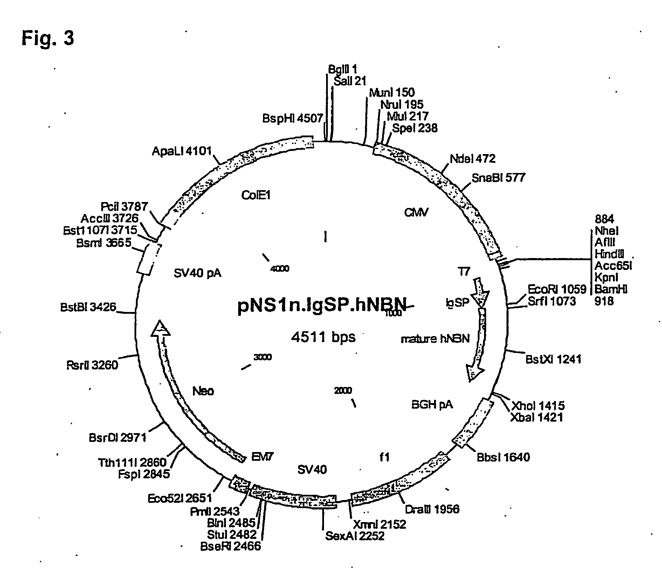

To generate a plasmid-based expression vector the resulting fragment was cloned in pNS1n digested with BamHl/Xhol. In this vector, the IgSP-NBN sequence is placed under transcriptional control of the CMV promoter (see Figure 3). Furthermore, the vector contains the Neo gene that confers G418 resistance when expressed in mammalian cells.

### Transient transfection studies

ARPE-19 is a human retinal pigment epithelial cell line (Dunn et al. 1996) grown in DMEM/Nutrient Mix F-12 with Glutamax (Invitrogen, Denmark) supplemented with 10% fetal bovine serum (Sigma-Aldrich, Denmark) at 37°C and 5% CO₂. Cells were passaged approximately twice a week by trypsinization and reseeding (1:5 split ratio). Cells were seeded in 6-well plates (Corning Costar, Biotech Line, Denmark) at a density of 10⁵ cells/well for transfection studies. The next day, cells were transfected with 3 µg plasmid/well in duplicate wells using Fugene6 (Roche, Germany) according to the manufacturer's specifications. NBN activity present in cell supernatants collected 3 days after transfection was assayed in a RetL3 ELISA.

### RetL3 ELISA

The RetL3 USA detects binding of a Ret-AP conjugate to a complex of NBN bound to the NBN-specific GFRα3 receptor. This assay will only detect NBN molecules which are functionally active. Briefly, a 96-well plate (B&W Isoplate HB, Perkin Elmer, Denmark) was coated with 100µl 1µg/ml Goat anti human Fc (Jackson Immunoresearch Laboratories, TriChem, Denmark) in 50mM NaHCO₃ (pH=9.6) for 16 h at 4°C. After wash in PBS/0.05 % Tween20 (PBST), wells were blocked in 0.2% I-Block (Tropix, Applied Biosystems, Denmark) in PBST for 1hr at room temperature, followed by a brief wash in PBST. Cell supernatants or recombinant mouse Artemin (R&D systems, UK) were diluted in DMEM/10% FCS and subsequently incubated in the wells with 1µg/ml GFRα3/Fc fusion protein (R&D Systems, UK) in RET-AP conditioned media (Biogen, USA) for 1.5 h at room temperature. Wells were then washed first in PBST and then in AP-buffer (200 mM Tris (pH=9.8), 10 mM MgCl₂) followed by 30 min incubation with 10% Sapphire Enhancer (Tropix, Applied Biosystems, Denmark) and 2% CSPD (Tropix, Applied Biosystems, Denmark) in AP-buffer. Luminescence was determined by using Microbeta Trilux Counter (Perkin Elmer, Denmark).

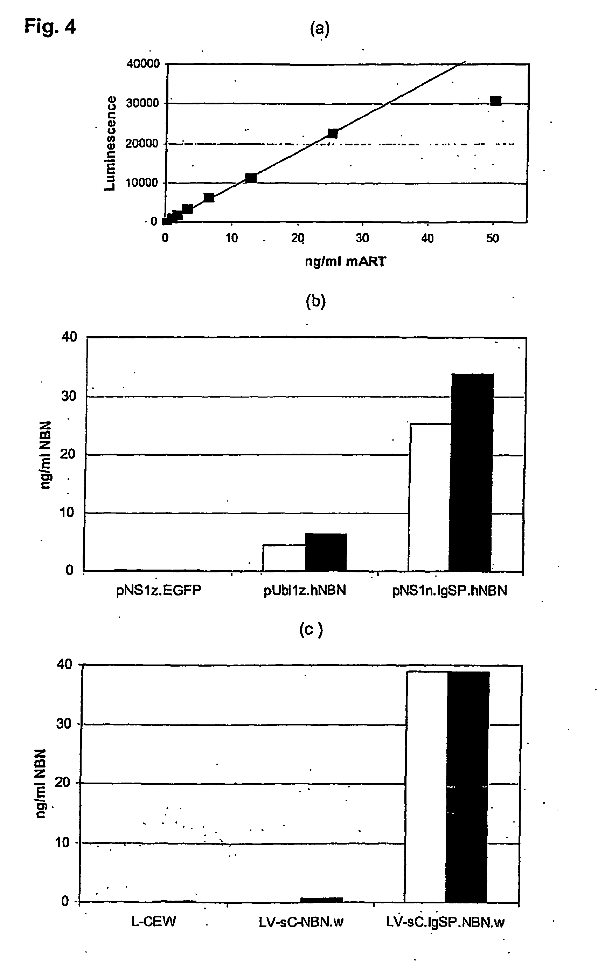

### Results (Figure 4, panel (b))

NBN activities of 25.3-33.8 ng/ml were detected by using the RetL3 ELISA in supernatants collected from ARPE-19 cells transiently transfected with the pNS1n-IgSP.NBN construct. In contrast, approximately 5-fold lower NBN activity (4.4-6.4 ng/ml) is detected in ARPE-19 cells transiently transfected with a wild-type (pre-pro)NBN expression construct (pUbi1z.hNBN) included in the same experiment. Very low or undetectable NBN activity was detected in cell supernatants of ARPE-19 transiently transfected with an EGFP expression construct (pNS1z-EGFP) confirming the specificity of the assay.

These results indicate that the use of a chimeric IgSP-NBN construct leads to higher release of mature NBN from mammalian cells capable of binding and activating the specific NBN receptor complex as compared to the release of NBN using a wildtype (prepro)NBN construct.

### EXAMPLE 2: In vitro transduction with IgSP-NBN construct

### Generation of a lentiviral IgSP-NBN construct and virus stocks

To generate a lentiviral construct, pNS1n-IgSP-NBN was digested with BamHI-Xhol and the IgSP-hNBN PCR fragment (as described in example 1) was ligated into BamHI/XhoI-digested pHsCXW resulting in pHsCXW.IgSP.NBNw (Figure 3). pHsCXW is a derivative of a self-inactivating lentiviral transfer construct" pHR'-SIN-₁₈ including a WPRE element (Dull et al., J.Virol., 72(11):8463-71(1998); Zufferey et al., J.Virol., 72(12):9873-80(1998): Zufferey et al. J.virol., 73 (4):2886-92 (1999)) gener ated by replacing the large non-viral part of the transfer construct with the pUC19 backbone. The sequence of pHsCXW can be accessed through GenGank ID: AY468486.

Replication-defective LV-sC.IgSP.NBN.W virus particles are generated by co-transfection of pHsC.IgSP.NBN.W with pMD.G (VSV-G pseudo-typing vector) and pBR8.91 (packaging vector) (Zufferey et al., Nat. Biotech., 15:871-75(1997)) into 293T cells providing the required viral proteins in *trans.* Briefly, 293T cells cultured in DMEM with 4.5 g/l glucose and glutamax (Life Technologies, 32430-027) supplemented with 10 % FCS (Life Technologies, 10099-141) are seeded in T75 flasks (2x106 cells /flask) the day before transfection. For each T75 flask cells are transfected with 5 µg pMD.G, 15 µg pBR8.91 and 20 µg of transfer vector using Lipofectamine+ following the manufacturer's instructions. Virus containing cell supernatant is collected 2-3 days after the transfection, filter-sterilized through a 0.45 m cellulose acetate or polysulphonic filter and concentrated by ultracentrifugation at 50,000xg for 90 min. at 4°C. After a second round of ultracentrifugation, the concentrated virus pellet is resuspended in DMEM, aliquoted and stored at -80°C. To determine virus titer, reverse transcriptase (RT) activity is assayed (Cepko and Pear, Current Protocols in Molecular Biology, 9.13.5-6, supplement 36) and transducing untis (TU)/ml calculated from the determined RT activity using an EGFP lentivirus with known transducing activity as reference.

### Transduction studies

ARPE-19 cells were transduced with NBN expression vectors. Briefly, cells were seeded in 6-well plates (Corning Costar, Biotech Line, Denmark) at a density of 10⁵ cells/well. The next day, 2x10⁵ TU of virus was added pr. well (duplicates) together with 5 µg/ml polybrene for 4 h. The medium was changed and cultures were incubated for 3 days. Then cell supernatants were collected for RetL3 ELISA as described in Example 2.

### Results (Figure 4, panel (c)).

NBN activities of 39 ng/ml were detected by using the RetL3 ELISA in supernatants collected from ARPE-19 cells transduced with the LV-sC.IgSP.NBN.W virus. In contrast, very low or undetectable NBN activity was detected in ARPE-19 cells transduced with a lenti-virus containing the wild-type (prepro)NBN cDNA (LV-sC-NBN.W) or a control EGFP lenti-virus (LV-sCEW).

These results indicate that, in contrast to a viral construct containing the wild type (prepro)NBN cDNA, the use of a chimeric IgSP-NBN viral construct allows high release of mature NBN from mammalian cells capable of binding and activating the specific NBN receptor complex.

### EXAMPLE 3: Analysis of NBN protein expressed from IgSP-NBN constructs

### Western blot analysis of cell supernatants

NBN present in cell supernatants from transfected or transduced cell cultures was concentrated by affinity binding to GFRα3-Ig prior to Western blot analysis. Briefly, 4 wells of a Nunc MaxiSorp plate were coated with 300µl Goat anti human Fc 1 µg/ml (Jackson Immunoresearch Laboratories, TriChem, Denmark) in 50mM NaHCO₃ (pH=9.6) for 16 h at 4°C. The wells were blocked with 400 µl 1 % BSA in PBS for 1 h at room temperature, followed by 3 washes in PBST. 300 µl/well GFRα3/Fc fusion protein 1 µg/ml (R&D Systems, UK) was then added and the plate was incubated for 1 h at room temperature to maximise binding. The wells were then emptied and washed again 3 times with PBST. 300 µl supernatant collected from transfected or transduced cell cultures was then added to each of the 4 wells and incubated for at least 3 h with gently shaking at room temperature. The wells were then washed twice in PBST. 20 µl of non-reducing sample buffer (2% SDS, 0.4 M Tris (ph=8.0) was added to the first well and the plate shaken rapidly for 5 min. to elute the bound proteins. The content was transferred to the next well and the procedure was repeated to elute the proteins bound in the remaining wells. After addition of DTT to 10 mM, the samples were heated to 96°C for 5 minutes and analysed by SDS PAGE on a 15 % polyacrylamide gel using the MultiPhor II system according to the manufacturer's recommendations (Amersham Pharmacia, Denmark). The proteins were the blotted to PVDF membranes (BioRad, Denmark) that was immunostained using a rabbit polyclonal anti-NBN antibody (#378) as detecting antibody. Membranes were developed using the ECL+ system (Amersham Pharmacia, Denmark) and subjected to film exposure.

### Results

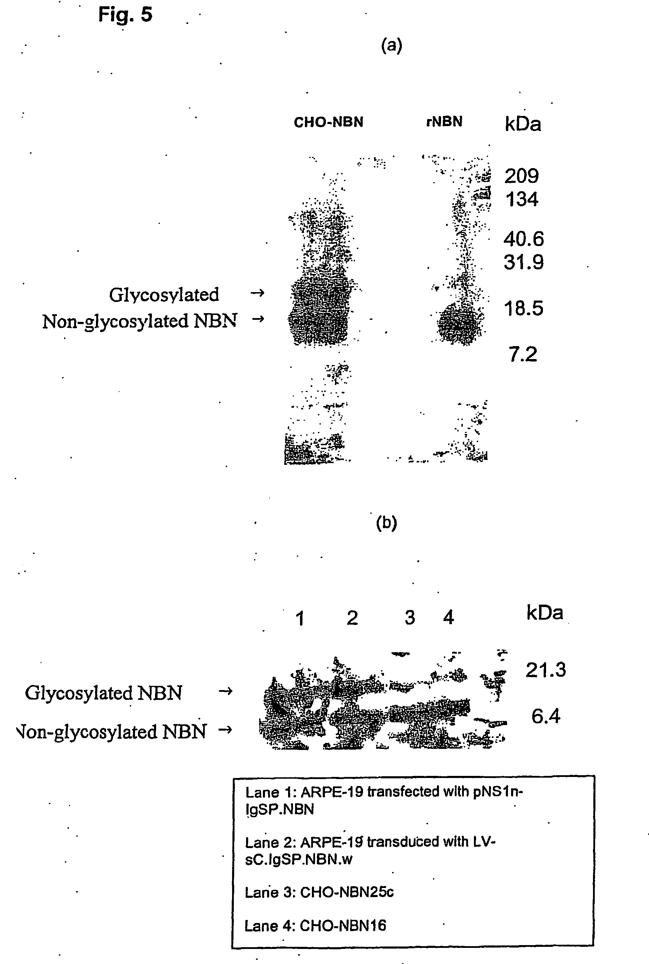

The #378 is a rabbit polyclonal antibody raised against an NBN-derived peptide (ALRPPPGSRPVSQPC). As seen in Figure 5 panel (a) a single band of a molecular weight between 7.2 and 18.5 kDa is recognized in reduced (+DTT) samples containing purified rat recombinant NBN produced in E. coli corresponding to the monomeric unglycosylated NBN113. A band of the same MW in addition to several additional bands are recognized in reduced samples from stable CHO-NBN clones established by transfection with wt(prepro)NBN. The identities of a number of these bands have been determined in previous studies using deglycosylation and N-terminal sequencing. A band with slightly lower molecular weight represents a smaller and unglycosylated version of mature NBN (NBN104). In addition a very broad band with an apparent MW of approximately 21 kDa represent glycosylated versions of NBN113 and NBN104. Bands of higher MW represent (glycosylated) pro-NBN seen occasionally in GFRα3-affinity purified samples.

As seen in Figure 5 panel b, the double band of MW between 6.4 and 21.3 kDa corresponding to unglycosylated NBN113 and NBN104 is detected in two stable clones of CHO-NBN cells. The same double band is also detected in ARPE-19 transduced or transfected with IgSP-NBN expression constructs. Furthermore, a double band of MW close to 21 kDa corresponding to glycosylated NBN113 and NBN104 is seen in all samples analysed. These results indicate that processing and posttranslational modification are similar when expressed from wt(prepro)NBN and IgSP-NBN constructs.

### EXAMPLE 4: Sequences of chimeric IgSP-NBN viral construct and prediction of signal peptide.

IgSP-NBN - Nucleotide: sequence present in construct

IgSP-NBN is a fusion construct with the signal peptide from an immunoglobulin gene fused directly to mature NBN (113).

The IgSP-NBN contains an intron
Intron-exon prediction by NetGene (CBS-DTU server):

| Length: 478 nucleotides. | | | | | | |
|---|---|---|---|---|---|---|
| 13.4% A, 36.2% C, 32.2% G, 18.2% T, 0.0% X, 68.4% G+C | | | | | | |
| Donor splice sites, direct strand | | | | | | |
| | pos 5'->3' | phase | strand | confidence | 5' | exon intron |
| 3' | | | | | | |
| | 47 | 2 | + | 0.00 | | GTGGTTACAGAGTAAGGGSCT |
| | 248 | 0 | + | 0.60 | | CGACGAGCTG^GTCGTTTCC |

| Donor splice sites, complement strand | | | | | | |
|---|---|---|---|---|---|---|
| No donor site predictions above threshold. | | | | | | |
| Acceptor splice sites, direct strand | | | | | | |
| | pos 5'->3' | phase | strand | confidence | 5' | intron exon |
| 3' | | | | | | |
| | 125 | 1 | + | 0.83 | | CTTTCTACAGAC^GGTGL4ATTC |
| | 153 | 2 | + | 0.18 | | GCCCGGGCAG^CCGCGCTCGG |
| | 167 | 1 | + | 0.20 | | GCTCGGGCAG^CGGGGGCGCG |
| | 199 | 0 | + | 0.42 | | GCGCTCGCAG^CTGGTGCCGG |

### Nucleotide sequence of spliced transcript:

### Translation of spliced transcript:

The translated fusion protein is predicted to contain a 19 amino acid signal peptide, which is cleaved from the mature NBN (113) sequence using Signal P (available at the CBS DTU server at www.cbs.dtu.dk). (identification of prokaryotic and eukaryotic peptides and prediction of their cleavage sites. H. Nielsen, J. Engelbrecht, S. Brunak, G. von Hejne, Protein Engineering 10, 1-6, 1997.)

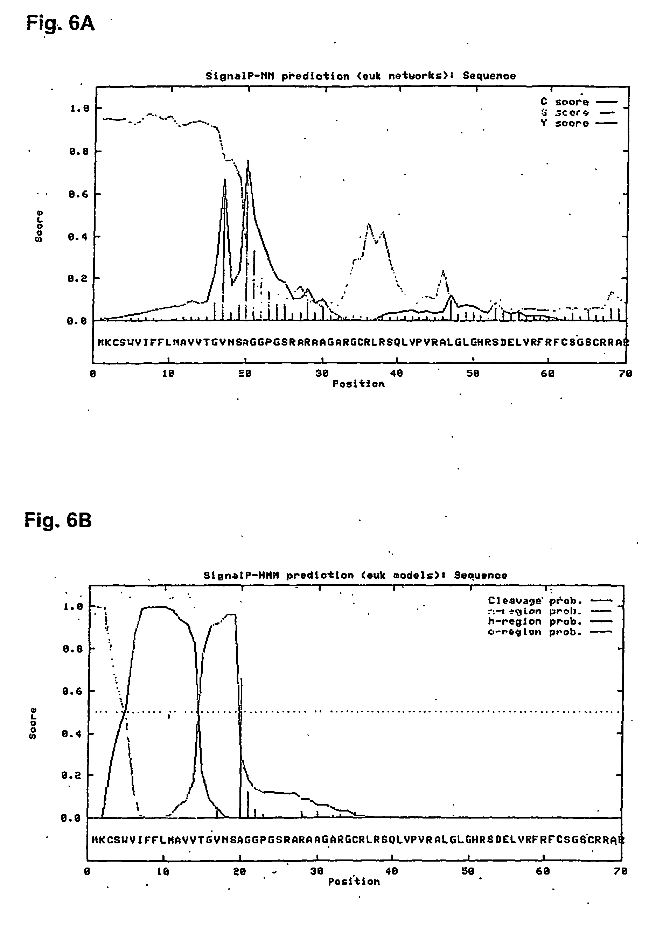

### SignaIP-NN result (see Fig 6a):

| >Sequence | length = 70 | | | |
|---|---|---|---|---|
| # Measure | Position | Value | Cutoff | signal peptide? |
| max. C | 20 | 0.757 | 0.33 | YES |
| max. Y | 20 | 0.758 | 0.32 | YES |
| max. S | 7 | 0.970 | 0.82 | YES |
| mean S | 1-19 | 0.906 | 0.47 | YES |

| | | | | |
|---|---|---|---|---|
| # Most likely cleavage site between pos. 19 and 20: VNS-AG | | | | |

### SignalP-HMM result (See Figure 6b):

Prediction: Signal peptide
Signal peptide probability: 0.999
Signal anchor probability: 0.000
Max cleavage site probability: 0.660 between pos. 19 and 20
Using neural networks (NN) and hidden Markov models (HMM) trained on eukaryotes

### EXAMPLE 5: Prediction of positions for signal peptides.

The prediction of cleavage site when Igsp is fused to a neublastin polypeptide of various length is shown below using the signal P programme 2.0 identified above.

**Cleavage sites:**

| Protein | SignaIP-NN | SignaIP-HMM | Remarks |
|---|---|---|---|
| Pre-pro-NBN | 39/40 *(0,852) | 39/40 (0,348) | Very long signal peptide with low probability in N-terminus |
| IgSP-NBN113 | 19/20 (0,757) | 19/20 (0,660) | |
| IgSP-NBN106 | 19/20 (0,831) | 22/23 (0,586) | 19/20 cleavage predicted with less probability in HMM (0,2) |
| IgSP-NBN104 | 19/20 (0,643) | 22/23 (0,440) | 19/20 cleavage predicted with less probability in HMM (0,18). Furthermore, an additional cleavage site 25/26 is predicted with same probablity |
| IgSP-NBN102 | 16/17 (0,643) | 19/20 (0,359) | 19/20 cleavage predicted with a probability of 0,5 by NN |
| IgSP-NBN99 | 19/20 (0,718) | 19/20 (0,496) | |

### IgSP:

(19)MKCSWVIFFLMAVVTGVNS

### Mature forms of NBN:

### EXAMPLE 6: Cloning of deltapro-NBN into pHsCXW:

Deltapro-NBN (SEQ. ID. NO: 82) was generated by overlap PCR in three amplification steps: 1) The relatively long 117 bp leader sequence (i.e. 39 a.a. signal peptide) of preproNBN with 5' BamHI/Kozak overhang and 10 base 3' overlap to mature NBN (143 bp); 2) mature NBN with 5' 10 base NBN leader sequence overlap and 3' XhoI (362 bp); 3) the products of steps 1 and 2 were combined in a final PCR reaction that generated Δpro-NBN (492 bp).

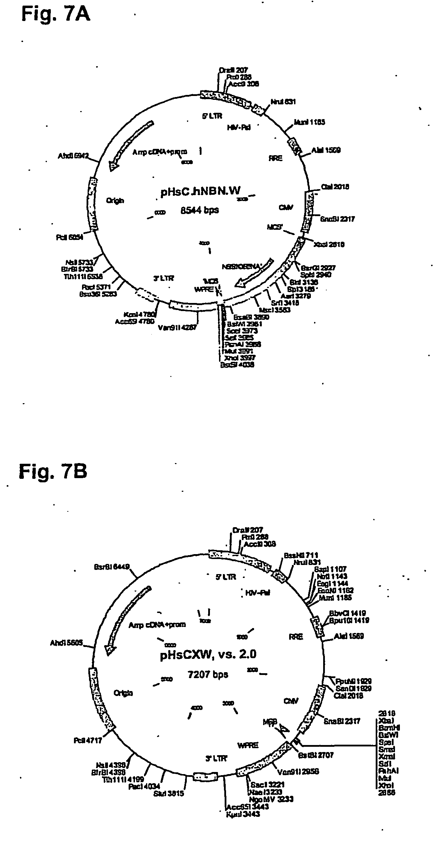

The first PCR reaction (NBN leader):
Primers used:
   BamHI+Kozak+hNBNsp, 5'-TATAGGATCCGCCACCATGGAACTTGGACTTG-GAGG-3'
   hNBNsp3'-matNBN FLAP as, 5'- GGCCCCCAGCGGCCTCTGCGACGCTGCTCA-3'
   Plasmid pHsC.hNBN.W (see plasmid map in Fig. 7a) was used as template for the PCR reaction, which was run using Pfu-turbo polymerase.

### PCR conditions:

| | | |
|---|---|---|
| 94 °C | 3 min | |
| 94 °C | 30 s | |
| 55 °C | 30 s | 35 cycles |
| 72 °C | 1 min | |
| 72 °C | 5 min | |

The second PCR reaction (matureNBN):
Primers used:
   hNBNsp3' FLAP-matNBN s, 5'-CGCAGAGGCCGCTGGGGGCCCGGGCAGC-3'
   NBNas+Xhol, 5'-TATACTCGAGCGAGCCCTCAGCCCAGGCA-3'
      Plasmid pHsC.hNBN.W (see plasmid map Fig. 7a) was used as template for the PCR reaction, which was run using Pfu-turbo polymerase.

### PCR conditions:

| | | |
|---|---|---|
| 94 °C | 3 min | |
| 94 °C | 30 s | |
| 65 °C | 30 s | 35 cycles |
| 72 °C | 1 min | |
| 72 °C | 5 min | |

The third PCR reaction (deltapro-NBN):
Primers used:
   BamHI+Kozak+hNBNsp,
   5'-TATAGGATCCGCCACCATGGAACTTGGACTTGGAGG-3'
   NBNas+XhoI, 5'-TATACTCGAGCGAGCCCTCAGCCCAGGCA-3'

The PCR fragments from the two first PCR reactions (NBN leader and mature NBN both at a 1:10 dilution were used as template for the third PCR reaction, which was run using Pfu-turbo polymerase, and the same PCR profile as the first PCR run.

The PCR fragment from the third PCR reaction was cut with BamHI and Xhol and cloned between BamHI and Xhol sites in pHsCXW (see plasmid map in Fig. 7b).

### EXAMPLE 7: In vitro transfection with IgSP-NBN constructs and deltaproNBN constructs in different cell lines

secretion of NBN after transient transfection with different NBN constructs, including wt pre-pro NBN, delta-pro NBN and IgSP-NBN, was compared. Transient transfections were performed in ARPE-19, HEK293, CHO and HiB5 cells.

### Cell lines

ARPE-19 cells were cultured as described in example 1. HiB5 (Renfranz et al. 1991), HEK293 and CHO cells were grown in DMEM (Invitrogen, Denmark) with 10% fetal bovine serum (Invitrogen, Denmark), and medium for CHO cells were further supplemented with 20 mg/l L-proline. ARPE-19, HEK293 and CHO cells were grown at 37°C and HiB5 cells at 33°C in 5% CO₂. Cells were passaged approximately twice a week by trypsinization and reseeding (1:5 split ratio).

### NBN secretion after transient transfection

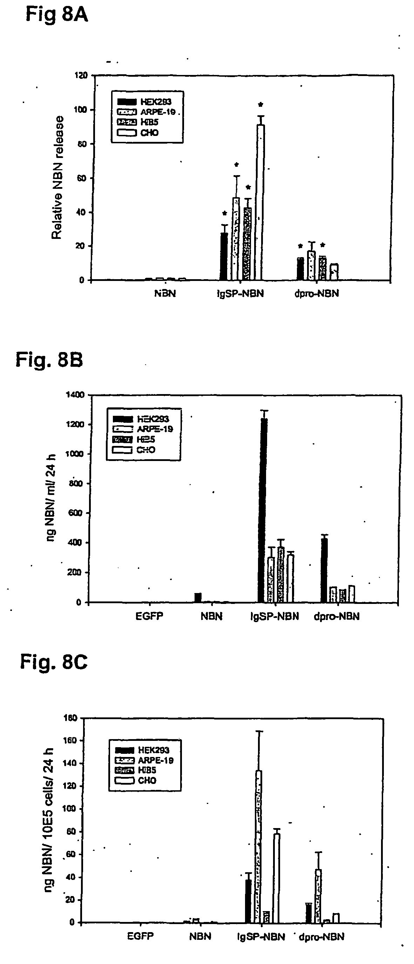

Cells were seeded in 6-well plates (Corning Costar, Biotech Line, Denmark) at a density of approximately 10⁵ cells/well. The next day, cells were transfected with pHsC.hNBN.W, pHsC.IgSP.hNBN.W and pHsC.deltapro-hNBN.W, respectively. ARPE-19 cells were transfected in triplicate wells using Fugene6 as described in example 1, whereas the other three cell lines were transfected using 2 µg plasmid/well in triplicate wells using Lipofectamine Plus (Invitrogen, Denmark) according to the manufacturer's instructions. The next day, fresh growth medium was added to the wells, and cells were incubated for further 24 hours before collecting conditioned medium. Sufficient transfection efficiency was ensured by evaluation of EGFP expression in wells transfected in parallel with pHsC.EGFP.W. NBN binding activity in conditioned medium was measured using the RetL3 ELISA, as described in example1. The RetL3 ELISA detects binding of a Ret-AP conjugate to a complex of NBN bound to the NBN-specific GFRα3 receptor. Values were calculated as ng NBN/ml/24 h and ng NBN/10⁵ cells/24 h and adjusted to relative NBN release with values from cells transfected with the wt NBN construct set to 1. Data in figure 8 represent these three calculations and are expressed as mean ± SEM (n=3). In panel A, * indicates a significant difference from cells transfected with the wt NBN construct (P<0.05, one way ANOVA , Fisher LSD Method)

### Results

As shown in the table below and figure 8A, the four tested cell lines showed increased NBN release when using the deltapro-NBN construct, compared to the wt NBN construct (9-17 fold higher NBN release, depending on cell line). When transfecting the four cell lines with the pHsC.IgSP.hNBN construct, NBN secretion was further enhanced (28-91 fold higher NBN release compared to wt NBN). Very low or undetectable NBN activity was seen in cell supernatants from ARPE-19 transiently transfected with the EGFP expression construct (pHs.C.EGFP.W) confirming the specificity of the assay.

| | **HEK293** | | **ARPE-19** | | **HiB5** | | **CHO** | |
|---|---|---|---|---|---|---|---|---|
| **Construct** | **Mean** | **S.E.M.** | **Mean** | **S.E.M.** | **Mean** | **S.E.M.** | **Mean** | **S.E.M.** |
| NBN | 1,0 | 0,2 | 1,0 | 0,3 | 1,0 | 0,2 | 1,0 | 0,1 |
| IgSP-NBN | 28,0 | 4,7 | 48,6 | 12,9 | 42,7 | 5,4 | 91,2 | 15,1 |
| idpro-NBN | 12,4 | 0,6 | 17,2 | 5,5 | 13,1 | 1,0 | 9,2, | 0,5 |

Panel B in Fig. 8B shows NBN concentrations in conditioned medium (24 h) from the transiently transfected cell lines. The highest concentration (1240±54 ng NBN/ml) was found in conditioned medium from HEK293 cells transfected with the IgSP-NBN construct Panel C in Fig 8C shows NBN release per 10⁵ cells per 24 h. IgSP-NBN transfected ARPE-19 cells showed the highest NBN release (133±35 ng/10⁵ cells/24 h).

The present results indicate that the use of a chimeric IgSP-NBN construct and of deltapro NBN construct in order to increase secretion of mature NBN from mammalian cells is applicable in different cell types.

### EXAMPLE 8: Prediction using SignalP 3.0

Prediction of positions for signal peptides using version 3.0. The predictions were carried out on deltapro NBNs and IgSP-NBNs of the following sequences.

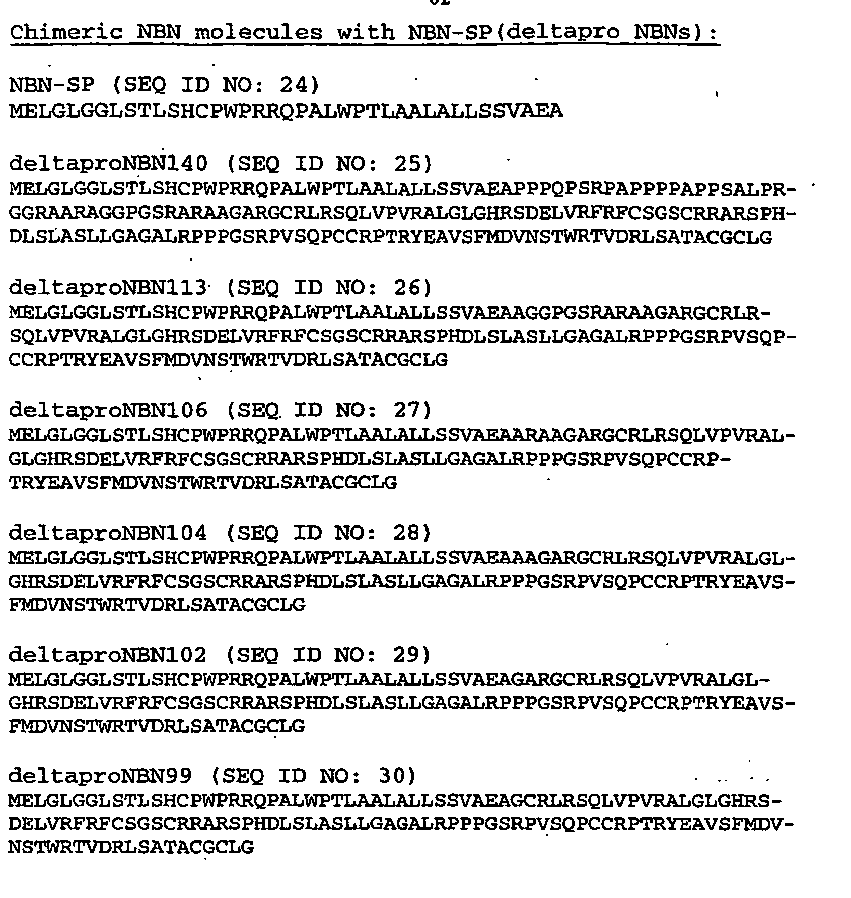

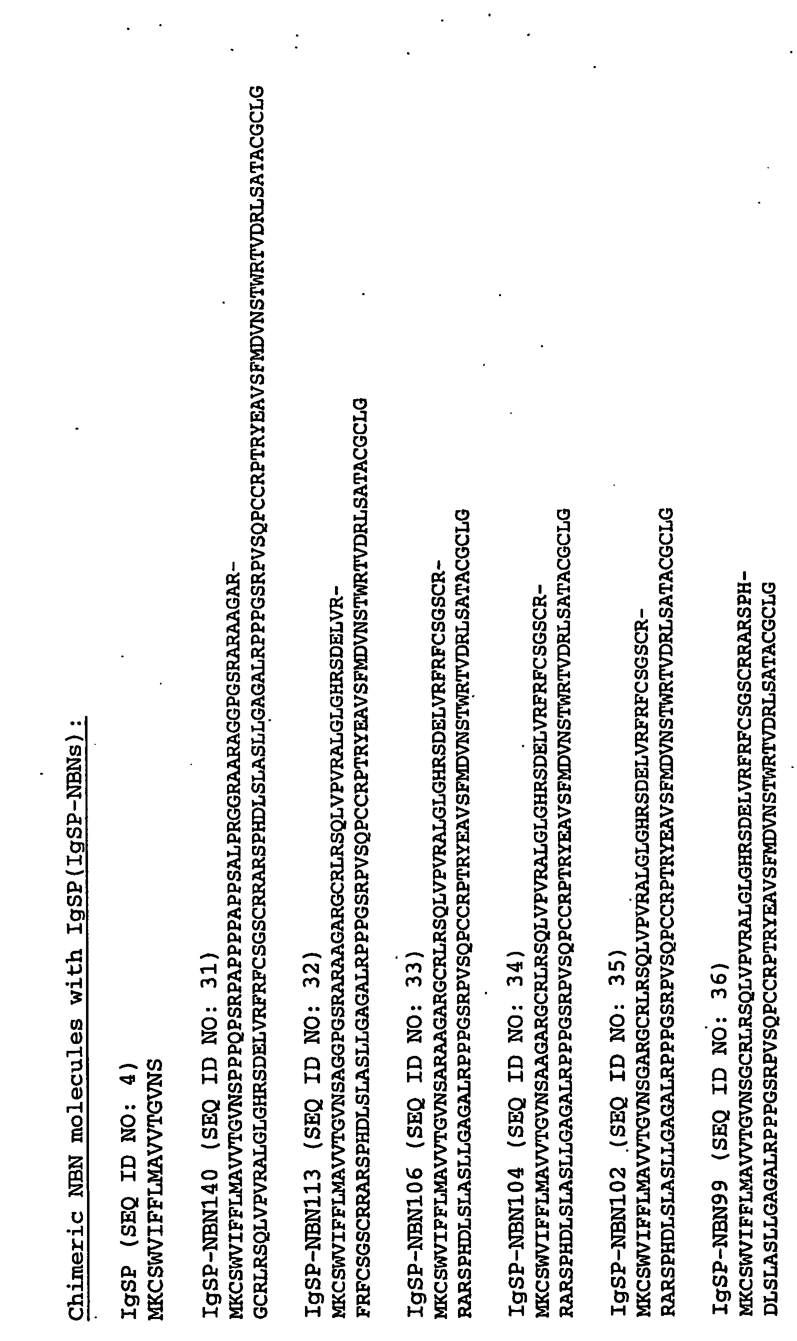

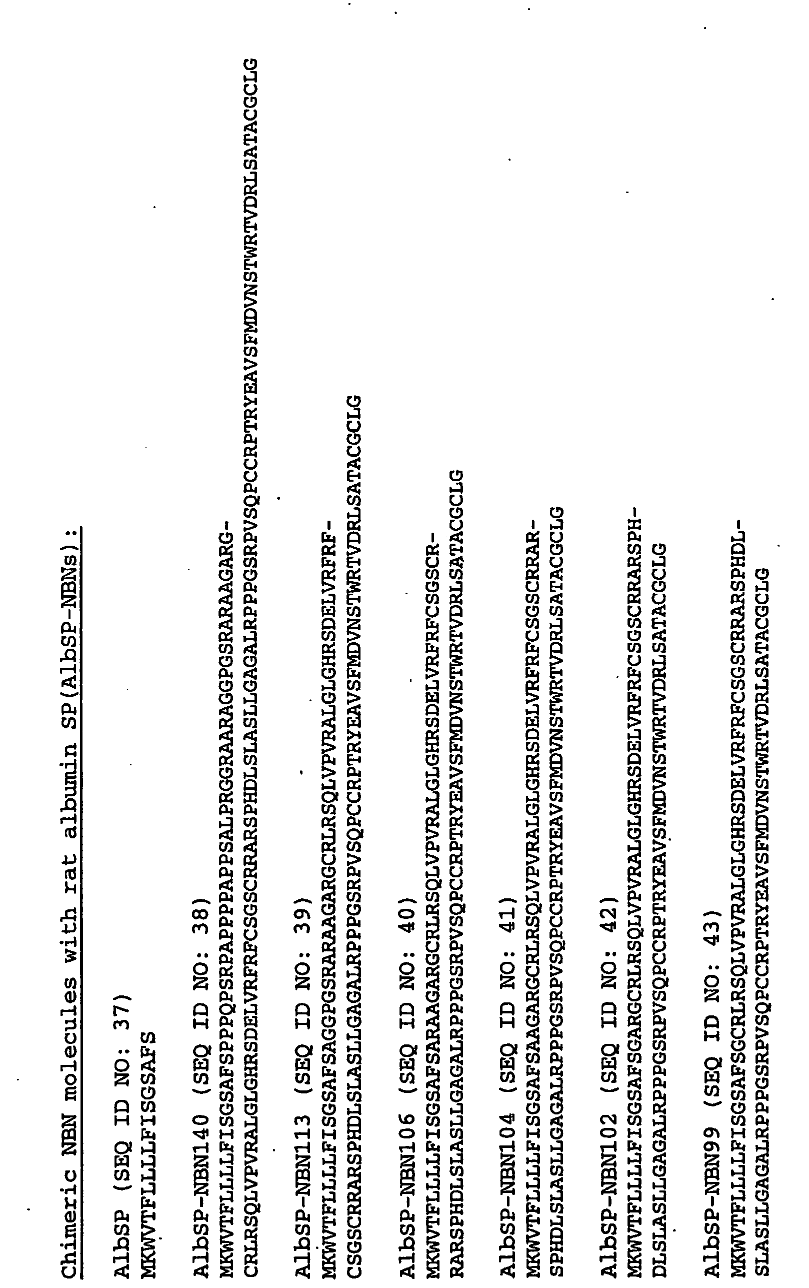

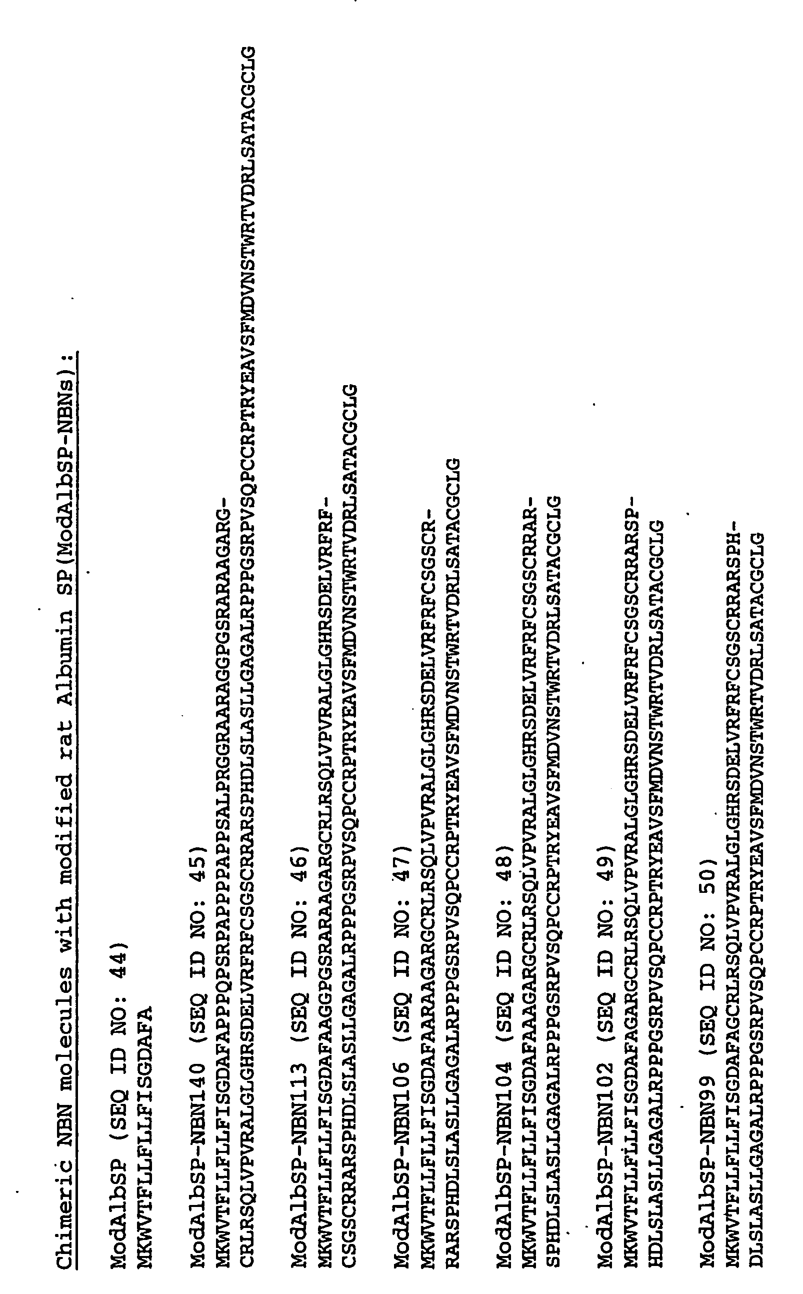

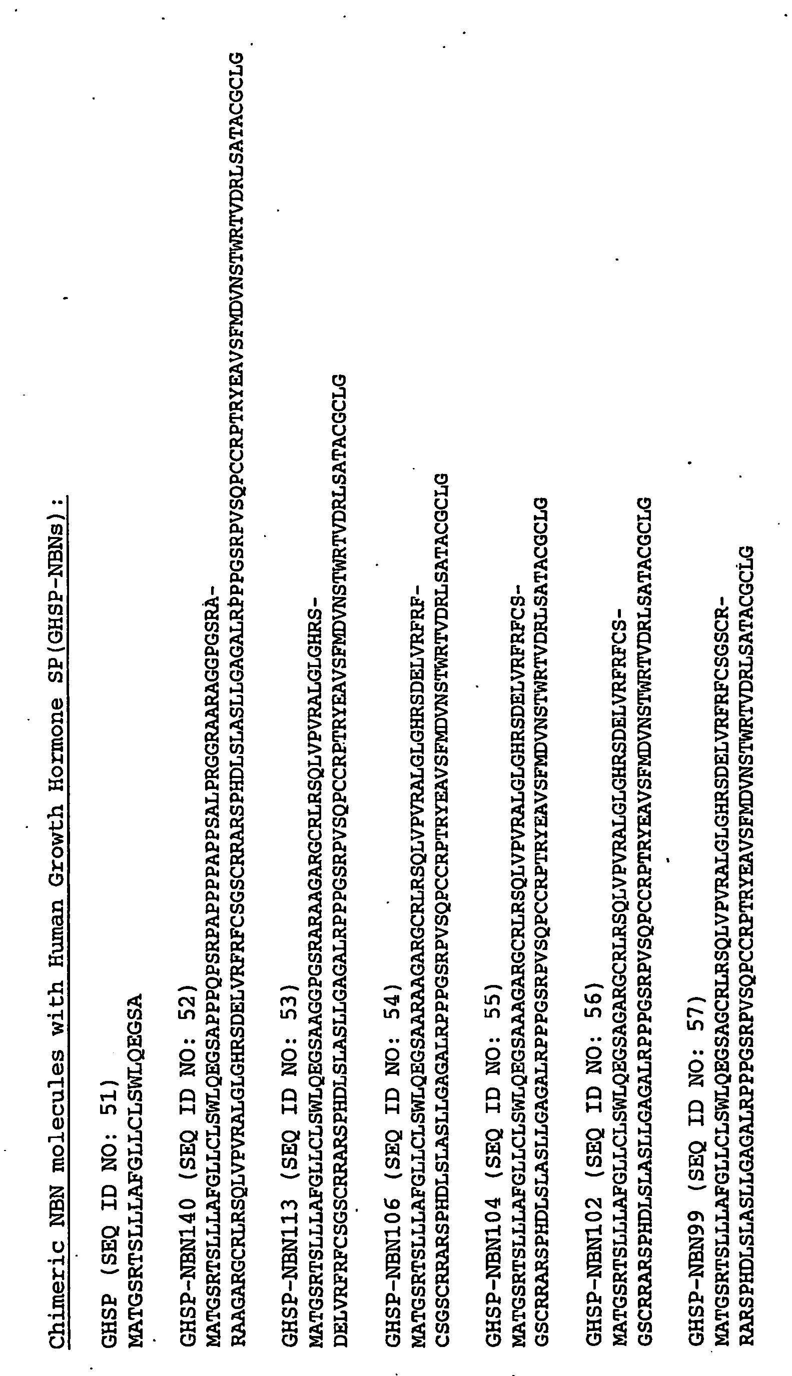

The signal peptide predictions are shown below in the tables:

**Signal peptide predictions for proteins with NBN signal peptide¹ (deltapro)**

| **Protein** | **Signal P 3.0 - NN** | | | | **SignalP - HMM** | | | **Remarks** |
|---|---|---|---|---|---|---|---|---|
| | **Mean S** | **D** | **Max. C** | **Cleavage site** | **SP probability** | **Cleavage site probability** | **Cleavage site** | |
| Prepro-NBN | 0.5 | 0.551 | 0.451 | 39/40 | 0.993 | 0.573 | 39/40 | |
| del-taproNBN140 | 0.437 | 0.569 | 0.711 | 39/40 | 0.993 | 0.845 | 39/40 | |
| del-taproNBN113 | 0.492 | 0.616 | 0.693 | 39/40 | 0.998 | 0.675 | 39/40 | |
| del-taproNBN106 | 0.505 | 0.564 | 0.495 | 39/40 | 0.998 | 0.415 | 39/40 | |
| del-taproNBN104 | 0.509 | 0.500 | 0.305* | (39/40) | 0.998 | 0.278* | (39/40) | |
| del-taproNBN102 | 0.492 | 0.508 | 0.352 | 39/40 | 0.996 | 0.514 | 39/40 | |
| Del-taproNBN99 | 0.497 | 0.555 | 0.478 | 39/40 | 0.991 | 0.724 | 39/40 | |
| Cuttoff values 0.48 0.43 0.32 0.5 | | | | | | | | |

| | | | | | | | | |
|---|---|---|---|---|---|---|---|---|
| ¹MELGLGGLSTLSHCPWPRRQPALWPTLAALALLSSVAEA- 39 amino acids *Below cuttoff values | | | | | | | | |

**Signal peptide predictions for proteins with IgSP²**

| **Protein** | **Signal P 3.0 - NN** | | | | **SignalP - HMM** | | | **Remarks** |
|---|---|---|---|---|---|---|---|---|
| | **Mean S** | **D** | **Max C** | **Cleavage site** | **SP probability** | **Cleavage site probability** | **Cleavage site** | |
| IgSP-NBN140 | 0.853 | 0.844 | 0.906 | 19/20 | 0.999 | 0.823 | 19/20 | |
| IgSP-NBN113 | 0.927 | 0.893 | 0.901 | 19/20 | 1.000 | 0.877 | 19/20 | |
| IgSP-NBN106 | 0.935 | 0.847 | 0.803 | 19/20 | 1.000 | 0.557 | 22/23 | Cleavage site at 19/20 predicted with lower probability than 22/23 by HMM |
| IgSP-NBN104 | 0.941 | 0.819 | 0.654 | 19/20 | 1.000 | 0.446* | (22/23) | Cleavage site at 19/20 predicted with lower probability than 22/23 by HMM |
| IgSP-NBN102 | 0.915 | 0.814 | 0.692 | 19/20 | 0.999 | 0.585 | 19/20 | |
| IgSP-NBN99 | 0.906 | 0.833 | 0.798 | 19/20 | 0.998 | 0.722 | 19/20 | |
| Cuttoff values | 0.48 | 0.43 | 0.32 | | | 0.5 | | |

| | | | | | | | | |
|---|---|---|---|---|---|---|---|---|
| ²MKCSWVIFFLMAVVTGVNS - 19 amino acids *Below cuttoff values | | | | | | | | |

**Signal peptide predictions for proteins with ModAlbSP⁴**

| **Protein** | **Signal P 3.0 - NN** | | | | **SignalP - HMM** | | | **Remarks** |
|---|---|---|---|---|---|---|---|---|
| | **Mean S** | **D** | **Max C** | **Cleavage site** | SP **probability** | **Cleavage site probability** | **Cleavage site** | |
| ModAlbSP-NBN140 | 0.930 | 0.918 | 0.961 | 19/20 | 1.000 | 0.708 | 19/20 | |
| ModAlbSP-NBN113 | 0.955 | 0.929 | 0.971 | 19/20 | 1.000 | 0.747 | 19/20 | |
| ModAlbSP-NBN106 | 0.959 | 0.901 | 0.939 | 19/20 | 1.000 | 0.474* | (19/20) | |
| ModAlbSP-NBN104 | 0.960 | 0.877 | 0.822 | 19/20 | 1.000 | 0.308* | (23/24) | Cleavage site at 19/20 predicted at almost same probability as 23 /24 by HMM |
| ModAlbSP-NBN102 | 0.947 | 0.871 | 0.818 | 19/20 | 1.000 | 0.373* | (23/24) | Cleavage site at 19/20 predicted at almost same probability as 23/24 by HMM |
| ModAlbSP-NBN99 | 0.931 | 0.878 | 0.911 | 19/20 | 1.000 | 0.634 | 19/20 | |
| Cuttoff values | 0.48 | 0.43 | 0.32 | | | 0.5 | | |

| | | | | | | | | |
|---|---|---|---|---|---|---|---|---|
| ⁴MKWWFLLFLLFISGDAFA - 19 amino acids *Below cuttoff values | | | | | | | | |

**Signal peptide predictions for proteins with AlbSP³**

| **Protein** | **Signal P 3.0 - NN** | | | | **SignalP - HMM** | | | **Remarks** |
|---|---|---|---|---|---|---|---|---|
| | **Mean S** | **D** | **Max C** | **Cleavage site** | **SP probability** | **Cleavage site probability** | **Cleavage site** | |
| AlbSP- NBN140 | 0.947 | 0.870 | 0.830 | 16/17 | 1.000 | 0.485* | (16/17) | Cleavage site between 18/19 predicted by almost same probability as 16/17 by NN and HMM |
| AlbSP-NBN113 | 0.954 | 0.847 | 0.681 | 18/19 | 1.000 | 0.500 | 18/19 | |
| AlbSP-NBN106 | 0.960 | 0.786 | 0.575 | 18/19 | 1.000 | 0.283* | (21/22) | Cleavage site between 18/19 predicted by almost same probability as 21/22 by HMM |
| AlbSP-NBN104 | 0.872 | 0.749 | 0.585 | 22/23 | 1.000 | 0.423* | (22/23) | Cleavage sites at 18/19 predicted with lower probability than 22/23 by NN |
| AlbSP-NBN102 | 0.831 | 0.777 | 0.896 | 22/23 | 1.000 | 0.684 | 22/23 | Cleavage sites at 18/19 predicted with lower probability than 22/23 by NN |
| AlbSP-NBN99 | 0.965 | 0.810 | 0.612 | 16/17 | 1.000 | 0.334* | (16/17) | Cleavage site at 18/19 predicted at almost same probability as 16/17 by NN and HMM |
| Cuttoff values | 0.48 | 0.43 | 0.32 | | | 0.5 | | |

| | | | | | | | | |
|---|---|---|---|---|---|---|---|---|
| ³MKWVTFLLLLFISGSAFS - 18 amino acids - *Below cuttoff values | | | | | | | | |

**Signal peptide predictions for proteins with GHSP⁵**

| **Protein** | **Signal P 3.0 - NN** | | | | **SignalP - HMM** | | | **Remarks** |
|---|---|---|---|---|---|---|---|---|
| | **Mean S** | **D** | **Max C** | **Cleavage site** | **SP probability** | **Cleavage site probability** | **Cleavage site** | |
| GHSP-NBN140 | 0.874 | 0.815 | 0.702 | 26/27 | 0.999 | 0.784 | 26/27 | |
| GHSP-NBN113 | 0.912 | 0.845 | 0.721 | 26/27 | 1.000 | 0.542 | 26/27 | |
| GHSP-NBN106 | 0.914 | 0.793 | 0.566 | 26/27 | 1.000 | 0.373* | (26/27) | |
| GHSP-NBN104 | 0.920 | 0.721 | 0.340 | 26/27 | 1.000 | 0.266* | (27/28) | Cleavage site at 26/27 predicted at almost same probability as 27/28 by HMM and NN |
| GHSP-NBN102 | 0.904 | 0.724 | 0.380 | 26/27 | 0.999 | 0.296* | (27/28) | Cleavage site at 26/27 predicted at almost same probability as 27/28 by HMM and NN |
| GHSP-NBN99 | 0.888 | 0.754 | 0.516 | 26/27 | 0.999 | 0.526 | 26/27 | |
| Cuttoff values | 0.48 | 0.43 | 0.32 | | | 0.5 | | |

| | | | | | | | | |
|---|---|---|---|---|---|---|---|---|
| ⁵MATGSRTSLLLAFGLLCLSWLQEGSA - 26 amino acids *Below cuttoff values | | | | | | | | |

### Example 9: Neublastin Gene Sequence Optimization

The sequence of the native human neublastin gene was examined for codon usage for optimizing expression of human neublastin in CHO cells. The codons most commonly used in CHO cells were analyzed based on data current to 2002 using a method known in the art (Nakamura et al., 1999, Nucleic Acids Res., 27(1):292). The codon usage for *Cricetulus griseus* relied upon is presented in the Table below.

**Table. Frequency of codon usage in Cricetulus normalized per 1,000 codons.**

| | | | | | | | | | | | |
|---|---|---|---|---|---|---|---|---|---|---|---|
| (Phe) | UUU | 19.2 | (Ser) | UCU | 16.0 | (Tyr) | UAU | 12.7 | (Cys) | UGU | 8.5 |
| (Phe) | UUC | 22.2 | (Ser) | UCC | 17.2 | (Tyr) | UAC | 16.1 | (Cys) | UGC | 10.0 |
| (Leu) | UUA | 6.0 | (Ser) | UCA | 10.2 | (***) | UAA | 0.5 | (***) | UGA | 1.0 |
| (Leu) | UUG | 14.2 | (Ser) | UCG | 3.5 | (***) | UAG | 0.5 | (Trp) | UGG | 12.9 |
| (Leu) | CUU | 13.3 | (Pro) | CCU | 17.5 | (His) | CAU | 9.5 | (Arg) | CGU | 5.7 |
| (Leu) | CUC | 18.2 | (Pro) | CCC | 17.7 | (His) | CAC | 12.7 | (Arg) | CGC | 9.5 |
| (Leu) | CUA | 7.5 | (Pro) | CCA | 15.4 | (Gln) | CAA | 10.4 | (Arg) | CGA | 7.0 |
| (Leu) | CUG | 39.0 | (Pro) | CCG | 4.1 | (Gln) | CAG | 33.2 | (Arg) | CGG | 10.4 |
| (Ile) | AUU | 17.5 | (Thr) | ACU | 14.5 | (Asn) | AAU | 17.7 | (Ser) | AGU | 11.5 |
| (Ile) | AUC | 25.5 | (Thr) | ACC | 21.2 | (Asn) | AAC | 21.1 | (Ser) | AGC | 16.5 |
| (Ile) | AUA | 6.6 | (Thr) | ACA | 15.6 | (Lys) | AAA | 24.5 | (Arg) | AGA | 9.5 |
| (Met) | AUG | 23.4 | (Thr) | ACG | 4.4 | (Lys) | AAG | 39.1 | (Arg) | AGG | 9.8 |
| (Val) | GUU | 11.3 | (Ala) | GCU | 22.5 | (Asp) | GAU | 23.9 | (Gly) | GGU | 13.2 |
| (Val) | GUC | 16.0 | (Ala) | GCC | 26.6 | (Asp) | GAC | 27.6 | (Gly) | GGC | 22.1 |
| (Val) | GUA | 8.0 | (Ala) | GCA | 16.7 | (Glu) | GAA | 27.8 | (Gly) | GGA | 15.9 |
| (Val) | GUG | 29.9 | (Ala) | GCG | 4.3 | (Glu) | GAG | 40.7 | (Gly) | GGG | 13.5 |

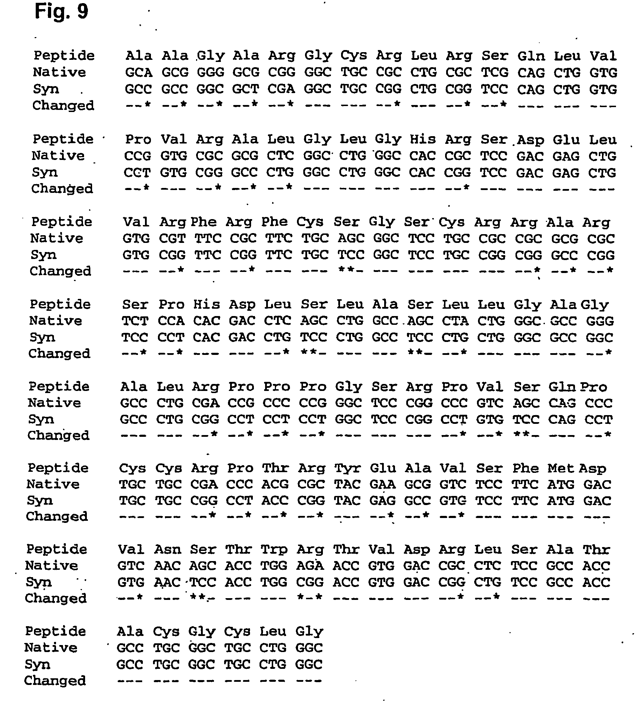

The native human nucleotide sequence encoding a C terminal 104 amino acid fragment (Roseblad et al., 2000, *Mol. Cell Neurosci.* 15(2):199; Baloh et al., *Neuron* 21:1291) and the nucleotide sequence of the synthetic gene are aligned in Figure 9 with the changed nucleotides indicated. The two sequences are 83.33% identical.

### Example 10: Cloning of the Neublastin Gene

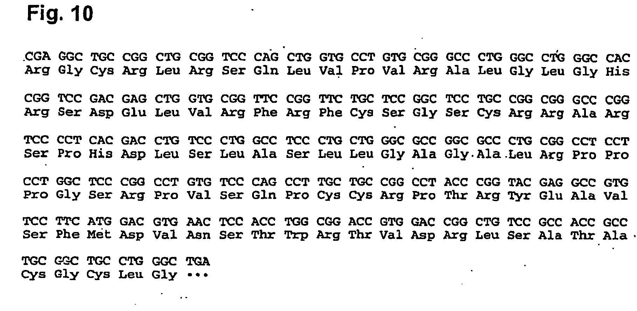

A 100 codon (300 nucleotides) 3' form of the neublastin gene was synthesized and cloned into an expression plasmid to facilitate the insertion of various signal peptide sequences linked to the 5' codons of neublastin. The 100 codon-form of the neublastin gene was assembled by combining 40 pmol of oligonucleotides KD3-464 through KD3-469 (Table 2) in 200 µL buffer (10 mM KCI, 10 mM (NH₄)₂SO₄, 20 mM Tris-Cl, 2 mM MgSO_{4,} 0.1% Triton X-100, pH 8.8) containing Deep Vent Polymerase (New England BioLabs, Beverly, MA). The contents were heated to 95°C for 4 minutes and cycled twenty times as follows: 95°C for 1 minute, 60°C for 30 seconds, and 72°C for 1 minute, followed by an extension at 72°C for four minutes. The termini were prepared by sequential digestion with Sall and Nhel. The 330 base pair fragment, which included a non-coding region of 30 base pairs flanking the neublastin gene, was gel-purified and ligated into plasmid pFRT/dhfr-1 (a derivative of pcDNA/FRT (Invitrogen, Carlsbad, CA) with the hygromycin gene replaced by a dihydrofolate reductase gene) that had been gel-purified and digested with Nhel and Xhol. The resulting plasmid was named pNBN026-35. The neublastin sequence within pNBN026-35 is presented in Figure 10.

The Table below identifies the oligonucleotides used in PCR and synthetic sequence assembly to generate signal peptide-neublastin fusion genes. Sequences are all indicated in the 5' to 3' orientation.

**table**

| Oligonucleotide Name | Oligonucleotide Sequence |
|---|---|
| KD3-464 | |
| KD3-465 | |
| KD3-466 | |
| KD3-467 | |
| KD3-468 | |
| KD3-469 | |
| KD3-471 | |
| KD3-472 | |
| KD3-477 | AAGCTTAGCTAGCGGATCCATGGCTACAGGTAAGC (SEQ ID NO: 79) |
| KD3-479 | |
| KD3-480 | |

### Example 11: Construction of Signal Peptide-Neublastin Fusion

Sequences encoding four different signal peptides were tested. These included signal sequences from neublastin, rat albumin, and human growth hormone. Additionally, a synthetic signal sequence resulted from two frame-shift mutations during PCR amplification to generate the neublastin signal peptide. The fusions were synthesized using either oligonucleotide assembly or PCR. The DNA fragments were ligated into pNBN026. The relevant DNA sequence of each of the four molecules described was confirmed by DNA sequence analysis.

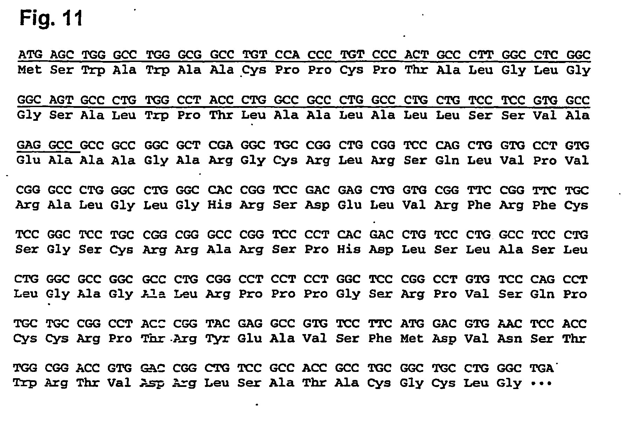

The synthetic signal sequence was synthesized by PCR amplification using oligonucleotides KD3-487, KD3-479, KD3-480, KD3-481, and KD3-482 (Table) and puReTaq polymerase (Pharmacia, Peapack, NJ,). PCR conditions included heating the reaction to 95°C for 4 minutes and then cycling twenty times at 95°C for 1 minute, 60°C for 30 seconds, 72°C for 1 minute, followed by an extension at 72°C for four minutes. The termini were prepared by digestion with Pstl and Xhol. The 330 base pair fragment was gel-purified and ligated into plasmid pNBN026 that was also gel-purified and digested with Pstl- and Xhol. The resulting plasmid was named pNBN030. There were two spontaneous frameshift mutations not predicted or encoded by the oligonucleotides which compensated for each other and kept the translated protein in frame. The DNA and protein sequences are shown in Figure 11.

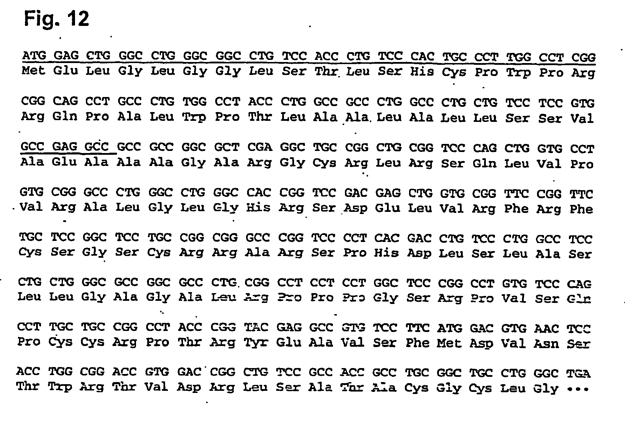

The neublastin signal sequence was synthesized by PCR amplification with oligonucleotides KD3-513 and KD3-514 (Table). The polymerase used was puReTaq (Pharmacia, Peapack, NJ,). PCR conditions included heating to 95°C for 4 minutes and cycling three times at 95°C for 1 minute, 60°C for 30 seconds, 72°C for 1 minute, followed by an extension at 72°C for four minutes. The termini were prepared by digestion with Nhel and Xhol. The 330 base pair fragment was gel-purified and ligated into plasmid pNBN030 that was gel-purified and digested with Nhel and Xhol. The resulting plasmid was named pNBN038. The DNA and protein sequences are shown in Figure 12.

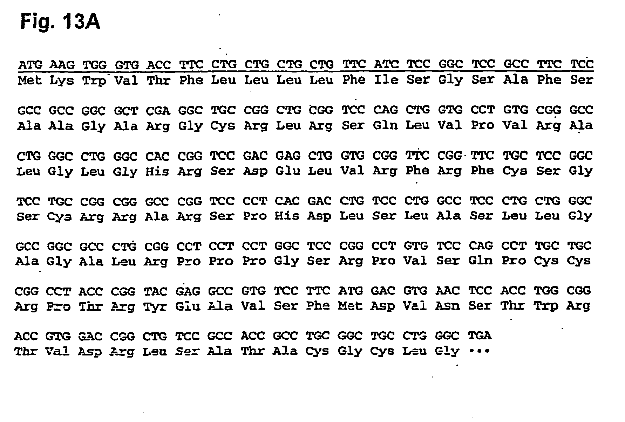

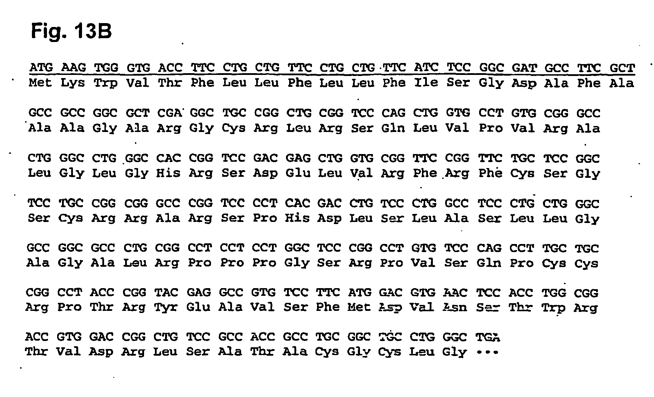

The albumin signal sequence was synthesized by PCR amplification with oligonucleotides KD3-487, KD3-471, and KD3-472 (Table). The polymerase used was puReTaq (Pharmacia, Peapack, NJ). PCR conditions included heating to 95°C for 4 minute and cycling twenty times at 95°C for 1 minute, 60°C for 30 seconds, 72°C for 1 minute, followed by an extension at 72°C for four minutes. The termini were prepared by digestion with Pstl and Xhol. The 330 base pair fragment was gel-purified and ligated into plasmid pNBN026 that was gel-purified and digested with Pstl and Xhol. The resulting plasmid was named pNBN029. The DNA and protein sequences are shown in Figure 13.

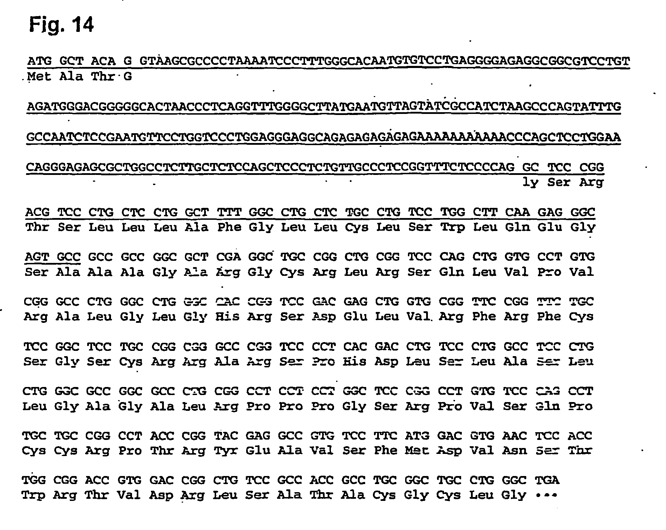

The human growth hormone signal sequence was synthesized by PCR amplification from plasmid pV30 (a pUC-based plasmid containing the genomic copy of the 5' end of the human growth hormone gene) with oligonucleotides KD3-487, KD3-477, and KD3-485 (Table 2). The polymerase used was puReTaq (Pharmacia, Peapack, NJ,). PCR conditions included heating to 95°C for 4 minutes and cycling twenty times at 95°C for 1 minute, 60°C for 30 seconds, 72°C for 1 minute, followed by an extension at 72°C for four minutes. The termini were prepared by digestion with Pstl and Xhol. The 330 base pair fragment was gel-purfied and ligated into plasmid pNBN026 that was gel-purified and digested with Pstl and Xhol. The resulting plasmid was named pNBN031. The DNA and protein sequences are shown in Figure 14.

### Example 12: CHO Cell Transfections

CHO-DG44 cells were previously transformed with DNA sequences containing the Flp Recombination Target (frt) (A1 cells). This A1 host cell line does not contain the dihydrofolate reductase gene (DHFR) and is thus DHFR-minus. Each of the plasmids described encodes the DHFR gene, the neublastin fusion gene, plus the frt site. Plasmid pOG44 encodes the Flp recombinase gene. Cotransfection of these plasmids into A1 cells resulted in the insertion of a single copy of the signal-peptide-neublastin fusion genes and DHFR into the chromosome. A1 cells were electroporated with the plasmid of interest plus plasmid pOG44 under conditions consistent with those described by the manufacturer (*i*.*e*. 0.4 mm cuvette, 280 volts, 950 microFarads)(BioRad, Hercules, California). Transformed cells expressing DHFR were selected for their ability to grow in alpha-minus medium Minimal Essential Medium-Alpha without nucleosides (Invitrogen, Carlsbad, CA) supplemented with 10% dialyzed fetal bovine serum (Hyclone, Logan, UT). Approximately two weeks later, colonies were isolated and expanded into larger vessels in the same selection medium. Cell cultures were transitioned to serum-free medium and analyzed.

### Example 13: Analysis of Transfected Cell Lines

Cell line candidates were screened for their ability to express neublastin. Aliquots of suspension cell cultures were centrifuged to separate cells from conditioned medium. The conditioned medium was removed from the cell pellet and both the media and the cell pellet were processed for reduced and denaturing electrophoresis on 16% polyacrylamide gels as generally described (Ausubel et al., supra). Upon completion of electrophoresis, the proteins were electroblotted onto a PVDF membrane and probed with rabbit polyclonal antiserum raised against neublastin. The antibody-Neublastin complex was detected by using a goat anti-rabbit polyclonal antiserum conjugated with horseradish-peroxidase (BioRad, Hercules, California).

Protein expressed from plasmids encoding the neublastin, synthetic, albumin, and human growth hormone signal peptides each expressed immuno-reactive neublastin in the cell pellet fractions. Only the albumin and human growth hormone signal peptides, however, expressed detectable levels of neublastin in conditioned medium. The electrophoretic mobility of all expressed neublastin polypeptides was consistent with an 11 kD, 104-amino acid form of neublastin.

### Example 14: Sequence of Neublastin Produced in CHO cells

Neublastin was purified from conditioned medium using an immunoaffinity column, generally as described (Ausubel et al., supra). The amino-terminal sequence was determined from protein purified from cell lines containing the albumin and growth hormone signal peptides. Neublastin was applied onto a micro TFA filter (Applied Biosystems, Foster City, CA) and subjected to automated Edman degradation chemistry. Amino terminal sequencing was performed on an ABI Procise 494 sequencer. The resulting PTH amino acids wer separated using an ABI 140C Microgradient system equipped with a PTH C18 reverse-phase column and analyzed using an ABI 7785A absorbance detector. For both constructs, the primary protein sequence began with the first residue of 104-amino acid C terminal fragment of full-length neublastin (*i*.*e*. alanine). The neublastin preparation expressed with the growth hormone signal peptide also included a 103-amino acid neublastin C terminal fragment lacking the amino-terminal alanine residue. The 103 amino acid form of neublastin began with an alanine. In both cases, the signal peptide functioned as anticipated, *i*.*e*., the neublastin polypeptide was secreted from the cell and the signal peptide was cleaved by the cell.

### Example 15: Mass Spectrometry of Recombinant Neublastin

Purified neublastin from conditioned medium of the cell lines containing constructs encoding the albumin and growth hormone signal peptides was analyzed by intact mass spectroscopy on a ZMD mass spectrometer (Waters, Milford, MA) as described generally by the manufacturer. For both constructs, the primary peak of deglycosylated samples corresponded to a 104-amino acid neublastin polypeptide (Figure 15). These two signal peptides functioned as anticipated, *i*.*e*., the neublastin polypeptide was secreted from the cell and the signal peptide was cleaved by the cell. Additionally, the glycosylated neublastin secreted from cells transfected, with constructs encoding neublastin and growth hormone signal peptide contained various glycoforms.

### Example 16: Detection of Neublastin Activity in Media From CHO Cells Transfected With Constructs Encoding Neublastin and Heterologous Signal Sequences

Biological activity was assessed using a kinase receptor activation ELISA (KIRA). The method has been previously described (Sadick et al., 1996, Anal. Biochem., 1996. 235(2):207. Briefly, NB41A3-mRL3 cells, an adherent murine neuroblastoma cell line which expresses Ret and GFRα3, were plated at 2 x 10⁵ cells per well in 24-well plates in Dulbecco's modified eagle medium (DMEM), supplemented with 10% fetal bovine serum, and cultured for 18 hours at 37°C and 5% CO₂.

The cells were washed with PBS, and treated with serial dilutions of neublastin in 0.25 mL of DMEM for 10 minutes at 37°C and 5% CO₂. Each sample was analyzed in duplicate. The cells were washed with 1 mL of PBS, and lysed for 1 hour at 4°C with 0.30 mL of 10mM Tris HC1, pH 8.0, 0.5% Nonidet P40, 0.2% sodium deoxycholate, 50mM NaF, 0.1 mM Na₃VO₄; 1 mM phenylmethylsulfonyl fluoride while gently rocking the plates. The lysates were further agitated by repeated pipetting and 0.25 mL of sample was transferred to a 96-well ELISA plate that had been coated with 5µg/mL of anti-Ret mAb (AA.GE7.3) (Upstate Biotechnology, Waltham, MA) in 50 mM carbonate buffer, pH 9.6 at 4°C for 18 hours and then blocked at room temperature for one hour with block buffer (20 mM Tris HC1 pH 7.5, 150 mM NaC1, 0.1% Tween-20 (TBST) containing 1% normal mouse serum and 3% bovine serum albumin).

After 2 hours of incubation at room temperature, the wells were washed 6 times with TBST. The plate was washed again before addition of 3,3',5,5'-tetramethylbenzidine dihydrochloride. After the color reaction, absorbance values were read at 450 nm from wells treated with lysate or lysis buffer only, and the background-corrected signal was plotted as a function of the concentration of ligand used for stimulation.

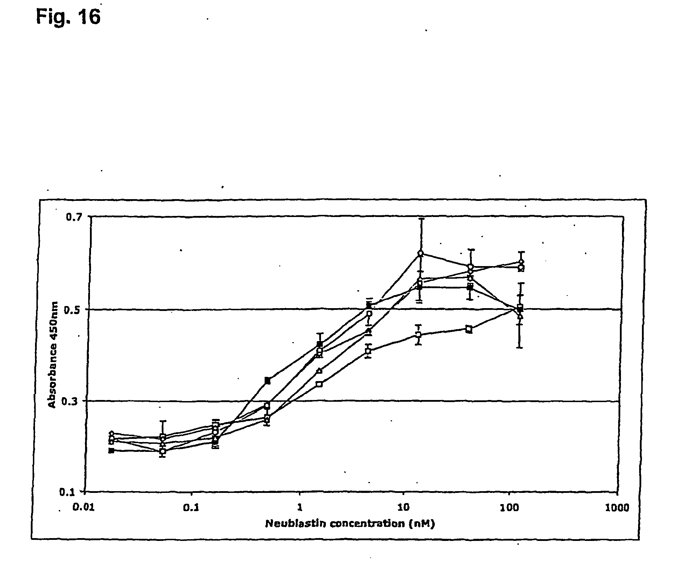

A series of dilutions of conditioned medium was tested, and functional neublastin was detected with a profile similar to a previously demonstrated batch of neublastin expressed, purified, and refolded from E. coli (Figure 16).

### Example 17: Mature Neublastin Expressed with a Heterologous Signal Peptide

Appropriate oligonucleotides can be produced according to the method described in Example 9, to clone a DNA sequence encoding a mature neublastin (*i*.*e*. a 113 C terminal fragment of full-length neublastin). A DNA sequence encoding a signal peptide from rat albumin or human growth hormone can be fused to the DNA sequence encoding a mature neublastin polypeptide as described, in Example 10. The DNA sequence can be transfected into a eukaryotic cell, *e.g*., a CHO cell, to produce a secreted mature neublastin.

To the extent publications and patents or patent applications cited herein contradict the disclosure contained in the specification, the specification is intended to supercede and/or take precedence over any such contradictory material.

### SEQUENCES

| SEQ ID NO | TYPE | DESCRIPTION |
|---|---|---|
| 1 | P | IgSP human |
| 2 | P | IgSP Monkey |
| 3 | P | IgSP Marmoset |
| 4 | P | IgSP Mouse |
| 5 | P | IgSP Pig |
| 6 | P | IgSP Rat |
| 7 | N | Nucleotide sequence of chimeric mouse IgSP-human |
| | | 113 NBN construct |
| 8 | N | Spliced transcript of SEQ ID No 7 |
| 9 | P | Chimeric protein encoded by SEQ ID No 8 and 7) |
| 10 | P | Human pre-pro Neublastin |
| 11 | P | Mouse pre-pro Neublastin |
| 12 | P | Rat pre-pro Neublastin |
| 13 | P | Mature Human 116 amino acid (aa) Neublastin |
| 14 | P | Mature Human 113 aa Neublastin |
| 15 | P | Mature mouse 119 aa Neublastin |
| 16 | P | Mature mouse 116 aa Neublastin |
| 17 | P | Mature rat 116 aa Neublastin |
| 18 | P | Mature rat 113 aa Neublastin |
| 19 | P | N-truncated human 104 aa Neublastin |
| 20 | P | N-truncated human 99 aa Neublastin |
| 21 | P | N-truncated human 140 aa Neublastin |
| 22 | P | N-truncated human 106 aa Neublastin |
| 23 | P | N-truncated human 102 aa Neublastin |
| 24 | P | Human NBN-SP |
| 25 | P | deltaproNBN140 |
| 26 | P | deltaproNBN113 |
| 27 | P | deltaproNBN106 |
| 28 | P | deltaproNBN104 |
| 29 | P | deltaproNBN102 |
| 30 | P | deltaproNBN99 |
| 31 | P | Chimeric mouse IgSP -human 140NBN protein |
| 32 | P | Chimeric mouse IgSP -human 113NBN protein |
| 33 | P | Chimeric mouse IgSP -human 106NBN protein |
| 34 | P | Chimeric mouse IgSP -human 104NBN protein |
| 35 | P | Chimeric mouse IgSP-human 102NBN protein |
| 36 | P | Chimeric mouse IgSP -human 99NBN protein |
| 37 | P | Rat albumin signal peptide |
| 38 | P | Chimeric rat AlbSP -human 140NBN protein |
| 39 | P | Chimeric rat AlbSP -human 113NBN protein |
| 40 | P | Chimeric rat AlbSP -human 106NBN protein |
| 41 | P | Chimeric rat AlbSP -human 104NBN protein |
| 42 | P | Chimeric rat AlbSP -human 102NBN protein |
| 43 | P | Chimeric rat AlbSP -human 99NBN protein |
| 44 | P | Modified rat albumin signal peptide |
| 45 | P | Chimeric ModAlbSP -human 140NBN protein |
| 46 | P | Chimeric ModAlbSP -human 113NBN protein |
| 47 | P | Chimeric ModAlbSP -human 106NBN protein |
| 48 | P | Chimeric ModAlbSP -human 104NBN protein |
| 49 | P | Chimeric ModAlbSP -human 102NBN protein |
| 50 | P | Chimeric ModAlbSP -human 99NBN protein |
| 51 | P | Human growth hormone signal peptide |
| 52 | P | Chimeric GHSP -human 140NBN protein |
| 53 | P | Chimeric GHSP -human 113NBN protein |
| 54 | P | Chimeric GHSP -human 106NBN protein |
| 55 | P | Chimeric GHSP -human 104NBN protein |
| 56 | P | Chimeric GHSP -human 102NBN protein |
| 57 | P | Chimeric GHSP -human 99NBN protein |
| 58 | N | Human 104NBN nucleotid sequence |
| 59 | N | Synthetic 104NBN nucleotide sequence |
| 60 | N | Neublastin sequence within plasmid pNBN026-35 |
| 61 | N | Chimeric Synthetic SP- Synthetic 104NBN nucleotide |
| | | sequence |
| 62 | P | Chimeric Synthetic SP- Synthetic 104NBN protein |
| 63 | N | Chimeric NBNSP- Synthetic 104NBN nucleotide |
| | | sequence |
| 64 | P | Chimeric NBNSP- Synthetic 104NBN protein |
| 65 | N | Chimeric AlbSP- Synthetic 104NBN nucleotide sequence |
| 66 | P | Chimeric AlbSP- Synthetic 104NBN protein |
| 67 | N | Chimeric GHSP with intron- Synthetic 104NBN nucleotide |
| | | sequence |
| 68 | P | Chimeric GHSP- Synthetic 104NBN protein |
| 69 | N | Chimeric ModAlbSP- Synthetic 104NBN nucleotide |
| | | sequence |
| 70 | P | Chimeric ModAlbSP- Synthetic 104NBN protein |
| 71 | N | Primer KD3-464 |
| 72 | N | Primer KD3-465 |
| 73 | N | Primer KD3-466 |
| 74 | N | Primer KD3-467 |
| 75 | N | Primer KD3-468 |
| 76 | N | Primer KD3-469 |
| 77 | N | Primer KD3-471 |
| 78 | N | Primer KD3-472 |
| 79 | N | Primer KD3-477 |
| 80 | N | Primer KD3-479 |
| 81 | N | Primer KD3-480 |
| 82 | N | deltaproNBN113 nucleotide sequence |

## Claims

1. A method for producing a biologically active Neublastin polypeptide, comprising culturing a cell comprising an expression vector comprising a nucleic acid comprising a promoter sequence operably linked to a nucleotide sequence encoding a signal peptide and a neublastin polypeptide, wherein said nucleotide sequence does not encode a neublastin pro-region, and wherein said signal peptide is an immunoglobulin signal peptide, a TGF-beta signal peptide, a GDF signal peptide, an IGF signal peptide, a BMP signal peptide, a Neurotrophin signal peptide, a PDGF signal peptide, an EGF signal peptide, an insulin signal peptide, an ADH signal peptide, an LH signal peptide, an FSH signal peptide, an ACTH signal peptide, an MSH signal peptide, a TSH signal peptide, a DHEA signal peptide, an interleukin signal peptide, a neurturin signal peptide, a GDNF signal peptide, a persephin signal peptide, an NGF signal peptide, a signal peptide being the signal peptide contained in SEQ ID NO:70, or a native Neublastin signal peptide.

2. The method according to claim 1, wherein the signal peptide is a heterologous signal peptide.

3. The method according to claim 1, wherein the signal peptide is a mammalian signal peptide.

4. The method according to claim 3, wherein the signal peptide is a human signal peptide, a rat signal peptide, a mouse signal peptide, a porcine signal peptide, a simian signal peptide, a canine signal peptide, a feline signal peptide, a bovine signal peptide, or an equine signal peptide.

5. The method according to claim 1, wherein the signal peptide is an immunoglobulin signal peptide.

6. The method according to claim 5, wherein the immunoglobulin signal peptide is selected from the group consisting of mouse IgSP of SEQ ID NO 4, rat IgSP of SEQ ID NO 6, porcine IgSP of SEQ ID NO 5, simian IgSP of SEO ID NO 2 or 3, and human IgSP of SEQ 10 NO 1.

7. The method of claim 6, wherein the IgSP is mouse IgSP of SEQ ID NO 4.

8. The method of claim 6, wherein the IgSP is human IgSP of SEQ ID NO 1.

9. The method according to claim 1, wherein the signal peptide is a native human Neublastin signal peptide.

10. The method according to any of the preceding claims, wherein the neublastin polypeptide is selected from the group consisting of mature NBN selected from Neublastin having a sequence identified as amino acids 1-140 of SEQ ID No 10, or amino acids 1-144 of SEQ ID No 11 or 12, SEQ ID No 13, 14, 15,16, 17 or 18, N-terminally truncated NBN, mutated NBN, or mutated and N-truncated NBN.

11. The method of claim 10, wherein the neublastin polypeptide is a mature NBN selected from the group consisting of neublastin having a sequence identified by SEQ ID No 13, 14, 15, 16, 17, or 18.

12. The method of claim 11, wherein the neublastin polypeptide is human mature113NBN of SEQ ID No 14.

13. The method according to any of the preceding claims 1-10, wherein the neublastin polypeptide is selected from the group consisting of the 116 C- terminal amino acids of human neublastin, the 115 C-terminal amino acids of human neublastin, the 114 C-terminal amino acids of human neublastin, the 113 C-terminal amino acids of human neublastin, the 112 C-terminal amino acids of human neublastin, the 111 C-terminal amino acids of human neublastin, the 110 C-terminal amino acids of human neublastin, the 109 C-terminal amino acids of human neublastin, the 108 C-terminal amino acids of human neublastin, the 107 C-terminal amino acids of human neublastin, the 106 C-terminal amino acids of human neublastin, the 105 C-terminal amino acids of human neublastin, the 104 C-terminal amino acids of human neublastin, the 103 C-terminal amino acids of human neublastin, the 102 C-terminal amino acids of human neublastin, the 101 C-terminal amino acids of human neublastin, the 100 C-terminal amino acids of human neublastin, and the 99 C-terminal amino acids of human neublastin.

14. The method of claim 1, wherein the neublastin polypeptide is selected from the group consisting of N-terminally truncated Neublastin with the 106, 104, 102 or 99 C terminal amino acids of SEQ ID NO 10.

15. The method according to claim 1, wherein the neublastin polypeptide is selected from the group consisting of the 116 C-terminal amino acids of human neublastin, the 113 C-terminal amino acids of human neublastin, and the 104 C- terminal amino acids of human neublastin.

16. The method of claim 10, wherein the N-terminally truncated Neublastin has the amino acid sequence of SEQ ID No 19.

17. The method of claim 10, wherein the N-terminally truncated Neublastin contains the 99 amino acids of SEQ ID NO 20.

18. The method of claim 1, wherein the neublastin polypeptide comprises an amino acid sequence derived from amino acids 8-113 of SEQ ID No. 14, wherein the variant neublastin polypeptide includes one or more of the amino acid substitutions selected from the group consisting of: an amino acid other than arginine at position 14 in the amino acid sequence of said variant polypeptide, an amino acid other than arginine at position 39 in the amino acid sequence of said variant polypeptide, an amino acid other than arginine at position 68 of said variant polypeptide, and an amino acid other than asparagine at position 95 of said variant polypeptide, wherein the positions of said amino acids are numbered in accordance with the polypeptide sequence of SEQ ID No. 14.

19. The method of claim 18, wherein said substitution at position 14, 39, or 68 is lysine.

20. The method according to any of the preceding claims, wherein the vector is a plasmid.

21. The method according to any of the preceding claim 1-19, wherein the vector is a virus vector.

22. The method according to any of the preceding claims, wherein the vector is a mammalian expression vector.

23. The method according to claim 21, wherein the vector is a replication-defective lentivirus particle.

24. The method according to claim 23, wherein said vector particle being produced from a lentiviral vector comprising a 5' lentiviral LTR, a tRNA binding site, a packaging signal, a promoter operably linked to a polynucleotide signal encoding said signal peptide and said neublastin peptide, an origin of second strand DNA synthesis and a 3' lentiviral LTR.

25. The method according to claim 21, wherein the vector is selected from the group consisting of retrovirus, such as HIV, SIV, FIV, EIAV, AAV, adenovirus, herpes virus, and MoMLV.

26. The method according to any of the preceding claims, wherein the promoter is selected from the group consisting of : ubiquitin promoter, CMV promoter, JeT promoter, SV40 promoter, Elongation Factor 1 alpha promoter, chick beta-actin, PGK, and MT-1.

27. The method according to claim 25, wherein the promoter is an inducible/repressible promoter, such as: Tet-On,Tet-Off, Rapamycin-inducible promoter, Mx1, and RU486.

28. The method according to any of the preceding claims, wherein the cell is a mammalian host cell.

29. The method of claim 28, wherein said mammal is selected from the group consisting of rodent, rabbit, dog, cat, pig, monkey, and human.

30. The method of claim 28, said cell being selected from the group consisting of CHO, HEK293, COS, PC12, HiB5, RN33b, neuronal cells, fetal cells, ARPE-19, immortalized fibroblast cells,C2C12, HeLa, HepG2, Retinal Pigment Epithelial (RPE) cells, striatal cells, neurons, astrocytes, and interneurons.

31. The method according to claim 30, wherein the cell is a CHO cell.

32. A nucleic acid comprising a polynucleotide sequence encoding a signal peptide and a neublastin polypeptide, wherein said polynucleotide sequence does not encode a neublastin pro-region, said signal peptide and said neublastin polypeptide being as defined in any of claims 1-19.

33. The nucleic acid according to claim 32, comprising a) the nucleotide sequence of SEQ ID NO: 64, 66, or 68, or b) a nucleotide sequence encoding the polypeptide of SEQ ID NO: 65, 67, or 69.

34. The nucleic acid according to claim 32 or 33, wherein the nucleotide sequence has been optimized for expression in a mammalian host.

35. An expression vector comprising the nucleic acid as defined in any of claims 32 34.

36. A pharmaceutical composition comprising the vector as defined in claim 35 and one or more of pharmaceutical acceptable adjuvants, excipients, carriers and/or diluents.

37. An isolated host cell transduced or transfected with the vector as defined in claim 35.

38. The cell according to claim 37, wherein the cell is a mammalian cell.

39. The mammalian cell according to claim 38, wherein the cell is capable of secreting Neublastin or a functional equivalent thereof in amounts in excess of 500ng 10⁶ cells/24 hours.

40. The mammalian cell according to claim 38, being selected from the group consisting of CHO, HEK293, COS, PC12, HiB5, RN33b, immortalized fibroblast cells, C2C12, HeLa, HepG2, RPE cell lines, and ARPE-19 cells.

41. The mammalian cell according to claim 39, being selected from the group consisting of CHO, HEK293, COS, and ARPE-19.

42. The mammalian cell according to claim 38, being selected from the group consisting of RPE cells, neuronal cells, neuronal precursor cells, stem cells, and fetal cells.

43. The mammalian cell according to claim 38, being attached to a support matrix.

44. A packaging cell line capable of producing an infective vector particle, said vector particle comprising a retrovirally derived genome comprising a 5' retroviral LTR, a tRNA binding site, a packaging signal, a promoter operably linked to a polynucleotide sequence encoding a signal peptide and a neublastin polypeptide, wherein said nucleotide sequence does not encode a neublastin pro-region and said signal peptide and neublastin peptide being as defined in any of claims 1-19, and an origin of second strand DNA synthesis, and a 3' retroviral LTR.

45. The packaging cell line according to claim 44, wherein the vector particle is replication defective.

46. The packaging cell line according to claim 44, wherein the genome is lentivirally derived and the LTRs are lentiviral.

47. An implantable cell culture device, the device comprising:
i.a semipermeable membrane permitting the diffusion of a growth factor therethrough; and
ii. at least one isolated host cell as defined in claim 37.

48. The device of claim 47, wherein the semipermeable membrane is immunoisolatory.

49. The device of claim 47, wherein the semipermeable membrane is microporous.

50. The device of claim 47, wherein the device further comprises a matrix disposed within the semipermeable membrane.

51. The device of claim 47, wherein the device further comprises a tether anchor.

52. Use of the vector according to claim 35 or the device according to any of the claims 47 to 51 as a medicament.

53. Use of the vector according to claim 35 for the preparation of a medicament for the treatment of a nervous system disorder.

54. The use of claim 53, wherein the nervous system disorder is selected from the group consisting of peripheral neuropathy including neuropathic pain, spinal cord injury, spinal root avulsion, tic doloreaux, and causalgia.

55. The use according to claim 52, wherein the medicament is for the treatment of an eye disease, such as corneal wounds and ulcers, and retinopathies.

56. Use of the vector according to claim 35 for the preparation of a medicament for the treatment of a CNS disorder.

57. The use according to claim 56, wherein the CNS disorder is a neurodegenerative disease.

58. The use of claim 57, wherein the neurodegenerative disease is peripheral neuropathy including neuropathic pain.

59. A polypeptide comprising a signal peptide and a neublastin polypeptide, wherein the polypeptide lacks a neublastin pro-region, said signal peptide and said neublastin polypeptide being as defined in any of claims 1-19.

## Patentansprüche

1. Verfahren zum Herstellen eines biologisch aktiven Neublastin Polypeptids, umfassend Kultivieren einer Zelle umfassend einen Expressionsvektor umfassend eine Nukleinsäure umfassend eine Promotorsequenz, die mit einer Nukleotidsequenz wirksam verbunden ist, die ein Signalpeptid und ein Neublastin Polypeptid kodiert, worin die Nukleotidsequenz keine Neublastin pro-Region kodiert, und worin das Signalpeptid ein Immunoglobulin Signalpeptid ist, ein TGF-beta Signalpeptid, ein GDF Signalpeptid, ein IGF Signalpeptid, ein BMP Signalpeptid, ein Neurotrophin Signalpeptid, ein PDGF Signalpeptid, ein EGF Signalpeptid, ein Insulin Signalpeptid, ein ADH Signalpeptid, ein LH Signalpeptid, ein FSH Signalpeptid, ein ACTH Signalpeptid, ein MSH Signalpeptid, ein TSH Signalpeptid, ein DHEA Signalpeptid, ein Interleukin Signalpeptid, ein Neurturin Signalpeptid, ein GDNF Signalpeptid, ein Persephin Signalpeptid, ein NGF Signalpeptid, ein Signalpeptid, das das in Seq. ID Nr. 70 enthaltene Signalpeptid ist, oder ein natives Neublastin Signalpeptid.

2. Verfahren nach Anspruch 1, worin das Signalpeptid ein heterologes Signalpeptid ist.

3. Verfahren nach Anspruch 1, worin das Signalpeptid ein Signalpeptid von einem Säuger ist.

4. Verfahren nach Anspruch 3, worin das Signalpeptid ein menschliches Signalpeptid, ein Signalpeptid von einer Ratte, ein Signalpeptid von einer Maus, ein schweineähnliches Signalpeptid, ein Signalpeptid von einem Affen, ein Signalpeptid von einem Hund, ein Signalpeptid von einer Katze, ein Signalpeptid von einem Rind, oder ein Signalpeptid von einem Pferd ist.

5. Verfahren nach Anspruch 1, worin das Signalpeptid ein Immunoglobulin Signalpeptid ist.

6. Verfahren nach Anspruch 5, worin das Immunoglobulin Signalpeptid ausgewählt ist aus der Gruppe bestehend aus IgSP der Maus mit Seq. ID Nr. 4, IgSP der Ratte mit Seq. ID Nr. 6, IgSP des Schweins mit Seq. ID Nr. 5, IgSP des Affens mit Seq. ID Nr. 2 oder 3, und menschliches IgSP mit Seq. ID Nr. 1.

7. Verfahren nach Anspruch 6, worin das IgSP IgSP der Maus mit Seq. ID Nr. 4 ist.

8. Verfahren nach Anspruch 6, worin das IgSP menschliches IgSP mit Seq. ID Nr. 1 ist.

9. Verfahren nach Anspruch 1, worin das Signalpeptid ein natives menschliches Neublastin Signalpeptid ist.

10. Verfahren nach einem der vorstehenden Ansprüche, worin das Neublastin Polypeptid ausgewählt ist aus der Gruppe bestehend aus reifem NBN ausgewählt von Neublastin mit einer Sequenz, die als Aminosäuren 1-140 von Seq. ID Nr. 10, oder Aminosäuren 1-144 von Seq. ID Nr. 11 oder 12, Seq. ID Nr. 13, 14, 15, 16, 17 oder 18, N-terminal trunkiertes NBN, mutiertes NBN, oder mutiertes und N-trunkiertes NBN identifiziert ist.

11. Verfahren nach Anspruch 10, worin das Neublastin Polypeptid ein reifes NBN ist ausgewählt aus der Gruppe bestehend aus Neublastin mit einer Sequenz, die durch Seq. ID Nr. 13, 14, 15, 16, 17, oder 18 identifiziert ist.

12. Verfahren nach Anspruch 11, worin das Neublastin Polypeptid menschliches, reifes 113NBN mit Seq. ID Nr. 14 ist.

13. Verfahren nach einem der vorstehenden Ansprüche 1-10, worin das Neublastin Polypeptid ausgewählt ist aus der Gruppe bestehend aus den 116 C-terminalen Aminosäuren von menschlichem Neublastin, den 115 C-terminalen Aminosäuren von menschlichem Neublastin, den 114 C-terminalen Aminosäuren von menschlichem Neublastin, den 113 C-terminalen Aminosäuren von menschlichem Neublastin, den 112 C-terminalen Aminosäuren von menschlichem Neublastin, den 111 C-terminalen Aminosäuren von menschlichem Neublastin, den 110 C-terminalen Aminosäuren von menschlichem Neublastin, den 109 C-terminalen Aminosäuren von menschlichem Neublastin, den 108 C-terminalen Aminosäuren von menschlichem Neublastin, den 107 C-terminalen Aminosäuren von menschlichem Neublastin, den 106 C-terminalen Aminosäuren von menschlichem Neublastin, den 105 C-terminalen Aminosäuren von menschlichem Neublastin, den 104 C-terminalen Aminosäuren von menschlichem Neublastin, den 103 C-terminalen Aminosäuren von menschlichem Neublastin, den 102 C-terminalen Aminosäuren von menschlichem Neublastin, den 101 C-terminalen Aminosäuren von menschlichem Neublastin, den 100 C-terminalen Aminosäuren von menschlichem Neublastin, und den 99 C-terminalen Aminosäuren von menschlichem Neublastin.

14. Verfahren nach Anspruch 1, worin das Neublastin Polypeptid ausgewählt ist aus der Gruppe bestehend aus N-terminal trunkiertem Neublastin mit den 106, 104, 102, oder 99 C-terminalen Aminosäuren der Seq. ID Nr. 10.

15. Verfahren nach Anspruch 1, worin das Neublastin Polypeptid ausgewählt ist aus der Gruppe bestehend aus den 116 C-terminalen Aminosäuren von menschlichem Neublastin, den 113 C-terminalen Aminosäuren von menschlichem Neublastin, und den 104 C-terminalen Aminosäuren von menschlichem Neublastin.

16. Verfahren nach Anspruch 10, worin das N-terminal trunkierte Neublastin die Aminosäuresequenz von Seq. ID Nr. 19 aufweist.

17. Verfahren nach Anspruch 10, worin das N-terminal trunkierte Neublastin die 99 Aminosäuren von Seq. ID Nr. 20 enthält.

18. Verfahren nach Anspruch 1, worin das Neublastin Polypeptid eine Aminosäuresequenz umfasst, die von den Aminosäuren 8-113 der Seq. ID Nr. 14 abgeleitet ist, worin die Variante des Neublastin Polypeptids eine oder mehrere der Aminosäuresubstitutionen umfasst, die ausgewählt sind aus der Gruppe bestehend aus: einer Aminosäure, die von Arginin an Position 14 in der Aminosäuresequenz der Variante des Polypeptids verschieden ist, einer Aminosäure, die von Arginin an Position 39 in der Aminosäuresequenz der Variante des Polypeptids verschieden ist, einer Aminosäure, die von Arginin an Position 68 in der Aminosäuresequenz der Variante des Polypeptids verschieden ist, und einer Aminosäure, die von Arginin an Position 95 in der Aminosäuresequenz der Variante des Polypeptids verschieden ist, worin die Positionen der Aminosäuren übereinstimmend mit der Polypeptidsequenz der Seq. ID Nr. 14 nummeriert sind.

19. Verfahren nach Anspruch 18, worin die Substitution an Position 14, 39, oder 68 Lysin ist.

20. Verfahren nach einem der vorstehenden Ansprüche, worin der Vektor ein Plasmid ist.

21. Verfahren nach einem der vorstehenden Ansprüche 1-19, worin der Vektor ein viraler Vektor ist.

22. Verfahren nach einem der vorstehenden Ansprüche, worin der Vektor ein Säuger-Expressionsvektor ist.

23. Verfahren nach Anspruch 21, worin der Vektor ein Lentiviruspartikel mit fehlerbehafteter Replikation ist.

24. Verfahren nach Anspruch 23, worin das Vektorpartikel aus einem lentiviralen Vektor hergestellt ist, das eine 5' lentivirale LTR, eine tRNA Bindestelle, ein Verpackungssignal, einen Promotor, der mit einem Polynukleotidsignal wirksam verbunden ist, das das Signalpeptid und das Neublastin Peptid kodiert, einen Ursprung einer Zweitstrang DNA Synthese und eine 3' lentivirale LTR umfasst.

25. Verfahren nach Anspruch 21, worin der Vektor ausgewählt ist aus der Gruppe bestehend aus einem Retrovirus, wie beispielsweise HIV, SIV, FIV, EIAV, AAV, Adenovirus, Herpes Virus, und MoMLV.

26. Verfahren nach einem der vorstehenden Ansprüche, wobei der Promotor ausgewählt ist aus der Gruppe bestehend aus: Ubiquitin-Promotor, CMV-Promotor, JeT-Promotor, SV40-Promotor, Elongationsfaktor 1-alpha-Promotor, Huhn-beta-Aktin, PGK und MT-1.

27. Verfahren nach Anspruch 25, wobei der Promotor ein induzierbarer/reprimierbarer Promotor ist, wie Tet-On, Tet-Off, Rapamycin-induzierbarer Promotor, Mx1 und RU486.

28. Verfahren nach einem der vorstehenden Ansprüche, wobei die Zelle eine Säugerwirtszelle ist.

29. Verfahren nach Anspruch 28, wobei der Säuger ausgewählt ist aus der Gruppe bestehend aus Nager, Kaninchen, Hund, Katze, Schwein, Affe und Mensch.

30. Verfahren nach Anspruch 28, wobei die Zelle ausgewählt ist aus der Gruppe bestehend aus CHO, HEK293, COS, PC12, HiB5, RN33b, neuronale Zellen, fetale Zellen, ARPE-19, immortalisierte Fibroblastenzellen, C2C12, HeLa, HepG2, retinale Pigmentepithelzellen (RPE), striatale Zellen, Neuronen, Astrozyten und Interneuronen.

31. Verfahren nach Anspruch 30, wobei die Zelle eine CHO-Zelle ist.

32. Nukleinsäure umfassend eine für ein Signalpeptid und ein Neublastin-Polypeptid kodierende Polynukleotidsequenz, worin die Polynukleotidsequenz keine Neublastin-Pro-Region kodiert, wobei das Signal-Peptid und das Neublastin-Polypeptid wie in einem der Ansprüche 1-19 definiert vorliegen.

33. Nukleinsäure nach Anspruch 32, umfassend a) die Nukleotidsequenz von SEQ ID Nr. 64, 66 oder 68, oder b) eine das Polypeptid von SEQ ID Nr. 65, 67 oder 69 kodierende Nukleotidsequenz.

34. Nukleinsäure nach Anspruch 32 oder 33, worin die Nukleotidsequenz zur Expression in einem Säugerwirt optimiert wurde.

35. Expressionsvektor umfassend die Nukleinsäure wie in einem der Ansprüche 32-34 definiert.

36. Pharmazeutische Zusammensetzung umfassend den wie in Anspruch 35 definierten Vektor und einen oder mehrere pharmazeutisch annehmbare Hilfsstoffe, Arzneimittelträger, Träger und/oder Verdünnungsmittel.

37. Isolierte Wirtszelle, die mit dem wie in Anspruch 35 definierten Vektor transduziert oder transfiziert wurde.

38. Zelle nach Anspruch 37, worin die Zelle eine Säugerzelle ist.

39. Säugerzelle nach Anspruch 38, worin die Zelle Neublastin oder ein funktionales Äquivalent davon in Mengen über 500 ng/10⁶ Zellen/24 Stunden absondern kann.

40. Säugerzelle nach Anspruch 38, ausgewählt aus der Gruppe bestehend aus CHO, HEK293, COS, PC12, HiB5, RN33b, immortalisierten Fibroblastenzellen, C2C12, HeLa, HepG2, RPE Zelllinien und ARPE-19 Zellen.

41. Säugerzelle nach Anspruch 39, ausgewählt aus der Gruppe bestehend aus CHO, HEK293, COS und ARPE-19.

42. Säugerzelle nach Anspruch 38, ausgewählt aus der Gruppe bestehend aus RPE Zellen neuronalen Zellen, neuronalen Vorläuferzellen, Stammzellen und fötalen Zellen.

43. Säugerzelle nach Anspruch 38, die an eine Trägermatrix angebracht ist.

44. Verpackungszelllinie, die ein infektiöses Vektorpartikel erzeugen kann, welcher Vektorpartikel ein von einem Retrovirus abgeleitetes Genom umfasst, das einen 5' retroviralen LTR umfasst, eine tRNA Bindungsstelle, ein Verpackungssignal, einen Promotor, der mit einer Polynukleotidsequenz wirksam verbunden ist, die ein Signalpeptid und ein Neublastin-Polypeptid kodiert, worin die Nukleotidsequenz keine Neublastin-Pro - Region kodiert und wobei das Signalpeptid und Neublastinpeptid wie in einem der Ansprüche 1-19 festgelegt sind, und einen Ursprung einer Zweitstrang DNA Synthese, und einen 3'-retroviralen LTR.

45. Verpackungszellelinie nach Anspruch 44, worin der Vektorpartikel eine fehlerhafte Replikation aufweist.

46. Verpackungszelllinie nach Anspruch 44, worin das Genom von einem Lentivirus abgeleitet ist und die LTRs lentiviral sind.

47. Implantierbare Zellkultureinrichtung, wobei die Einrichtung umfasst:
i. eine semipermeable Membran, die die Diffusion eines Wachstumsfaktors **dadurch** gestattet, und
ii. mindestens eine, wie in Anspruch 37 definierte, isolierte Wirtszelle.

48. Einrichtung nach Anspruch 47, worin die semipermeable Membran immunisolatorisch ist.

49. Einrichtung nach Anspruch 47, worin die semipermeable Membran mit Mikroporen versehen ist.

50. Einrichtung nach Anspruch 47, worin die Einrichtung weiterhin eine Matrix umfasst, die in der semipermeablen Membran angeordnet ist.

51. Einrichtung nach Anspruch 47, worin die Einrichtung weiterhin einen Halteanker umfasst.

52. Verwendung des Vektors nach Anspruch 35 oder der Einrichtung nach einem der Ansprüche 47 bis 51 als ein Medikament.

53. Verwendung des Vektors nach Anspruch 35 zur Herstellung eines Medikaments zur Behandlung einer Störung des Nervensystems.

54. Verwendung nach Anspruch 53, worin die Störung des Nervensystems ausgewählt ist aus der Gruppe bestehend aus peripherer Neuropathie einschließlich neuropathischem-Schmerz, Rückenmarksverletzung, spinalem Wurzelabriss, Tick-Doloreaux und Kausalgie.

55. Verwendung nach Anspruch 52, worin das Medikament für die Behandlung einer Augenerkrankung, wie beispielsweise Hornhautwunden und Geschwüre und Retinopathien, vorgesehen ist.

56. Verwendung des Vektors nach Anspruch 35 für die Herstellung eines Medikaments für die Behandlung einer Störung des ZNS.

57. Verwendung nach Anspruch 56, worin die Störung des ZNS eine neurodegenerative Erkrankung ist.

58. Verwendung nach Anspruch 57, worin die neurodegenerative Erkrankung periphere Neuropathie einschließlich neuropathischem Schmerz ist.

59. Polypeptid umfassend ein Signalpeptid und ein Neublastin-Polypeptid, worin dem Polypeptid eine Neublastin-Proregion fehlt, wobei das Signalpeptid und das Neublastin-Polypeptid wie in einem der Ansprüche 1-19 definiert sind.

## Revendications

1. Procédé de production d'un polypeptide de neublastine biologiquement actif, comprenant la culture d'une cellule comprenant un vecteur d'expression comprenant un acide nucléique comprenant une séquence promoteur reliée de manière fonctionnelle à une séquence nucléotidique codant pour un peptide signal et un polypeptide de neublastine, dans lequel ladite séquence nucléotidique ne code pas pour une pro-région de neublastine, et dans lequel ledit peptide signal est un peptide signal d'immunoglobuline, un peptide signal de TGF-β, un peptide signal de GDF, un peptide signal d'IGF, un peptide signal de BMP, un peptide signal de neurotrophine, un peptide signal de PDGF, un peptide signal d'EGF, un peptide signal d'insuline, un peptide signal d'ADH, un peptide signal de LH, un peptide signal de FSH, un peptide signal d'ACTH, un peptide signal de MSH, un peptide signal de TSH, un peptide signal de DHEA, un peptide signal d'interleukine, un peptide signal de neurturine, un peptide signal de GDNF, un peptide signal de perséphine, un peptide signal de NGF, un peptide signal qui est le peptide signal contenu dans la séquence SEQ ID n° 70, ou un peptide signal de neublastine natif.

2. Procédé selon la revendication 1, dans lequel le peptide signal est un peptide signal hétérologue.

3. Procédé selon la revendication 1, dans lequel le peptide signal est un peptide signal de mammifère.

4. Procédé selon la revendication 3, dans lequel le peptide signal est un peptide signal humain, un peptide signal de rat, un peptide signal de souris, un peptide signal de porc, un peptide signal de singe, un peptide signal de chien, un peptide signal de félin, un peptide signal de bovin ou un peptide signal de cheval.

5. Procédé selon la revendication 1, dans lequel le peptide signal est un peptide signal d'immunoglobuline.

6. Procédé selon la revendication 5, dans lequel le peptide signal d'immunoglobuline est choisi dans le groupe constitué par l'IgSP de souris de la séquence SEQ ID n° 4, l'IgSP de rat de la séquence SEQ ID n° 6, l'IgSP de porc de la séquence SEQ ID n° 5, l'IgSP de singe de la séquence SEQ ID n° 2 ou 3, et l'IgSP humain de la séquence SEQ ID n° 1.

7. Procédé selon la revendication 6, dans lequel l'IgSP est l'IgSP de souris de la séquence SEQ ID n° 4.

8. Procédé selon la revendication 6, dans lequel l'IgSP est l'IgSP humain de la séquence SEQ ID n° 1.

9. Procédé selon la revendication 1, dans lequel le peptide signal est un peptide signal de neublastine humain natif.

10. Procédé selon l'une quelconque des revendications précédentes, dans lequel le polypeptide de neublastine est choisi dans le groupe constitué par la NBN mature choisie parmi la neublastine ayant une séquence identifiée comme étant les acides amines 1 à 140 de la séquence SEQ ID n° 10, ou les acides amines 1 à 144 de la séquence SEQ ID n° 11 ou 12, SEQ ID n° 13, 14, 15, 16, 17 ou 18, la NBN tronquée à N terminal, la NBN mutée ou la NBN mutée et N-tronquée.

11. Procédé selon la revendication 10, dans lequel le polypeptide de neublastine est une NBN mature choisie dans le groupe constitué par une neublastine ayant une séquence identifiée par la séquence SED ID n° 13, 14, 15, 16, 17 ou 18.

12. Procédé selon la revendication 11, dans lequel le polypeptide de neublastine est la NBN mature humaine 113 de la séquence SEQ ID n° 14.

13. Procédé selon l'une quelconque des revendications 1 à 10 précédentes, dans lequel le polypeptide de neublastine est choisi dans le groupe constitué par les séquences de 116 acides aminés à C terminal de neublastine humaine, 115 acides aminés à C terminal de neublastine humaine, 114 acides aminés à C terminal de neublastine humaine, 113 acides aminés à C terminal de neublastine humaine, 112 acides aminés à C terminal de neublastine humaine, 111 acides aminés à C terminal de neublastine humaine, 110 acides aminés à C terminal de neublastine humaine, 109 acides aminés à C terminal de neublastine humaine, 108 acides aminés à C terminal de neublastine humaine, 107 acides aminés à C terminal de neublastine humaine, 106 acides aminés à C terminal de neublastine humaine, 105 acides aminés à C terminal de neublastine humaine, 104 acides aminés à C terminal de neublastine humaine, 103 acides aminés à C terminal de neublastine humaine, 102 acides aminés à C terminal de neublastine humaine, 101 acides aminés à C terminal de neublastine humaine, 100 acides aminés à C terminal de neublastine humaine, et 99 acides aminés à C terminal de neublastine humaine.

14. Procédé selon la revendication 1, dans lequel le polypeptide de neublastine est choisi dans le groupe constitué par une neublastine tronquée en un N terminal avec les 106, 104, 102 ou 99 acides amines à C terminal de la séquence SEQ ID n° 10.

15. Procédé selon la revendication 1, dans lequel le polypeptide de neublastine est choisi dans le groupe constitué par les séquences de 116 acides amines à C terminal de neublastine humaine, 113 acides amines à C terminal de neublastine humaine, et 104 acides amines à C terminal de neublastine humaine.

16. Procédé selon la revendication 10, dans lequel la neublastine tronquée en un N terminal a la séquence d'acides aminés SEQ ID n° 19.

17. Procédé selon la revendication 10, dans lequel la neublastine tronquée en un N terminal contient les 99 acides aminés de la séquence SEQ ID n° 20.

18. Procédé selon la revendication 1, dans lequel le polypeptide de neublastine comprend une séquence d'acides aminés dérivée des acides aminés 8 à 113 de la séquence SEQ ID n° 14, dans lequel le polypeptide de neublastine variant comprend une ou plusieurs substitutions d'acides aminés choisie(s) dans le groupe constitué par un acide aminé autre que l'arginine en position 14 de la séquence d'acides aminés dudit polypeptide variant, un acide aminé autre que l'arginine en position 39 dans la séquence d'acides aminés dudit polypeptide variant, un acide aminé autre que l'arginine en position 68 dudit polypeptide variant, et un acide aminé autre que l'asparagine en position 95 dudit polypeptide variant, où les positions desdits acides aminés sont numérotés conformément à la séquence du polypeptide SEQ ID n° 14.

19. Procédé selon la revendication 18, dans lequel ladite substitution en position 14, 39 ou 68 est la lysine.

20. Procédé selon l'une quelconque des revendications précédentes, dans lequel le vecteur est un plasmide.

21. Procédé selon l'une quelconque des revendications 1 à 19 précédentes, dans lequel le vecteur est un vecteur de virus.

22. Procédé selon l'une quelconque des revendications précédentes, dans lequel le vecteur est un vecteur d'expression de mammifère.

23. Procédé selon la revendication 21, dans lequel le vecteur est une particule de lentivirus à réplication défectueuse.

24. Procédé selon la revendication 23, dans lequel ladite particule de vecteur étant produite à partir d'un vecteur lentiviral comprenant une LTR lentivirale en 5', un site de liaison à l'ARNt, un signal d'encapsidation, un promoteur reliée de manière fonctionnelle à un signal polynucléotidique codant pour ledit peptide signal et ledit peptide de neublastine, une origine de la synthèse du deuxième brin d'ADN et une LTR lentivirale en 3'.

25. Procédé selon la revendication 21, dans lequel le vecteur est choisi dans le groupe constitué d'un rétrovirus, tel que le VIH, le VIS, le VIF, le virus EIAV, le VAA, l'adénovirus, le virus de l'herpès, et le virus MoMLV.

26. Procédé selon l'une quelconque des revendications précédentes, dans lequel le promoteur est choisi dans le groupe constitué par le promoteur d'ubiquitine, le promoteur de CMV, le promoteur de JeT, le promoteur de SV40, le promoteur du facteur d'élongation 1α, la 5-actine de poussin, PGK et MT-1.

27. Procédé selon la revendication 25, dans lequel le promoteur est un promoteur inductible/répressible, tel que Tet-On, Tet-Off, le promoteur inductible de la rapamycine, Mx1 et RU486.

28. Procédé selon l'une quelconque des revendications précédentes, dans lequel la cellule est une cellule hôte d'un mammifère.

29. Procédé selon la revendication 28, dans lequel ledit mammifère est choisi dans le groupe constitué par les rongeurs, les lapins, les chiens, les chats, les cochons, les singes et les êtres humains.

30. Procédé selon la revendication 28, dans lequel ladite cellule est choisie dans le groupe constitué par CHO, HEK293, COS, PC12, HiB5, RN33b, les cellules neuronales, les cellules foetales, ARPE-19, les cellules fibroblastes immortalisées, C2C12, HeLa, HepG2, les cellules épithéliales de pigment rétinien (RPE), les cellules striatales, les neurones, les astrocytes, et les interneurones.

31. Procédé selon la revendication 30, dans lequel la cellule est une cellule CHO.

32. Acide nucléique comprenant une séquence polynucléotidique codant pour un peptide signal et un polypeptide de neublastine, dans lequel ladite séquence polynucléotidique ne code pas pour une pro-région de neublastine, ledit peptide signal et ledit polypeptide de neublastine étant tels que définis selon l'une quelconque des revendications 1 à 19.

33. Acide nucléique selon la revendication 32, comprenant a) la séquence nucléotidique SED ID n° 64, 66 ou 68, ou b) une séquence nucléotidique codant pour le polypeptide de SEQ ID n° 65, 67 ou 69.

34. Acide nucléique selon la revendication 32 ou 33, dans lequel la séquence nucléotidique a été optimisée pour l'expression dans un hôte mammifère.

35. Vecteur d'expression comprenant l'acide nucléique tel que défini selon l'une quelconque des revendications 32 à 34.

36. Composition pharmaceutique comprenant le vecteur tel que défini selon la revendication 35 et un ou plusieurs adjuvants, excipients, supports et/ou diluants pharmaceutiquement acceptables.

37. Cellule hôte isolée transduite ou transférée avec le vecteur tel que défini dans la revendication 35.

38. Cellule selon la revendication 37, dans laquelle la cellule est une cellule de mammifère.

39. Cellule de mammifère selon la revendication 38, dans laquelle la cellule est capable de sécréter la neublastine ou un équivalent fonctionnel de celle-ci en des quantité en excès de 500 ng/10⁶ cellules/24 heures.

40. Cellule de mammifère selon la revendication 38, choisie dans le groupe constitué par CHO, HEK293, COS, PC12, HiB5, RN33b, les cellules fibroblastes immortalisées, C2C12, HeLa, HepG2, les lignées cellulaires RPE, et les cellules ARPE-19.

41. Cellule de mammifère selon la revendication 39, choisie dans le groupe constitué par CHO, HEK293, COS, et ARPE-19.

42. Cellule de mammifère selon la revendication 38, choisie dans le groupe constitué par les cellules RPE, les cellules neuronales, les cellules précurseurs neuronales, les cellules souches, et les cellules foetale.

43. Cellule de mammifère selon la revendication 38, attachées à un support matriciel.

44. Lignée cellulaire d'encapsidation capable de produire une particule de vecteur infectieux, ladite particule de vecteur comprenant un génome dérivé de manière rétrovirale comprenant une LTR rétrovirale en 5', un site de liaison de l'ARNt, un signal d'encapsidation, un promoteur relié de manière fonctionnelle à une séquence polynucléotidique codant pour un peptide signal et un polypeptide de neublastine, dans laquelle ladite séquence nucléotidique ne code pas pour une pro-région de neublastine et lesdits peptide signal et peptide de neublastine étant tels que définis selon l'une quelconque des revendications 1 à 19, et une origine de la synthèse du deuxième brin d'ADN, et une LTR rétrovirale en 3'.

45. Lignée cellulaire d'encapsidation selon la revendication 44, dans laquelle la particule de vecteur est une réplication défectueuse.

46. Lignée cellulaire d'encapsidation selon la revendication 44, dans laquelle le génome est dérivé de manière lentivirale et les LTR sont lentivirales.

47. Dispositif de culture de cellules implantable, le dispositif comprenant .
i. une membrane semi-perméable permettant la diffusion d'un facteur de croissance à travers celle-ci ; et
ii. au moins une cellule hôte isolée telle que définie selon la revendication 37.

48. Dispositif selon la revendication 47, dans lequel la membrane semi-perméable est immuno-isolante.

49. Dispositif selon la revendication 47, dans lequel la membrane semi-perméable est microporeuse.

50. Dispositif selon la revendication 47, dans lequel le dispositif comprend en outre une matrice placée dans la membrane semi-perméable.

51. Dispositif selon la revendication 47, dans lequel le dispositif comprend en outre une ancre d'attache.

52. Utilisation du vecteur selon la revendication 35 ou du dispositif selon l'une quelconque des revendications 47 à 51 en tant que médicament.

53. Utilisation du vecteur selon la revendication 35 pour la préparation d'un médicament pour le traitement d'un trouble du système nerveux.

54. Utilisation selon la revendication 53, dans laquelle le trouble du système nerveux est choisi dans le groupe constitué par une neuropathie périphérique, incluant une douleur neuropathique, une lésion de la moelle épinière, une alvusion des racines de la moelle épinière, un tic douloureux, et une causalgie.

55. Utilisation selon la revendication 52, dans laquelle le médicament est destiné au traitement d'une maladie oculaire, telle que les lésions et les ulcères de la cornée, et les rétinopathies.

56. Utilisation du vecteur selon la revendication 35, pour la préparation d'un médicament destiné au traitement d'un trouble du SNC.

57. Utilisation selon la revendication 56, dans laquelle le trouble du SNC est une maladie neurodégénérative.

58. Utilisation selon la revendication 57, dans laquelle la maladie neurodégénérative est une neuropathie périphérique incluant une douleur neuropathique.

59. Polypeptide comprenant un peptide signal et un polypeptide de neublastine, dans lequel le polypeptide n'a pas une pro-région de neublastine, ledit peptide signal et ledit polypeptide de neublastine étant tels que définis selon l'une quelconque des revendications 1 à 19.
